# EUROPEAN PATENT APPLICATION

(11) **EP 4 282 484 A2**
(43) Date of publication of application: **29.11.2023**
(21) Application number: 23183309.6
(22) Date of filing: 02.12.2015
(51) Int. Cl.: A61P 35/00

(54) **BISPECIFIC ANTIBODIES AGAINST CD3EPSILON AND BCMA FOR USE IN TREATMENT OF DISEASES**

(30) Priority: 03.12.2014 EP 14196168
(62) Divisional of application: 15804458.6
(71) Applicant: Bristol-Myers Squibb Company, Princeton, NJ 08543 (US)
(72) Inventor: VU, Minh Diem, 8832 Wollerau (CH); STREIN, Klaus, 69469 Weinheim (DE); HUNZIKER, Erich, 8832 Wilen (CH)
(74) Representative: Redhouse, Juliet Lauren

(57) **Abstract**

The invention relates to bispecific antibodies against CD3ε and BCMA for use in the treatment of diseases. The invention provides methods of determining the responsiveness of a patient to such treatment and relates to diagnostic assays.

## Description

The present invention relates to bispecific antibodies against CD3ε and BCMA for use in the treatment of diseases. The present invention provides methods of determining the response of a patient to such treatment and related diagnostic assays.

### Background of the Invention

Human B cell maturation target, also known as BCMA; TR17_HUMAN, TNFRSF17 (UniProt Q02223), is a member of the tumor necrosis receptor superfamily that is preferentially expressed in differentiated plasma cells [Laabi et al. 1992; Madry et al. 1998]. BCMA is a non glycosylated type III transmembrane protein, which is involved in B cell maturation, growth and survival. BCMA is a receptor for two ligands of the TNF superfamily: APRIL (a proliferation-inducing ligand), the high-affinity ligand to BCMA and the B cell activation factor BAFF, the low-affinity ligand to BCMA (THANK, BlyS, B lymphocyte stimulator, TALL-1 and zTNF4). APRIL and BAFF show structural similarity and overlapping yet distinct receptor binding specificity. The negative regulator TACI also binds to both BAFF and APRIL. The coordinate binding of APRIL and BAFF to BCMA and/or TACI activates transcription factor NF-κB and increases the expression of pro-survival Bcl-2 family members (e.g. Bcl-2, Bcl-xL, Bcl-w, Mcl-1, A1) and the downregulation of pro-apoptotic factors (e.g. Bid, Bad, Bik, Bim, etc.), thus inhibiting apoptosis and promoting survival. This combined action promotes B cell differentiation, proliferation, survival and antibody production (as reviewed in Rickert RC et al., Immunol Rev (2011) 244 (1): 115-133).

Novak AJ et al. BLOOD, 103, (2004) 689-694 relates to the expression of BCMA, TACI, and BAFF-R on multiple myeloma cells and the mechanism for growth . Li et al., Med Oncol 27 (2010) 439-445 mention that BCMA is expressed on plasma cells. Dispenzieri et al., Mayo Clin Proc 82(3), (2007), 323-341 relates to the treatment of Multiple Myeloma Based on Mayo Stratification of Myeloma and Risk-Adapted Therapy (mSMART). Schaumann D. (thesis, Berlin 2006) reports BCMA has been found to be essential for the survival of long-lived plasma cells and that long-lived plasma cells are effector cells in autoimmune diseases, see also O'Connor et al., J Exp Med.199, ( 2004) 91-98.WO2012143498 relates to a method for the stratification of a multiple myeloma (MM) patients. WO 200932058 relates to the predicting an individual's likelihood of having a condition associated with autoimmune activity, such as systemic lupus erythematosus SLE.

Sanchez E, et al., Br J Haematology 158, 727-38 (2012) and WO2014089335 report that BCMA concentrations were higher in the supernatants of cultured bone marrow mononuclear cells from multiple myeloma (MM) patients than in healthy subjects and suggest that serum BCMA levels may be a biomarker for monitoring disease status and overall survival of MM patients.

The TCR/CD3 complex of T-lymphocytes consists of either a TCR alpha (α)/beta (β) or TCR gamma (y)/delta (δ) heterodimer coexpressed at the cell surface with the invariant subunits of CD3 labeled gamma (γ), delta (δ), epsilon (ε), zeta (ζ), and eta (η). Human CD3ε is described under UniProt P07766 (CD3E_HUMAN). An anti CD3ε antibody described in the state of the art is SP34 (Yang SJ, The Journal of Immunology (1986) 137; 1097-1100). SP34 reacts with both primate and human CD3. SP34 is available from PharMingen. A further anti CD3 antibody described in the state of the art is UCHT-1 (see WO2000041474). A further anti CD3 antibody described in the state of the art is BC-3 (Fred Hutchinson Cancer Research Institute; used in Phase I/II trials of GvHD, Anasetti et al., Transplantation 54: 844 (1992)).

A wide variety of recombinant bispecific antibody formats have been developed in the recent past, e.g. by fusion of, e.g. an IgG antibody format and single chain domains (see Kontermann RE, mAbs 4:2, (2012) 1-16).

Antibodies against BCMA are described e.g. in Gras M-P. et al. Int Immunol. 7 (1995) 1093-1106, WO200124811, WO200124812, WO2010104949 and WO2012163805. Antibodies against BCMA and their use for the treatment of lymphomas and multiple myeloma are mentioned e.g. in WO2002066516 and WO2010104949. WO2013154760 relates to chimeric antigen receptors (CAR) comprising a BCMA recognition moiety and a T-cell activation moiety. Ryan, MC et al., Mol. Cancer Ther. 6 (2007) 3009-3018 relate to targeting of BCMA for plasma cell malignancies and expression of BCMA on the surface of multiple myeloma cells (MM cells). Bispecific antibodies against CD3 and BCMA are mentioned in WO2007117600, WO2009132058, WO2012066058, WO2012143498, and WO2013072415, WO2014122143 and WO2014122144. WO2013072406 and WO2014140248 mention E:T ratios in some figures and examples; however it is only reported that in the respective killing assay experiments there were used 10 effector cells for 1 target cell (cell lines not patient samples). This E:T ratio is therefore artificial and there were not shown any E:T ratios in myeloma patient bone marrow samples or given any hint on the relation of E:T ratio to antibody treatment.

WO2012143498 mentions a method for the stratification, diagnosing, or selecting an antibody-based multiple myeloma (MM) therapy of a multiple myeloma (MM) patient if malignant B-cells express BCMA protein on their surface.

There is a need for an improved therapy of a patient suffering from a disorder involving plasma cells.

### Summary of the Invention

T-cell bispecific antibodies are potent compounds to effectively kill e.g. target cells by activation of T cells directly at the proximity of target cells. T-cell bispecific antibodies binding to BCMA e.g. on the surface of malignant plasma cells in the case of multiple myeloma cells or anti-nuclear antibody secreting-plasma cells in the case of systemic lupus erythematosus or rheumatoid arthritis, can be dosed dependently to kill these plasma cells. The inventors have recognized certain parameters which are influencing the killing of the plasma cells. These parameters are the magnitude of BCMA expression measured by an appropriate flow cytometry method, presence of certain concentrations of soluble BCMA, the ratio of T cells to malignant plasma cells and the presence of certain concentrations of the soluble BCMA ligand APRIL. The findings of the inventors provide an important guidance for e.g. the treating physician(s) to tailor the therapy with BCMA-T-cell bispecific antibodies to the individual patient and the findings of the inventors also provide the scientific basis for test kits to measure said parameters.

The invention relates to a bispecific antibody specifically binding to the extracellular domain of human BCMA (further named also as "BCMA") and human CD3ε (further named also as "CD3"), for use in the treatment of a patient suffering from a disorder involving plasma cells, and whereby in an isolated body fluid sample of said patient, comprising CD138⁺ CD38⁺ cells, BCMA expression on said CD138⁺ CD38⁺ cells, measured by using an anti-BCMA antibody with a Kd value, which is 0.70 to 1.3 fold of the Kd value of the anti-BCMA antibody part of said bispecific antibody, is 80 or more, preferably 100 or more, preferably 200 or more, even more preferably 300 or more over baseline determined as Relative Median or Mean Fluorescence Intensity MFI.

The invention relates to a bispecific antibody specifically binding to the extracellular domain of human BCMA and human CD3, for use in the treatment of a patient suffering from a disorder involving plasma cells, said disorder being characterized in that in an isolated body fluid sample of said patient, comprising CD138+ CD38+ cells, BCMA expression on said CD138+ CD38+ cells, measured by using an anti-BCMA antibody with a Kd value, which is 0.70 to 1.3 fold of the Kd value of the anti-BCMA antibody part of said bispecific antibody, is 80 or more, preferably 100 or more, preferably 200 or more, even more preferably 300 or more over baseline determined as Relative Median or Mean Fluorescence Intensity MFI.

Preferably the invention relates to a bispecific antibody for use according to the invention, characterized in that said bispecific antibody and said anti-BCMA antibody are monovalent for BCMA binding. Preferably the Kd value of said anti-BCMA antibody (part of said bispecific antibody) is 100nM or lower.

Preferably the invention relates to a bispecific antibody for use according to the invention, characterized in that said bispecific antibody and said anti-BCMA antibody are bivalent for BCMA binding.

Preferably the invention relates to a bispecific antibody for use according to the invention characterized in that said bispecific antibody and said anti-BCMA antibody are trivalent for BCMA binding.

Preferably the invention relates to a bispecific antibody for use according to the invention, characterized in that said bispecific antibody comprises as its heavy and light chain CDRs, CDRs of the same amino acid sequences as said anti-BCMA antibody.

Preferably the invention relates to a bispecific antibody for use according to the invention, characterized in that said bispecific antibody comprises as its heavy and light chain variable regions, variable regions of the same amino acid sequences as said anti-BCMA antibody.

The invention relates to a bispecific antibody specifically binding to the extracellular domain of human BCMA and human CD3ε, for use in the treatment of a patient suffering from a disorder involving plasma cells, whereby the ratio of T cells (effector cells) to target cells (E:T ratio) in an isolated body fluid sample of said patient is 0.35 : 1, preferably 0.5 : 1 or higher, preferably 1:1 or higher, more preferably 5:1 or higher, even more preferably 10:1 or higher. Preferably the E:T ratio is measured as ratio of CD3⁺ cells to CD138⁺ CD38⁺ cells, preferably as ratio of the CD3⁺ cell subset of CD45⁺ CD19⁻ CD56⁻ T cells to CD138⁺ CD38⁺ CD45⁺ CD19⁻ CD56⁺ cells. If the patient suffers from multiple myeloma, such target cells are therefore multiple myeloma cells.

The invention relates to a bispecific antibody specifically binding to the extracellular domain of human BCMA and human CD3ε, for use in the treatment of a patient suffering from a disorder involving plasma cells said disorder being characterized in that the ratio of T cells (effector cells) to target cells (E:T ratio) in an isolated body fluid sample of said patient is 0.35 : 1or higher, preferably 0.5 : 1 or higher, preferably 1:1 or higher, more preferably 5:1 or higher, preferably 10:1 or higher and preferably 0.35 : 1 to 22 : 1. The E:T ratios found in the samples of the patients for whom samples could be treated effectively were found as 0.35 and higher. Samples with E:T ratio between 0.35 : 1 and 11:1 were tested respectively. E:T ratios up to 22:1 were also detected in patient samples (not tested). Based on these findings the inventors recognized that patients with such samples or with samples with even higher E:T values could also be treated effectively with a bispecific antibody according to the invention. Preferably the E:T ratio is measured as ratio of CD3+ cells to CD138+ CD38+ cells, preferably as ratio of the CD3+ cell subset of CD45+ CD19- CD56- T cells to CD138+ CD38+ CD45+ CD19- CD56+ cells. If the patient suffers from multiple myeloma, such target cells are therefore multiple myeloma cells.

The invention relates to a bispecific antibody specifically binding to the extracellular domain of human BCMA and human CD3ε, for use in the treatment of a patient suffering from a disorder involving plasma cells, whereby said therapy comprises successively
i) isolating from said patient a body fluid sample,
ii) measuring the amount of soluble BCMA in said sample, and
iii) if the amount of soluble BCMA in said sample is 2.5 ng/mL or higher, and
iv) if said soluble BCMA in said patient sample specifically binds to said bispecific antibody, treating said patient with said bispecific antibody at higher doses and/or at a more frequent treatment schedule.

The invention relates to a bispecific antibody specifically binding to the extracellular domain of human BCMA and human CD3ε, for use in the treatment of a patient suffering from a disorder involving plasma cells, said disorder being characterized in that in an isolated body fluid sample from said patient the amount of soluble BCMA is 2.5 ng/mL or higher, and said soluble BCMA in said patient sample specifically binds to said bispecific antibody, characterized in that said treatment of said patient with said bispecific antibody is performed with a dose per week which is 1.5 fold up to 10 fold or/and in that the time interval between dose-administrations is shortened from once per week administration up to once per day compared to a standard dose. Preferably said treatment of said patient with said bispecific antibody is performed with a dose per week which is 1.5 fold up to 2.0 fold compared to a standard dose. Preferably said treatment of said patient with said bispecific antibody is performed in that the time interval between dose-administrations is shortened from once per week administration up to twice a week compared to the standard dose.

The invention relates to a bispecific antibody specifically binding to the extracellular domain of human BCMA and human CD3ε, for use in the treatment of a patient suffering from a disorder involving plasma cells, whereby said therapy comprises successively
i) isolating from said patient a body fluid sample,
ii) measuring the amount of soluble BCMA in said sample, and
iii) if the amount of soluble BCMA in said sample is 2.5 ng/mL or higher, and
iv) if said soluble BCMA in said patient sample specifically binds to said bispecific antibody, treating said patient with said bispecific antibody at a higher dose for the first dose or at a more frequent treatment schedule with a shorter period between the first dose and the second dose of said bispecific antibody or with a shorter period between the first dose and the third dose of said bispecific antibody..

The invention relates to a bispecific antibody specifically binding to BCMA and CD3ε which competes with soluble BCMA for binding to human BCMA receptor and/or blocks APRIL mediated activation of NF-κB for use in the treatment of a patient suffering from a disorder involving plasma cells, whereby said therapy comprises successively
i) isolating from said patient a body fluid sample comprising plasma cells and T cells,
ii) measuring the amount of APRIL in said sample, and
iii) if the amount of APRIL in said patient sample is more than 100 ng/mL, treating said patient with said bispecific antibody at higher doses and/or at a more frequent treatment schedule.

The invention relates to a bispecific antibody specifically binding to BCMA and CD3ε which competes with soluble BCMA for binding to human BCMA receptor and/or blocks APRIL mediated activation of NF-κB for use in the treatment of a patient suffering from a disorder involving plasma cells, said disorder being characterized in that in an isolated body fluid sample from said patient the amount of APRIL is higher than 10 ng/mL and up to 100 ng/mL, characterized in that said treatment of said patient with said bispecific antibody is performed per week with a dose which is 1.5 fold up to 20 fold or/and in that the time interval between dose-administrations is shortened from once per week administration up to once a day compared to a standard dose. Preferably said treatment of said patient with said bispecific antibody is performed with a dose per week which is1.5 fold up to a 3.0fold compared to a standard dose. Preferably said treatment of said patient with said bispecific antibody is performed in that the time interval between dose-administrations is shortened from once per week administration up to three times a week compared to the standard dose.

The invention relates to a bispecific antibody specifically binding to BCMA and CD3ε which competes with soluble BCMA for binding to human BCMA receptor, whereby said antibody competes with APRIL for binding to BCMA, whereby said antibody competes with APRIL for binding to BCMA, whereby said antibody competes with APRIL for binding to BCMA and/or blocks APRIL mediated activation of NF-κB for use in the treatment of a patient suffering from a disorder involving plasma cells, whereby said therapy comprises successively
i) isolating from said patient a body fluid sample comprising plasma cells and T cells,
ii) measuring the amount of APRIL in said sample, and
iii) if the amount of APRIL in said patient sample is more than 100 ng/mL, treating said patient with said bispecific antibody at a two times higher dose at APRIL concentrations of 100 ng/mL and a further increased dose up to 80 times higher if APRIL concentration increases up to 1000 ng/mL, compared to the dose recommended for a patient with soluble APRIL concentration below 100 ng/mL or treating said patient with a respective more frequent treatment schedule to reach said higher doses with a shorter period between any two doses of said bispecific antibody.

The invention relates to a bispecific antibody specifically binding to BCMA and CD3ε which competes with soluble BCMA for binding to human BCMA receptor, whereby said antibody competes with APRIL for binding to BCMA, whereby said antibody competes with APRIL for binding to BCMA, whereby said antibody competes with APRIL for binding to BCMA and/or blocks APRIL mediated activation of NF-κB for use in the treatment of a patient suffering from a disorder involving plasma cells, said disorder being characterized in that in an isolated body fluid sample of said patient comprising plasma cells and T cells, the amount of APRIL is more than 100 ng/mL, characterized in treating said patient with said bispecific antibody at a two times higher dose at APRIL concentrations of 100 ng/mL and a further increased dose up to 80 times higher if APRIL concentration increases up to 1000 ng/mL, compared to the dose recommended for a patient with soluble APRIL concentration below 100 ng/mL or treating said patient with a respective more frequent treatment schedule to reach said higher doses with a shorter period between any two doses of said bispecific antibody.

The amount of APRIL is preferably measured by use of an ELISA method.

The invention relates to a method of determining BCMA protein expression in an isolated body fluid sample comprising CD138⁺ CD38⁺ cells, of a patient, suffering from a disorder involving plasma cells, said method comprising measuring BCMA expression on said CD138⁺ CD38⁺ cells by using an anti-BCMA antibody with a Kd value, which is 0.70 to 1.3 fold of the Kd value of the anti-BCMA antibody part of a bispecific antibody specifically binding to BCMA and CD3ε, intended for use in the treatment of said patient, and determining by flow cytometry whether Relative Median or Mean Fluorescence Intensity MFI is 80 or more, preferably 100 or more, preferably 200 or more, even more preferably 300 or more over baseline.

The invention relates to a method of treating a patient, suffering from a disorder involving plasma cells, comprising
analyzing isolated body fluid sample comprising CD138⁺ CD38⁺ cells from said patient for BCMA expression on said CD138⁺ CD38⁺ cells by using an anti-BCMA antibody with a Kd value, which is 0.70 to 1.3 fold of the Kd value of the anti-BCMA antibody part of a bispecific antibody specifically binding to BCMA and CD3ε, intended for use in the treatment of said patient, and if Relative Median or Mean Fluorescence Intensity MFI is 80 or more, preferably 100 or more over baseline, preferably 200 or more, even more preferably 300 or more treating said patient with said bispecific antibody.

Preferably the invention relates to selecting a treatment plan that is most effective for a patient, suffering from a disorder involving plasma cells, and whereby an isolated body fluid sample of said patient show MFI for BCMA of 80 or more, preferably 100 or more, preferably 200 or more, even more preferably 300 or more over baseline.

The invention relates to a method for predicting the likelihood of a patient, suffering from a disorder involving plasma cells, to respond to a treatment with a bispecific antibody specifically binding to BCMA and CD3ε, whereas the cell-surface BCMA expression in an isolated body fluid sample of said patient, comprising CD138⁺ CD38⁺ cells, and measured by using an anti-BCMA antibody with a Kd value, which is 0.70 to 1.3 fold of the Kd value of the anti-BCMA antibody part of said bispecific antibody, of 80 or more, preferably 100 or more, preferably 200 or more, even more preferably 300 or more over baseline determined as Relative Median or Mean Fluorescence Intensity MFI is predictive of the patient's likelihood to respond to said treatment.

The invention relates to an in vitro method of determining cell-surface BCMA expression in an isolated body fluid sample, comprising determining whether Relative Median or Mean Fluorescence Intensity MFI for said CD138⁺ CD38⁺ cells, using an anti-BCMA antibody with a Kd value, which is 0.70 to 1.3 fold of the Kd value of the anti-BCMA antibody part of a therapeutic bispecific antibody specifically binding to BCMA and CD3ε, is 80 or more, preferably 100 or more, preferably 200 or more, even more preferably 300 or more over baseline.

The invention relates to an in vitro method of selecting a treatment plan that is most effective for treating a patient, suffering from a disorder involving plasma cells, whereby for said patient
cell-surface BCMA expression in an isolated body fluid sample, comprising CD138⁺ CD38⁺ cells, measured by using an anti-BCMA antibody with a Kd value, which is 0.70 to 1.3 fold of the Kd value of the anti-BCMA antibody part of a therapeutic bispecific antibody, specifically binding to BCMA and CD3ε, is 100 or more , preferably 200 or more, even more preferably 300 over baseline determined as Relative Median or Mean Fluorescence Intensity MFI, whereby the treatment plan involves the use of a therapeutic bispecific antibody specifically binding to BCMA and CD3ε.

The invention relates to a method for selecting a therapy for treating a patient, suffering from a disorder involving plasma cells, comprising
i) if cell-surface BCMA expression in an isolated body fluid sample, comprising CD138⁺ CD38⁺ cells, measured by using an anti-BCMA antibody with a Kd value, which is 0.70 to 1.3 fold of the Kd value of the anti-BCMA antibody part of a therapeutic bispecific antibody, specifically binding to BCMA and CD3ε, is 100 or more, preferably 200 or more, even more preferably 300 or more over baseline determined as Relative Median or Mean Fluorescence Intensity MFI, treating said patient with said therapeutic antibody, or
ii) if cell-surface BCMA expression in an isolated body fluid sample, comprising CD138⁺ CD38⁺ cells, measured by using an anti-BCMA antibody with a Kd value, which is 0.70 to 1.3 fold of the Kd value of the anti-BCMA antibody part of a therapeutic bispecific antibody, specifically binding to BCMA and CD3ε, is lower than 100, preferably lower than 50, even preferable lower than 10 over baseline determined as Relative Median or Mean Fluorescence Intensity MFI, not treating said patient with said therapeutic antibody.

The invention relates to a method for determining in an isolated body fluid sample of a patient, suffering from a disorder involving plasma cells, whether the ratio of CD3⁺ cells to CD138⁺ CD38⁺ cells is 0.35 : 1, preferably 0.5 : 1 or higher, preferably 1:1 or higher, more preferably 5:1 or higher, even more preferably 10:1 or higher.

The invention relates to a method of treating a patient suffering from a disorder involving plasma cells, comprising analyzing in an isolated body fluid sample of said patient whether the ratio of CD3⁺ cells to CD138⁺ CD38⁺ cells is 0.35 : 1, preferably 0.5 : 1 or higher, preferably 1:1 or higher, more preferably 5:1 or higher, even more preferably 10:1 or higher.

Preferably the invention relates to selecting a treatment plan that is most effective for a patient which show a ratio of CD3⁺ cells to CD138⁺ CD38⁺ cells of 0.35 : 1, preferably 0.5 : 1 or higher, preferably 1:1 or higher, more preferably 5:1 or higher, even more preferably 10:1 or higher.

Preferably the invention relates to selecting a treatment plan that is most effective for a patient, whereby
i) if the ratio of CD3⁺ cells to CD138⁺ CD38⁺ cells in a body fluid sample of said patient is 0.35 : 1, preferably 0.5 : 1 or higher, preferably 1:1 or higher, more preferably 5:1 or higher, even more preferably 10:1 or higher, treating said patient with a therapeutic bispecific antibody specifically binding to BCMA and CD3ε in monotherapy
ii) if the ratio of CD3⁺ cells to CD138⁺ CD38⁺ cells in an isolated body fluid sample of said patient is lower than 0.5 : 1, preferably lower than 0.25 : 1, treating said patient with a therapeutic bispecific antibody specifically binding to BCMA and CD3ε in combination with T-cell proliferative therapy or T-cell chemoattractant therapy.

The invention relates to a method for predicting the likelihood of a patient, suffering from a disorder involving plasma cells, to respond to a treatment with a bispecific antibody specifically binding to BCMA and CD3ε, by measuring in an isolated body fluid sample of said patient whether the ratio of CD3⁺ cells to CD138⁺ CD38⁺ cells is 0.35 : 1, preferably 0.5 : 1 or higher, preferably 1:1 or higher, more preferably 5:1 or higher, even more preferably 10:1 or higher, which is predictive of the patient's likelihood to respond to a treatment.

The invention relates to an in vitro method of determining in an isolated body fluid sample of a patient suffering from a disorder involving plasma cells whether the ratio of CD3⁺ cells to CD138⁺ CD38⁺ cells is 0.5 : 1 or higher, preferably 1:1 or higher, more preferably 5:1 or higher, even more preferably 10:1 or higher,.

The invention relates to an in vitro method of selecting a treatment plan that is most effective for treating a patient, suffering from a disorder involving plasma cells, whereby in an isolated body fluid sample of said patient the ratio of CD3⁺ cells to CD138⁺ CD38⁺ cells is determined as 0.35 : 1, preferably 0.5 : 1 or higher, preferably 1:1 or higher, more preferably 5:1 or higher, even more preferably 10:1 or higher and whereby the treatment plan involves the use of a therapeutic bispecific antibody specifically binding to BCMA and CD3ε.

The invention relates to a method for selecting a therapy with a bispecific antibody specifically binding to BCMA and CD3ε for a patient, suffering from a disorder involving plasma cells, comprising
i) if in an isolated body fluid sample of said patient the ratio of CD3⁺ cells to CD138⁺ CD38⁺ cells is 0.35 : 1, preferably 0.5 : 1 or higher, preferably 1:1 or higher, more preferably 5:1 or higher, even more preferably 10:1 or higher, treating said patient with said therapeutic antibody, or
ii) if in an isolated body fluid sample of said patient the ratio of CD3⁺ cells to CD138⁺ CD38⁺ cells is lower than 0.35 : 1, preferably 0.5 : 1, preferably lower than 0.25 : 1 treating said patient with a bispecific antibody specifically binding to BCMA and CD3 in combination with T-cell proliferative therapy or T-cell chemoattractant therapy.

The invention relates to a method of determining in an isolated body fluid sample comprising CD138⁺ CD38⁺ cells, of a patient suffering from a disorder involving plasma cells, whether the amount of soluble BCMA in said sample is 2.5 ng/mL or higher, preferably 10 ng/mL or higher, more preferably 50 ng/mL or higher, even more preferably 250 ng/mL or higher.

The invention relates to a method of treating a patient suffering from a disorder involving plasma cells, comprising determining whether the amount of soluble BCMA in said body fluid sample is 2.5 ng/mL or higher, preferably 10 ng/mL or higher, more preferably 50 ng/mL or higher, even more preferably 250 ng/mL or higher.

Preferably the invention relates to selecting a treatment plan that is most effective for a patient which show MFI for BCMA of 80 or more, preferably 100 or more, preferably 200 or more, even more preferably 300 or more over baseline. The invention relates to a method for predicting the likelihood of a patient, suffering from a disorder involving plasma cells, to respond to a treatment with a bispecific antibody specifically binding to BCMA and CD3ε, whereby an amount of soluble BCMA in said body fluid sample of 2.5 ng/mL or higher, preferably 10 ng/mL or higher, more preferably 50 ng/mL or higher, even more preferably 250 ng/mL or higher is predictive of the patient's likelihood to respond to a treatment.

The invention relates to an in vitro method of determining in an isolated body fluid sample, whether the amount of soluble BCMA in said sample is 2.5 ng/mL or higher, preferably 10 ng/mL or higher, more preferably 50 ng/mL or higher, even more preferably 250 ng/mL or higher.

Preferably the invention relates to selecting a treatment plan that is most effective for a patient which show an amount of soluble BCMA of 2.5 ng/mL or higher, preferably 10 ng/mL or higher, more preferably 50 ng/mL or higher, even more preferably 250 ng/mL or higher.

The invention relates to an in vitro method of selecting a treatment plan that is most effective for treating a patient, suffering from a disorder involving plasma cells, by determining whether the amount of soluble BCMA in said sample is 2.5 ng/mL or higher, preferably 10 ng/mL or higher, more preferably 50 ng/mL or higher, even more preferably 250 ng/mL or higher, and the treatment plan involves the use of a bispecific antibody specifically binding to BCMA and CD3ε.

The invention relates to a method for selecting a therapy for treating a patient, suffering from a disorder involving plasma cells a therapy, comprising
i) if the amount of soluble BCMA in said sample is lower than 2.5 ng/mL, treating said patient with said therapeutic antibody, or
ii) if the amount of soluble BCMA in said sample is 2.5 ng/mL or higher and,
   if said soluble BCMA in said patient sample does not bind to said bispecific antibody, treating said patient with said therapeutic antibody, or
iii) if the amount of soluble BCMA in said sample is 2.5 ng/mL or higher, preferably 10 ng/mL or higher, more preferably 50 ng/mL or higher, even more preferably 250 ng/mL or higher and,
if said soluble BCMA in said patient sample specifically binds to said bispecific antibody, treating said patient with said bispecific antibody at higher doses and/or at a more frequent treatment schedule.

Preferably the invention relates to a method for selecting a therapy for treating a patient, suffering from a disorder involving plasma cells a therapy, comprising
i) if the amount of soluble BCMA in said sample is lower than 2.5 ng/mL, treating said patient with said therapeutic antibody, or
ii) if the amount of soluble BCMA in said sample is 2.5 ng/mL or higher and,
   if said soluble BCMA in said patient sample does not bind to said bispecific antibody, treating said patient with said therapeutic antibody, or
iii) if the amount of soluble BCMA in said sample is 2.5 ng/mL or higher, preferably 10 ng/mL or higher, more preferably 50 ng/mL or higher, even more preferably 250 ng/mL or higher and, if said soluble BCMA in said patient sample specifically binds to said bispecific antibody, treating said patient with said bispecific antibody at a higher dose for the first dose or at a more frequent treatment schedule with a shorter period between the first dose and the second dose of said bispecific antibody or with a shorter period between the first dose and the third dose of said bispecific antibody.

The invention relates to a method of determining in an isolated body fluid sample comprising CD138⁺ CD38⁺ cells, of a patient suffering from a disorder involving plasma cells, whether the amount of soluble APRIL in said sample is 100 ng/mL or higher, preferably 1000 ng/mL or higher.

The invention relates to a method of treating a patient, suffering from a disorder involving plasma cells and diagnosed that the amount of soluble APRIL in an isolated body fluid sample of said patient is 100 ng/mL or higher, preferably 1000 ng/mL or higher, with a bispecific antibody specifically binding to BCMA and CD3ε.

Preferably the invention relates to selecting a treatment plan that is most effective for a patient which show an amount of soluble APRIL of 100 ng/mL or higher, preferably 1000 ng/mL or higher.

The invention relates to a method for predicting the likelihood of a patient, suffering from a disorder involving plasma cells, to respond to a treatment with a bispecific antibody specifically binding to BCMA and CD3ε, whereby the amount of soluble APRIL in said sample of 100 ng/mL, preferably 1000 ng/mL or higher is predictive of the patient's likelihood to respond to a treatment.

The invention relates to an in vitro method of determining in an isolated body fluid sample, whether the amount of soluble APRIL in said sample is 100 ng/mL or higher, preferably 1000 ng/mL or higher.

The invention relates to an in vitro method of selecting a treatment plan that is most effective for treating a patient, suffering from a disorder involving plasma cells, by determining whether the amount of soluble APRIL in said sample is 100 ng/mL or higher, preferably 1000 ng/mL or higher, and the treatment plan involves the use of an APRIL competitive bispecific antibody or an APRIL non-competitive bispecific antibody.

The invention relates to a method for selecting a therapy for treating a patient, suffering from a disorder involving plasma cells a therapy, comprising
i) the amount of soluble APRIL in said sample is 100 ng/mL or lower, preferably 20 ng/mL or lower treating said patient with said therapeutic antibody, or
ii) the amount of soluble APRIL in said sample is 100 ng/mL or higher, preferably 1000 ng/mL or higher, treating said patient with APRIL non-competitive bispecific antibodies, or
iii) the amount the amount of soluble APRIL in said sample is higher than 100 ng/mL, preferably 1000 ng/mL or higher, treating said patient with said bispecific antibody at higher doses and/or at a more frequent treatment schedule.

Preferably the invention relates to a method for selecting a therapy for treating a patient, suffering from a disorder involving plasma cells a therapy, comprising
i) the amount of soluble APRIL in said sample is 100 ng/mL or lower, preferably 20 ng/mL or lower treating said patient with said therapeutic antibody, or
ii) the amount of soluble APRIL in said sample is 100 ng/mL or higher, preferably 1000 ng/mL or higher, treating said patient with BCMA ligand competitive bispecific antibodies, or
iii) if the amount of APRIL in said patient sample is more than 100 ng/mL, treating said patient with said bispecific antibody at a two times higher dose at APRIL concentrations of 100 ng/mL and a further increased dose up to 80 times higher if APRIL concentration increases up to 1000 ng/mL, compared to the dose recommended for a patient with soluble APRIL concentration below 100 ng/mL or treating said patient with a respective more frequent treatment schedule to reach said higher doses with a shorter period between any two doses of said bispecific antibody.

The invention relates to a method for determining a treatment plan that is most effective for a patient suffering from a disorder involving plasma cells.

The invention relates to a method for determining a treatment plan for a new patient, suffering from a disorder involving plasma cells, comprising:
providing, utilizing at least one method for investigation the BCMA related plasma cell status of said new patient;
searching, utilizing at least the result of one method, for a prior treatment plan for a prior patient suffering from the same disorder with at least one similar representation; and
reviewing the prior treatment plan for the prior patient in order to determine how to improve the treatment of the new patient based on information in at least one prior treatment plan.

The invention relates to a method for diagnosing and treating a disorder involving plasma cells in a patient comprising analyzing in an isolated body fluid sample of said patient BCMA expression on CD138+ CD38+ cells according to the invention, wherein the patient is diagnosed having said disease, if said BCMA expression is 80 or more, preferably 100 or more, preferably 200 or more, even more preferably 300 or more over baseline determined as Relative Median or Mean Fluorescence Intensity MFI and administering treatment with a bispecific antibody according to the invention to the diagnosed patient.

The invention relates to a method for diagnosing and treating a disorder involving plasma cells in a patient comprising analyzing in an isolated body fluid sample the ratio of T cells (effector cells) to target cells (E:T ratio), wherein the patient is diagnosed with said disease if said ratio is 0.35 : 1, preferably 0.35 : 1, preferably 0.5 : 1 or higher and administering treatment with a bispecific antibody according to the invention to the diagnosed patient.

The invention relates to a method for diagnosing and treating a disorder involving plasma cells in a patient comprising analyzing in an isolated body fluid sample patient the amount of soluble BCMA according to the invention wherein the patient is diagnosed with said disease if said soluble BCMA is 2.5 ng/mL or higher, and said soluble BCMA in said patient sample specifically binds to said bispecific antibody, and administering treatment with a bispecific antibody according to the invention to the diagnosed patient.at higher doses and/or at a more frequent treatment schedule.

The invention relates to a method for diagnosing and treating a disorder involving plasma cells in a patient comprising analyzing in an isolated body fluid sample patient the amount of soluble BCMA according to the invention wherein the patient is diagnosed with said disease if said soluble BCMA is 2.5 ng/mL or higher, and said soluble BCMA in said patient sample specifically binds to said bispecific antibody, and administering treatment with a bispecific antibody according to the invention to the diagnosed patient is performed at a higher dose for the first dose or at a more frequent treatment schedule with a shorter period between the first dose and the second dose of said bispecific antibody or with a shorter period between the first dose and the third dose of said bispecific antibody.

The invention relates to a method for diagnosing and treating a disorder involving plasma cells in a patient comprising analyzing in an isolated body fluid sample patient the amount of APRIL according to the invention wherein the patient is diagnosed with said disease if the amount of APRIL is more than 100 ng/mL, and administering treatment with a bispecific antibody according to the invention to the diagnosed patient is performed with said bispecific antibody which competes with soluble BCMA for binding to human BCMA receptor and/or blocks APRIL mediated activation of NF-κB at higher doses and/or at a more frequent treatment schedule .

The invention relates to a method for diagnosing and treating a disorder involving plasma cells in a patient comprising analyzing in an isolated body fluid sample patient comprising plasma cells and T cells, the amount of APRIL according to the invention wherein the patient is diagnosed with said disease if the amount of APRIL is more than 100 ng/mL, and administering treatment with a bispecific antibody according to the invention to the diagnosed patient is performed with said bispecific antibody which competes with APRIL for binding to human BCMA receptor and/or blocks APRIL mediated activation of NF-xB at a two times higher dose at APRIL concentrations of 100 ng/mL and a further increased dose up to 80 times higher if APRIL concentration increases up to 1000 ng/mL, compared to the dose recommended for a patient with soluble APRIL concentration below 100 ng/mL or treating said patient with a respective more frequent treatment schedule to reach said higher doses with a shorter period between any two doses of said bispecific antibody.

Preferably the disease (disorder) is selected from the group consisting of multiple myeloma, systemic lupus erythematosus, and rheumatoid arthritis.

Preferably valence should be similar between the diagnostic antibody and the therapeutic antibody (e.g. a monovalent antibody for BCMA determination should be used for patient stratification for a BCMA antibody therapy with monovalent binding to the tumor target on malignant cells such as scFV-based BiTE molecules). Even more preferably is to use a BCMA antibody for BCMA determination which is the same as the BCMA binder of the BCMA antibody therapy.

Preferably the affinity to human BCMA of said bispecific antibody is 200 nM or lower, measured at an antibody concentration of 25 nM in presence of human BCMA Fc fusion at a concentration 500 nM or lower in an affinity setup surface plasmon resonance assay.

Preferably the potency (EC50) to kill BCMA-positive H929 cells (ATCC CRL-9068) of said bispecific antibody is measured as 2 nM or lower, when used at concentrations of 100 nM and lower, in presence of human PBCMs and H929 cells at a E:T ratio of 10:1 for 24h, in a redirected T-cell killing LDH release assay.

Preferably the affinity to human BCMA of said BCMA binding part, measured in an antibody of human IgG1 type, is 200 nM or lower at an antibody concentration of 25 nM in presence of human BCMA Fc fusion at a concentration of 500 nM or lower in an affinity setup surface plasmon resonance assay.

Preferably the antibody according to the invention is further characterized in that it binds also specifically to cynomolgus BCMA.

Preferably the bispecific antibody according to the invention comprising constant heavy regions CH2/CH3 of IgG1 subclass is characterized in comprising the mutations L234A, L235A and P239G (numbering according to Kabat) to avoid FcR and C1q binding and minimizing ADCC/CDC. The advantage is that such an antibody of the invention mediates its tumor cell killing efficacy purely by the powerful mechanism of T-cell redirection/activation. Additional mechanisms of action like effects on complement system and on effector cells expressing FcgammaR are avoided and the risk of side-effects is decreased.

Preferably an antibody according to the invention is characterized by showing tumor growth inhibition of more than 70%, preferably of more than 85%, preferably of close to 100% in a multiple myeloma xenograft model (e.g. xenograft with NCI-H929 cells or RPMI8226 cells or U266B1 cells or L-363 cells) at a dose of 1 mg/kg body weight (BW) administered intravenously (i.v.) or subcutaneously (s.c.) or intraperitoneal (i.p.) twice a week or once a week, preferably 0.5 mg/kg BW administered i.v. or i.p. or s.c. twice a week or once a week, preferably at 0.1 mg/kg BW administered i.v. or i.p. or s.c. twice a week or once a week, preferably at 0.05 mg/kg BW administered i.v. or i.p. or s.c. twice a week or once a week, preferably at 0.01 mg/kg BW administered i.v. or i.p. or s.c twice a week or once a week, preferably at 5µg/kg BW administered i.v. or i.p. or s.c. twice a week or once a week.

Preferably an antibody according to the invention is characterized by an elimination half-life in mice, preferably cynomolgus monkeys of longer than 24 hours, preferably 3 days or longer, preferably half-life is measured for the doses which are effective in the xenograft model at twice or once a week administration.

Bispecific antibodies binding to a target on tumor cells and to CD3 and having the molecular format (scFv)₂ have very short elimination half-life of 1 to 4 hours. In the clinical trials with the (scFv)₂ bispecific CD19xCD3 antibody blinatumomab, this compound had to be administered via a pump carried by the patients over weeks and months (Topp et al. J Clin Oncol 2011; 29(18): 2493-8). Compared to a twice a week or once a week iv or sc administration, treatment administered via a pump is much less convenient for the patients and also much more risky (e.g. failure of pump, issues with the catheter).

Preferably an antibody according to the invention is characterized in showing an EC50 value for binding to NCI-H929 cells (ATCC^{®} CRL-9068^{™}) of 500 nM or lower, preferably an EC50 value of 350 nM or lower, preferably an EC50 value of 100 nM and lower.

Preferably an antibody according to the invention is characterized by its capability to induce redirected killing of NCI-H929 tumor cells in the presence of human T cells with an EC50 lower than 1 nM, preferably 0.5 nM, preferably 0.1 nM and lower.

Preferably a bispecific antibody according to the invention is characterized by its capability to induce redirected killing of multiple myeloma patient primary myeloma cells in the presence of human T cells.

A further embodiment of the invention is a kit comprising a diagnostic anti-BCMA antibody and a therapeutic bispecific antibody against BCMA and CD3according to the invention.

A further embodiment of the invention is a kit comprising an anti-BCMA antibody and a bispecific antibody against BCMA and CD3, characterized in that the anti-BCMA antibody has a Kd value, which is 0.70 to 1.3 fold of the Kd value of the anti-BCMA antibody part of said bispecific antibody, is 80 or more, preferably 100 or more, preferably 200 or more, even more preferably 300 or more over baseline determined as Relative Median or Mean Fluorescence Intensity MFI and instructions for use, in particular instructions as how to perform the methods of the present invention. Preferably said anti-BCMA antibody and said bispecific antibody against BCMA and CD3 are both mono-, bi-, or trivalent and have preferably the same CDRs or VH and VL sequence.

The kit comprises at least one container and a label or package insert on or associated with the container. Suitable containers include, for example, bottles, vials, syringes, etc. The containers may be formed from a variety of materials such as glass or plastic. The container can have a sterile access port for extracting a therapeutic agent (for example the container may be an intravenous solution bag or a vial having a stopper pierceable by a hypodermic injection needle). The label or package insert can indicate that the composition is used for treating MM, SLE, RA or another disorder involving plasma cells.

Additionally, the kit may further comprise a second container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### Preferably the kit comprises;

1) For determination of cell-surface BCMA expression: Vials or tubes pre-loaded with labelled-antibodies (preferably four antibodies, one specifically binding to CD138, one to CD38, one to CD19, and one to BCMA (with the properties according to the present invention)) to determine BCMA on malignant PC; tubes with isotype control antibodies;
2) For determination of E:T ratio: Vials or tubes pre-loaded with labelled-antibodies to detect malignant PC and T cells (preferably four antibodies, one specifically binding to CD138, one to CD38, one to CD19, and one to CD3); tubes with isotype control antibodies;
3) For determination of soluble BCMA: ELISA kit comprising a microtiter plate, a capture antibody (polyclonal BCMA antibody), biotin-conjugated detection antibody (specifically binding to BCMA (with the properties according to the present invention)), mass-calibrated standard, streptavidin-HRP or streptavidin-ALP, detailed protocol, PBS, Wash Buffer - 0.05% Tween 20 in PBS, pH 7.2-7.4, Reagent Diluent1 - 1% BSA5 in PBS, Substrate Solution - 1:1 mixture of Color Reagent A, (H₂O₂) and Color Reagent B (Tetramethylbenzidine), Stop Solution - 2 N H₂SO₄;
4) For determination of soluble APRIL: ELISA kit comprising microtiter plate, capture antibody (anti-human APRIL antibody), biotin-conjugated detection antibody (anti-human APRIL antibody), mass-calibrated standard, streptavidin-HRP or streptavidin-ALP, detailed protocol, PBS, Wash Buffer - 0.05% Tween 20 in PBS, pH 7.2-7.4, Reagent Diluent1 - 1% BSA5 in PBS, Substrate Solution - 1:1 mixture of Color Reagent A, (H₂O₂) and Color Reagent B (Tetramethylbenzidine), Stop Solution - 2 N H₂SO₄.

### Description of the Figures

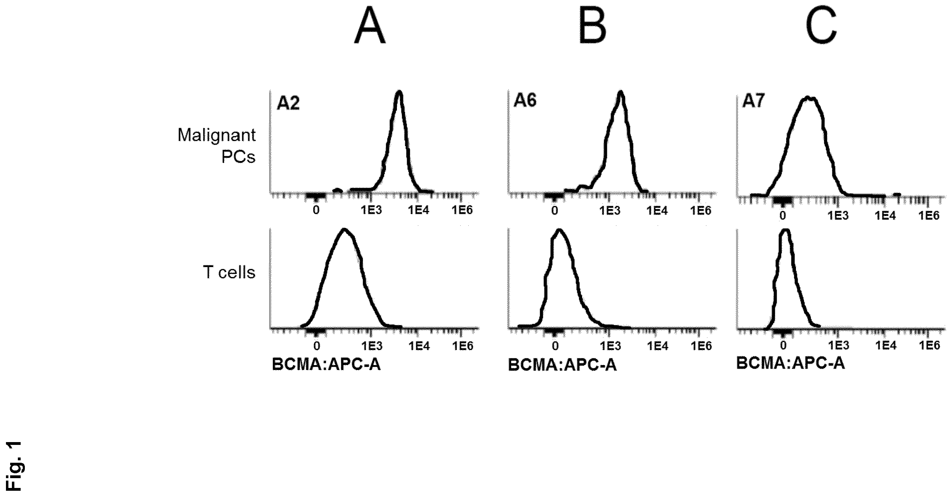

Figure 1. BCMA expression on patient malignant plasma cells as detected by flow cytometry and defined by relative mean or median fluorescence intensity. Representative FACS histogram plots of (A) Medium-high BCMA expression, (B) moderate BCMA expression and (C) low BCMA expression on patient myeloma cells as detected by flow cytometry (MFI). There is a clear shift to the right on the x axis corresponding to positive BCMA expression on patient myeloma cells when compared to the negative control (APC-conjugated BCMA-1 antibody gated on T cells). Based on the relative MFI values, myeloma patients express BCMA on their malignant plasma cells but BCMA expression varies from low expression (relative MFI values < 10³) to moderate expression (10³ - 0.3 × 10⁴) to medium-high expression (0.3 × 10⁴ - 10⁴) (see Example 1.1).

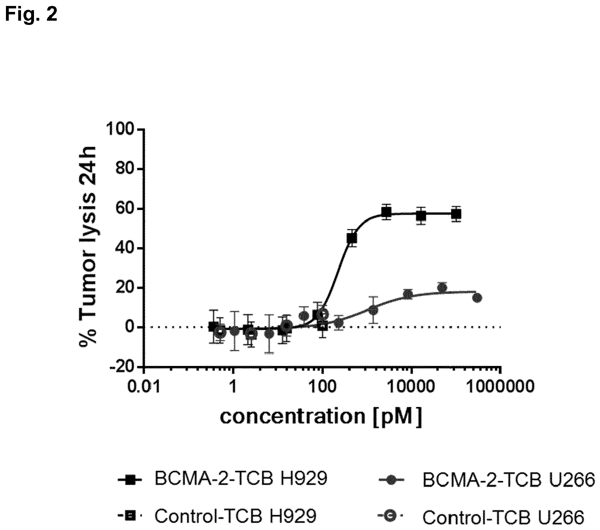

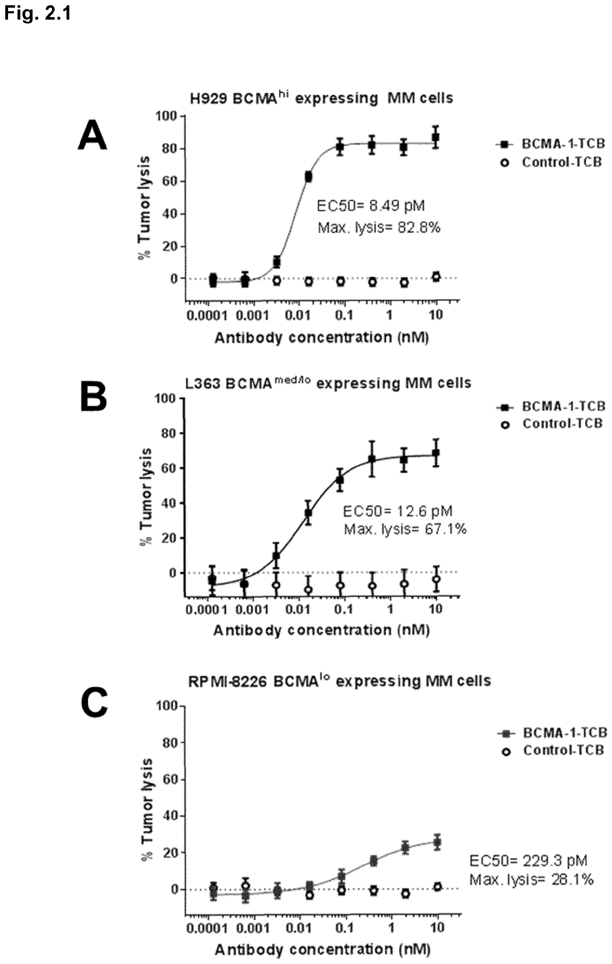

Figure 2. Killing potency of BCMA-TCB is influenced by BCMA expression on the surface of target cells: BCMA^{hi}-expressing H929 vs. BCMA^{med/lo}-expressing U266 myeloma cells. BCMA-2-TCB induced killing of BCMA^{hi}-expressing H929 myeloma cells with an EC50 of 115 pM and maximum killing of 60%, while the same BCMA-TCB antibody was only able to kill BCMA^{med/lo}-expressing U266 myeloma target cells with an EC50 of 370 pM and maximum killing at 18% when performed in a head-to-head comparison (see Example 1.3).
Figure 2.1. The potency of BCMA-1-TCB to induce killing of BCMA expressing myeloma cell lines (BCMA^{hi}-expressing H929, BCMA^{mid/lo}-expressing L363 and BCMA^{lo}-expressing RPMI-8226 MM cells) was tested and compared. BCMA-1- TCB induced killing of (A) BCMA^{hi}-expressing H929 myeloma cells with an EC50 of 8.49 pM and maximum killing of 82.8%, while the same BCMA-1-TCB antibody was only able to kill (B) BCMA^{med/lo}-expressing L363 myeloma target cells with an EC50 of 12.6 pM and maximum killing at 67.1% or (C) BCMA^{lo}-expressing RPMI-8226 with an EC50 of 229.3 pM and maximum killing at 28.1% when performed in a head-to-head comparison (Example 1.3).

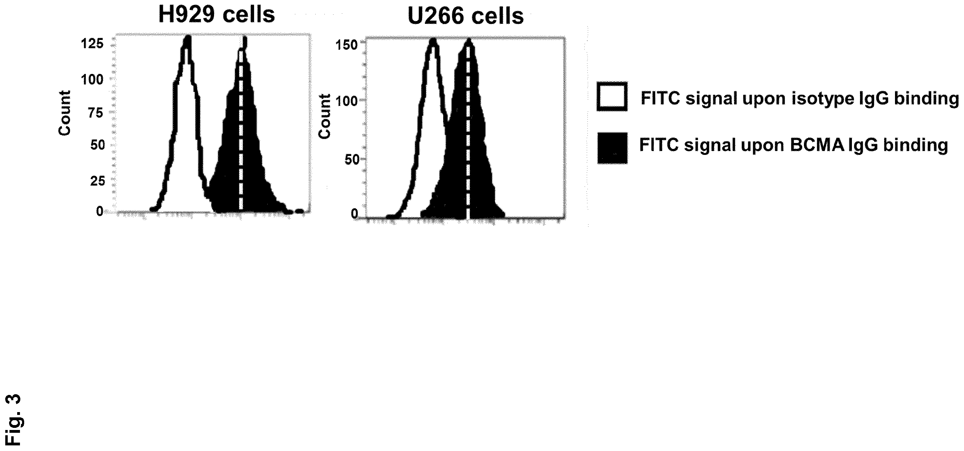

Figure 3. BCMA expression on human myeloma cell lines as detected by flow cytometry and defined by relative mean or median fluorescence intensity.

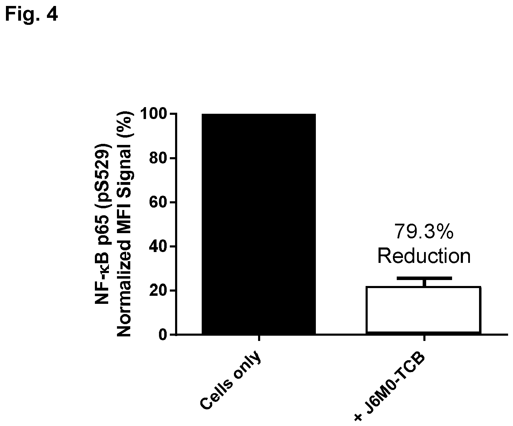

Figure 4. Effect of APRIL-competing BCMA-TCB antibody on APRIL-induced NF-κB activation as detected by phosphoflow cytometry. (A) Effect of APRIL competing J6M0-TCB on APRIL (1000 ng/mL) mediated NF-κB activation in H929 cells. Detection of intracellular phosphorylated NF-κB by phosphoflow cytometry (see Example 4.2.1).

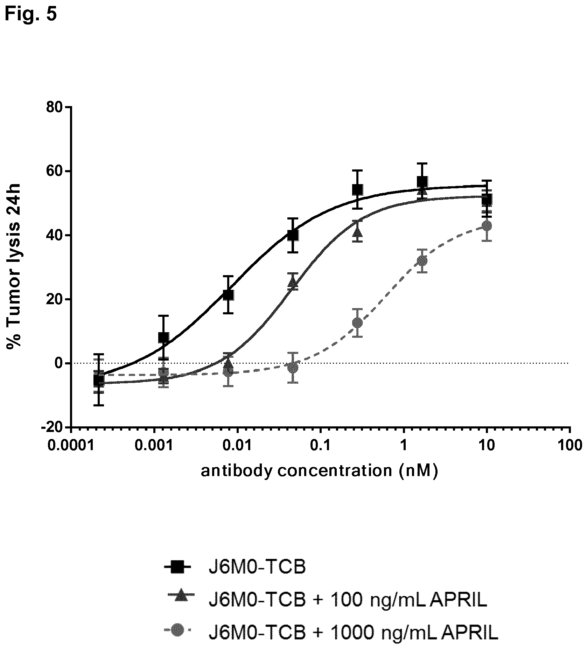

Figure 5. Influence of soluble APRIL on APRIL-competing BCMA-TCB antibody to induce T-cell redirected killing of BCMA-positive H929 myeloma cells as detected by colorimetric LDH release assay. APRIL blocking/competing J6M0-TCB in absence of exogenous soluble APRIL and in presence of 100 ng/mL or 1000 ng/mL of exogenous soluble APRIL. E:T ratio used as 10 PBMCs:1 H929 cell; cells were incubated for 24h before measurement of LDH release. APRIL blocking/competing J6M0-TCB induced a concentration-dependent killing of BCMA-positive H929 myeloma with a low picomolar potency (EC50_{APRIL0}= 5.8 pM) in the absence of exogenous APRIL. When 100 ng/mL of APRIL was added into the culture, such concentration of ligand only minimally affected the killing potency mediated by J6M0-TCB as shown with an 2.4-fold increase in the EC50 (EC50_{APRIL100}= 14.2 pM). However, when 1000 ng/mL of APRIL was added into the culture the killing potency mediated by J6M0-TCB was greatly reduced as reflected by an increase in the EC50 of 84.3-fold (EC50_{APRIL1000}= 488.9 pM) (see Example 4.2.2).

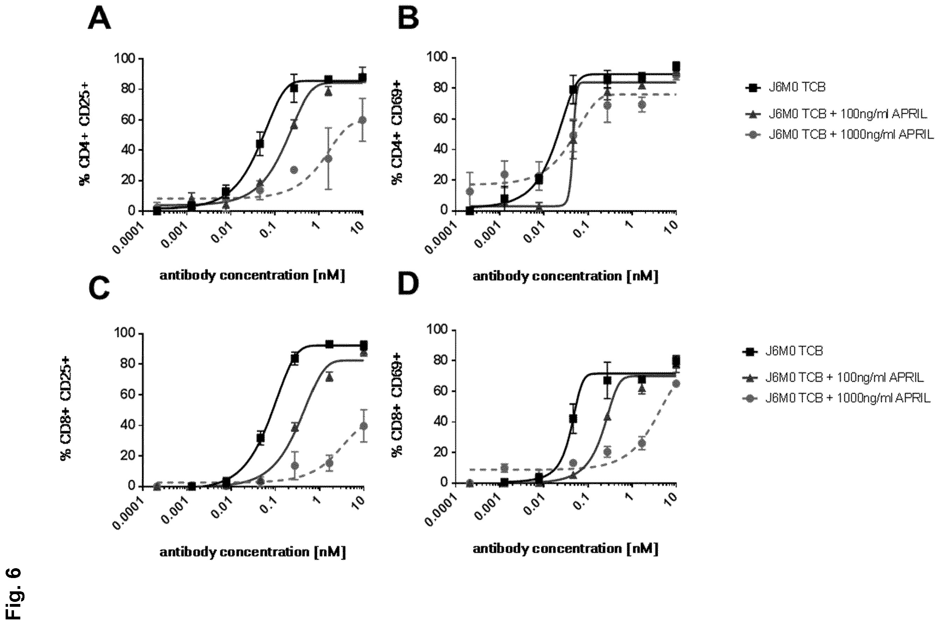

Figure 6. Influence of soluble APRIL on APRIL-competing BCMA-TCB antibody to induce T-cell activation as detected by flow cytometry. Expression level of the early activation marker CD69 (B, D), and the late activation marker CD25 (A, C) on CD4⁺ and CD8⁺ T cells after 48 hours of incubation (representative results from two independent experiments). APRIL-competing/blocking J6M0-TCB antibody induced an up-regulation of CD69 and CD25 activation markers in a concentration-dependent and specific manner in the presence of BCMA-positive target cells in absence of exogenous soluble APRIL (squares). When 100 ng/mL of soluble APRIL was added into the culture, a slight shift to the right of the concentration-response curves was observed for both activation markers CD69 and CD25 on CD4⁺ and CD8⁺ T cells. When 1000 ng/mL of soluble APRIL was added into the culture, there was a clear reduction of T-cell activation on both CD4⁺ and CD8⁺ T cells. No activation of CD4⁺ and CD8⁺ T cells was observed when human PBMCs were treated with DP47-TCB control antibody, suggesting that despite binding to CD3 on the T cells T-cell activation does not occur when the TCB antibody does not bind to BCMA-positive target cells (data not shown). The results clearly suggest that high levels of soluble APRIL reduce the potency of BCMA-TCB antibodies to induce T-cell activation upon binding to the tumor target and T cells, especially when the BCMA-TCB is competes with APRIL (see Example 4.3).

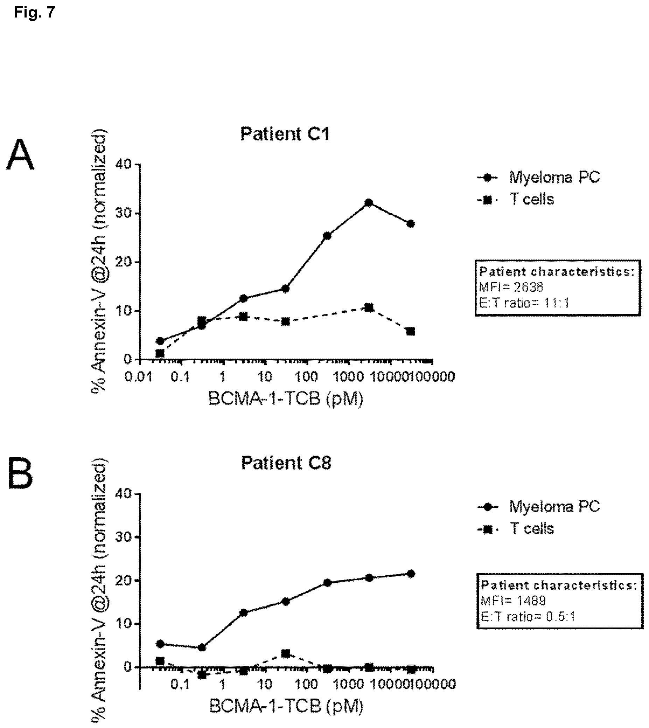

Figure 7: Influence of BCMA expression and E:T ratio on the potency of BCMA-TCB to induce killing of patient bone marrow malignant plasma cells by autologous marrow infiltrating T cells. BCMA-1-TCB induced a concentration dependent specific killing of malignant plasma cells from both patient C1 (A) and patient C8 (B) already after only 24h of incubation. However, killing of myeloma cells was more pronounced in patient C1 bone marrow samples than in patient C8 bone marrow samples. This could be attributed to a more favorable E:T ratio of 11:1 and BCMA expression (i.e. relative MFI value of 2636) in patient C1 bone marrow samples than in patient C8 bone marrow samples with an unfavorable E:T ratio of 0.5:1 and weaker BCMA expression on myeloma cells (i.e. relative MFI value of 1489). The results suggest that measurement of BCMA expression on malignant plasma cells in combination with a measurement of E:T ratio in patient bone marrow may more accurately predict whether myeloma patients may respond to BCMA-TCB treatment.

### Detailed Description of the Invention

The inventors have recognized that disorders involving plasma cells, especially, multiple myeloma, systemic lupus erythematosus, and/or rheumatoid arthritis can be classified (divided) in several subtypes. Such subtypes are:
1. Patients, comprising CD138+ CD38+ cells in an isolated body fluid sample, characterized by BCMA expression on said CD138+ CD38+ cells, measured by using an anti-BCMA antibody with a Kd value, which is 0.70 to 1.3 fold of the Kd value of the anti-BCMA antibody part of said bispecific antibody, is 80 or more, 100 or more, preferably 200 or more, even more preferably 300 or more over baseline determined as Relative Median or Mean Fluorescence Intensity MFI.
2. Patients for whom in an isolated body fluid samplethe ratio of T cells (effector cells) to target cells (E:T ratio) in an isolated body fluid sample is 0.35 : 1 or higher, preferably 0.5 : 1 or higher.
3. Patients, for whom the amount of soluble BCMA in an isolated body fluid sample is 2.5 ng/mL or higher.
4. Patients, for whom the amount of APRIL in an isolated body fluid sample is more than 100 ng/mL.
4. Patients, comprising CD138+ CD38+ cells in an isolated body fluid, characterized by BCMA expression on said CD138+ CD38+ cells, measured by using an anti-BCMA antibody with a Kd value, which is 0.70 to 1.3 fold of the Kd value of the anti-BCMA antibody part of said bispecific antibody, is 80 or more, preferably 100 or more, preferably 200 or more, even more preferably 300 or more over baseline determined as Relative Median or Mean Fluorescence Intensity MFI and for whom in said isolated body fluid sample the ratio of T cells (effector cells) to target cells (E:T ratio) sample is 0.35 : 1, preferably 0.5 : 1 or higher.

BCMA receptor plays a critical role for the survival of normal and malignant plasma cells (i.e. myeloma cells) by binding to its ligands APRIL and BAFF which are abundant in the bone marrow of myeloma patients and BCMA expression in myeloma cells have been detected by many groups both at the mRNA level and surface protein level (O'Connor et al. J Exp Med 2004, 199(1):91-8; Novak et al. Blood 2004, 103(2): 689-94; Ryan et al. Mol Cancer Ther 2007, 6(11): 3009-18; Quinn et al. Blood 2011, 117(3): 890; Carpenter et al. Blood 2013, 19(8): 2048-60; Frigyesi et al. Blood 2014, 123(9):1336-40; Claudio et al. Blood 2002,100(6):2175-86; Tai et al. Cancer Res 2006, 123(20):3128-38; Moreaux et al., Blood 2004,103(8):3148-57; Ju et al. Clin Biochem 2009,42(4-5):387-99; Moreaux et al. Eur J Haematol 2009, 83(2):119-29). Therefore in myeloma patients BCMA is expressed on their malignant plasma cells. However the inventors have observed that myeloma patients do express BCMA on the cell surface but at different level of expression, ranging from medium/high to moderate to low, as detected by optimal measurement by flow cytometry, that the inventors have recognized that the use of suboptimal techniques or methods for detection of BCMA expression for patient stratification (e.g. use of BCMA antibody with low affinity binding to human BCMA) could not detect the low expression of BCMA on malignant plasma cells of myeloma patients and in such case misinform clinicians that these myeloma patients would not respond to a BCMA antibody therapy while they could.

T cell bispecific (TCB) antibodies have very high concentration/tumor-cell-receptor-occupancy dependent potency in cell killing (e.g. EC50 in in vitro cell killing assays in the sub- or low picomolar range; Dreier et al. Int J Cancer 2002), T-cell bispecific antibodies (TCB) are given at much lower doses than conventional monospecific antibodies. For example, blinatumomab (CD19xCD3) is given at a continuous intravenous dose of 5 to 15 µg/m²/day (i.e. only 0.035 to 0.105 mg/m²/week) for treatment of acute lymphocytic leukemia or 60 µg/m²/day for treatment of Non Hodgkin Lymphoma, and the serum concentrations at these doses are in the range of 0.5 to 4 ng/mL (Klinger et al., Blood 2012; Topp et al., J Clin Oncol 2011; Goebeler et al. Ann Oncol 2011). Because low doses of TCB can exert high efficacy in patients, it is envisaged that for an antibody according to the invention subcutaneous administration is possible and preferred in the clinical settings (preferably in the dose range of 0.25 to 2.5 mg/m²/week). Even at these low concentrations/doses/receptor occupancies, TCB can cause considerable adverse events (Klinger et al., Blood 2012). Therefore it is critical to control tumor cell occupancy/coverage. Therefore the doses for treatment with a TCB should be chosen based on an effective method for patient classification.

Therefore, an optimal determination method for BCMA expression on patient myeloma cells is needed. Such optimal determination method for BCMA expression can be performed using flow cytometry with appropriate BCMA antibodies for determination. For example, for use of BCMA determination on myeloma cells for patient stratification, according to the invention, a BCMA antibody is used for detection that has similar affinity range to human BCMA (e.g. as measured by surface plasmon resonance (SPR)) as the BCMA antibody therapy. Further preferred is that the antibody valence should be the same between the BCMA antibody for detection and the BCMA antibody therapy (e.g. a monovalent antibody for BCMA detection should be used for patient stratification for a BCMA antibody therapy with monovalent binding to the tumor target on malignant cells such as in the case of scFV-based BiTE molecules). Further preferred is that the avidity range (as measured by SPR) is similar between the BCMA antibody for detection and the BCMA antibody therapy. Further preferred is to use a BCMA antibody for determination which is the same as the BCMA binder of the BCMA antibody therapy.

The term "target" as used herein means either BCMA or CD3.The term "first target and second target" means either CD3 as first target and BCMA as second target or means BCMA as first target and CD3 as second target.

The term "BCMA" as used herein relates to human B cell maturation target, also known as BCMA; TR17_HUMAN, TNFRSF17 (UniProt Q02223), which is a member of the tumor necrosis receptor superfamily that is preferentially expressed in differentiated plasma cells. The extracellular domain of BCMA consists according to UniProt of amino acids 1-54 (or 5-51). The term "antibody against BCMA, anti BCMA antibody" as used herein relates to an antibody specifically binding to BCMA.

The term "CD3ε or CD3" as used herein relates to human CD3ε described under UniProt P07766 (CD3E_HUMAN). The term "antibody against CD3, anti CD3 antibody" relates to an antibody binding to CD3ε. Preferably the antibody comprises a variable domain VH comprising the heavy chain CDRs of SEQ ID NO: 3, 4 and 5 as respectively heavy chain CDR1, CDR2 and CDR3 and a variable domain VL comprising the light chain CDRs of SEQ ID NO: 6, 7 and 8 as respectively light chain CDR1, CDR2 and CDR3. Preferably the antibody comprises the variable domains of SEQ ID NO:1 (VH) and SEQ ID NO:2 (VL). The term "antibody against CD3, anti CD3 antibody" as used herein relates to an antibody specifically binding to CD3ε.

"Specifically binding to CD3 or BCMA or to CD3 ε or BCMA" refer to an antibody that is capable of binding to the human CD3ε or the extracellular domain of human BCMA (the targets) with sufficient affinity such that the antibody is useful as a therapeutic agent in targeting CD3 or BCMA. In some embodiments, the extent of binding of an anti-CD3 or BCMA antibody to an unrelated, non-CD3 or non-BCMA protein is about 10-fold preferably >100-fold less than the binding of the antibody to CD3 or BCMA as measured, e.g., by surface plasmon resonance (SPR) e.g. Biacore^{®}, enzyme-linked immunosorbent (ELISA) or flow cytometry (FACS). Preferably the antibody that binds to CD3 or BCMA has a dissociation constant (Kd) of 10⁻⁸ M or less, preferably from 10⁻⁸ M to 10⁻¹³ M, preferably from 10⁻⁹ M to 10⁻¹³ M. Preferably the anti-CD3 and/or anti-BCMA antibody binds to an epitope of CD3 and/or BCMA that is conserved among CD3 and/or BCMA from different species, preferably among human and cynomolgus. "Bispecific antibody specifically binding to CD3 and BCMA" or "antibody according to the invention" refers to a respective definition for binding to both targets. An antibody specifically binding to BCMA (or BCMA and CD3) does not bind to other human antigens. Therefore in an ELISA, OD values for such unrelated targets will be equal or lower to that of the limit of detection of the specific assay, preferably > 0.3 ng/mL, or equal or lower to OD values of control samples without plate-bound-BCMA or with untransfected HEK293 cells.

The term "CD3ε or CD3 binding part" as used herein relates to the combination of an antibody heavy chain consisting of VH and CH1 and an antibody light chain consisting of VL and CL as enclosed in a Fab fragment of an antibody specifically binding to CD3.

The term "BCMA binding part" as used herein relates to the combination of an antibody heavy chain consisting of VH and CH1 and an antibody light chain consisting of VL and CL as enclosed in a Fab fragment of an antibody specifically binding to BCMA.

The term "bispecific antibody specifically binding to BCMA and CD3 or TCB or antibody against BCMA and CD3" relates to a bispecific antibody specifically binding to the extracellular domain of human BCMA and human CD3ε. Such antibody can be monovalent for BCMA, e.g. as single chain antibody as mentioned in WO2013072406, WO2013072415 and WO2014140248, or can be bi- or trivalent as disclosed e. g. in WO2014122143, WO2014122144. WO2013072406 and WO2014140248 mention E:T ratios in some figures and examples; however it is only reported that in the respective killing assay experiments there were used 10 effector cells for 1 target cell (cell lines not patient samples). This E:T ratio is therefore artificial and there were not shown any E:T ratios in myeloma patient bone marrow samples or given any hint on the relation of E:T ratio to antibody treatment. Preferably the bispecific antibody comprises as CDRs of the CD3 binding part the CDRs of SEQ ID NO: 2 to 4 and 6 to 8 and preferably the VL and VH domains of SEQ ID NO: 1 and 5. Preferably the bispecific antibody comprised as CDRs of the BCMA binding part the CDRs or preferably the VH and VL domains listed in Table 1. Preferably the bispecific antibody comprises as CDRs of the BCMA binding part the CDRs of SEQ ID NO: 10 to 12 and 14 to 16 or preferably the VL and VH domains of SEQ ID NO: 9 and 13. Preferably the bispecific antibody comprises as CDRs of the BCMA binding part the CDRs of SEQ ID NO: 18 to 20 and 22 to 24 or preferably the VL and VH domains of SEQ ID NO: 17 and 21. Antibody J6M0 is described in WO2012163805. A TCB comprising J6M0 comprises as CDRs of the CD3 binding part the CDRs of SEQ ID NO: 2 to 4 and 6 to 8 and preferably the VL and VH domains of SEQ ID NO: 1 and 5.

The term "an APRIL non-competitive bispecific antibody" relates to a bispecific antibody, characterized in that the binding of said antibody is not reduced by 100 ng/mL APRIL for more than 20% measured in an ELISA assay compared to the binding of said antibody to human BCMA without APRIL. The term "an APRIL non-competitive anti-BCMA antibody" relates to an anti-BCMA antibody, characterized in that the binding of said antibody is not reduced by 100 ng/mL APRIL for more than 20% measured in an ELISA assay compared to the binding of said antibody to human BCMA without APRIL. Such antibodies are described in WO2014122143, WO2014122144, EP14179705 and EP14194151.

**Table 1**

| Antibody | SEQ ID NO: | | | | | | | |
|---|---|---|---|---|---|---|---|---|
| | VL | CDRL1 | CDRL2 | CDRL3 | VH | CDRH1 | CDRH2 | CDRH3 |
| CH2527 (CD3) | 1 | 2 | 3 | 4 | 5 | 6 | 7 | 8 |
| 83A10 (BCMA) | 9 | 10 | 11 | 12 | 13 | 14 | 15 | 16 |
| pSCHLI372 (BCMA) | 17 | 18 | 19 | 20 | 21 | 22 | 23 | 24 |

The term "therapeutic antibody" refers to a bispecific antibody specifically binding to BCMA and CD3 that functions in depleting malignant plasma cells in a patient suffering from multiple myeloma. The therapeutic antibody mediates a cytotoxic effect or cell lysis, particularly by inducing T-cell activation followed by T-cell mediated apoptosis involving perforin and granzyme B.

Preferably a therapeutic antibody according to the invention is characterized in showing an EC50 value for binding to NCI-H929 cells (ATCC^{®} CRL-9068^{™}) of 500 nM or lower, preferably an EC50 value of 350 nM and lower, preferably an EC50 value of 100 nM and lower.

Preferably, a therapeutic antibody according to this invention is characterized by its capability to bind to U266 (ATCC^{®} TIB-196^{™}) cells.

In one preferred embodiment, a therapeutic antibody according to the invention is characterized by its capability to bind to human T cells.

Preferably, a therapeutic antibody according to this invention is characterized by its capability to bind to cynomolgus monkey BCMA transiently expressed on HEK-cells.

In a preferred embodiment, a therapeutic antibody according to this invention is characterized by its capability to induce CD4⁺ and CD8⁺ T-cell activation in the presence of tumor cells expressing BCMA.

Preferably a therapeutic antibody according to the invention is characterized by its capability to induce redirected killing of NCI-H929 tumor cells in the presence of human T cells with an EC50 lower than 1 nM, preferably 0.5 nM, preferably 0.1 nM and lower.

The term "diagnostic antibody" refers to an antibody specifically binding to the extracellular domain of BCMA. According to the invention said diagnostic antibody is, if cell-surface BCMA expression will be determined, an anti-BCMA antibody with a Kd value, which is 0.70 to 1.3 fold of the Kd value of the anti-BCMA antibody part of the therapeutic bispecific antibody intended to use for the treatment of the patient. Preferably the antibody is derived from the BCMA binding part of said therapeutic bispecific antibody and preferably comprised the same CDRs or VH and VL domains as said BCMA binding part of said therapeutic antibody. According to the invention said diagnostic antibody is, if MFI is determined, preferably a labeled antibody. According to the invention said diagnostic antibody is, if soluble BCMA will be determined an antibody useful for ELISA.

The terms "labeled antibody" refers to an antibody, having attached a detectable label. Preferably the detectable label is a fluorophore when FACS is used for analyzing. For other analyzing methods also all other known labels can be used (see, for example, Harlow and Lane, eds. (Antibodies: A Laboratory Manual (1988) Cold Spring Harbor Laboratory Press, Cold Spring Harbor, N.Y.)). Example for preferred fluorophores are fluorescein isothiocyanate (FITC), Alexa Fluor, R-phycoerythrin (PE), Allophycocyanin (APC), PerCP, tandem conjugates (e.g. PE-Cyanine, PerCP-Cyanine), rhodamine (tetramethyl rhodamine isothiocyanate, TRITC), green fluorescent proteins (GFPs), and phycobiliproteins.

The term "antibody" as used herein refers to a monoclonal antibody. An antibody consists of two pairs of a "light chain" (LC) and a "heavy chain" (HC) (such light chain (LC) /heavy chain pairs are abbreviated herein as LC/HC). The light chains and heavy chains of such antibodies are polypeptides consisting of several domains. Each heavy chain comprises a heavy chain variable region (abbreviated herein as HCVR or VH) and a heavy chain constant region. The heavy chain constant region comprises the heavy chain constant domains CH1, CH2 and CH3 (antibody classes IgA, IgD, and IgG) and optionally the heavy chain constant domain CH4 (antibody classes IgE and IgM). Each light chain comprises a light chain variable domain VL and a light chain constant domain CL. The variable domains VH and VL can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The "constant domains" of the heavy chain and of the light chain are not involved directly in binding of an antibody to a target, but exhibit various effector functions.

The "light chain of an antibody" as used herein is a polypeptide comprising in N-terminal to C-terminal direction a light chain variable domain (VL), and a light chain constant domain (CL), abbreviated as VL-CL. "The "heavy chain of an antibody" as used herein is a polypeptide comprising in N-terminal to C-terminal direction a heavy chain variable domain (VH) and a constant heavy chain domain 1 (CH1).

The term "antibody" includes e.g. mouse antibodies, human antibodies, chimeric antibodies, humanized antibodies and genetically engineered antibodies (variant or mutant antibodies) as long as their characteristic properties are retained. Especially preferred are human or humanized antibodies, especially as recombinant human or humanized antibodies. The terms "monoclonal antibody" or "monoclonal antibody composition" as used herein refer to a preparation of antibody molecules of a single amino acid composition.

The terms "bispecific antibody" and "antibody according to the invention" as used herein refer to an antibody in which one of the two pairs of heavy chain and light chain (HC/LC) is specifically binding to BCMA and the other one is specifically binding to CD3 or preferably to CD3 and BCMA. The term "valent" as used within the current application denotes the presence of a specified number of binding sites in an antibody molecule. A bivalent antibody according to this invention has two binding sites, one for CD3 and the other for BCMA. As such, the term "trivalent", denote the presence of three binding sites in an antibody according to the invention, which are two binding sites for BCMA and one binding site for CD3.

There are five types of mammalian antibody heavy chains denoted by the Greek letters: α, δ, ε, γ, and µ (Janeway CA, Jr et al (2001). Immunobiology. 5th ed., Garland Publishing). The type of heavy chain present defines the class of antibody; these chains are found in IgA, IgD, IgE, IgG, and IgM antibodies, respectively (Rhoades RA, Pflanzer RG (2002). Human Physiology, 4th ed., Thomson Learning). Distinct heavy chains differ in size and composition; α and γ contain approximately 450 amino acids, while µ and ε have approximately 550 amino acids. Each heavy chain has two regions, the constant region and the variable region. The constant region is identical in all antibodies of the same isotype, but differs in antibodies of different isotype. Heavy chains γ, α and δ have a constant region composed of three constant domains CH1, CH2, and CH3 (in a line) , and a hinge region for added flexibility (Woof J, Burton D Nat Rev Immunol 4 (2004) 89-99); heavy chains µ and ε have a constant region composed of four constant domains CH1, CH2, CH3, and CH4 (Janeway CA, Jr et al (2001). Immunobiology. 5th ed., Garland Publishing). The variable region of the heavy chain differs in antibodies produced by different B cells, but is the same for all antibodies produced by a single B cell or B cell clone. The variable region of each heavy chain is approximately 110 amino acids long and is composed of a single antibody domain.

In mammals there are two types of light chain, which are called lambda (λ) and kappa (κ). A light chain has two successive domains: one constant domain CL and one variable domain VL. The approximate length of a light chain is 211 to 217 amino acids. Preferably the light chain is a kappa (κ) light chain, and the constant domain CL is preferably derived from a kappa (K) light chain (the constant domain CK). Preferably the heavy and light chain constant domains of the antibody according to the invention are human domains.

The "antibodies" according to the invention can be of any class (e.g. IgA, IgD, IgE, IgG, and IgM, preferably IgG or IgE), or subclass (e.g., IgG1, IgG2, IgG3, IgG4, IgA1 and IgA2, preferably IgG1), whereby both antibodies, from which the bivalent bispecific antibody according to the invention is derived, have an Fc part of the same subclass( e.g. IgG1, IgG4 and the like, preferably IgG1), preferably of the same allotype (e.g. Caucasian).

A "Fab fragment of an antibody" as used herein is a fragment on an antibody that binds to antigens. A Fab fragment of an antibody consists of two pairs of domains. In a wild-type antibody it is composed of one constant and one variable domain of each of the heavy chain (CH1 and VH) and the light chain (CL and VL). In a wild-type antibody and according to the invention the domain of the heavy and light chain domain pairs of a Fab fragment are not chemically linked together and are therefore not scFvs (single chain variable fragments).

A "Fc part of an antibody" is a term well known to the skilled artisan and defined on the basis of papain cleavage of antibodies. The antibodies according to the invention contain as Fc part, preferably a Fc part derived from human origin and preferably all other parts of the human constant regions. The Fc part of an antibody is directly involved in complement activation, C1q binding, C3 activation and Fc receptor binding. While the influence of an antibody on the complement system is dependent on certain conditions, binding to C1q is caused by defined binding sites in the Fc part. Such binding sites are known in the state of the art and described e.g. by Lukas, TJ., et al., J. Immunol. 127 (1981) 2555-2560; Brunhouse, R., and Cebra, J.J., MoI. Immunol. 16 (1979) 907-917; Burton, D.R., et al., Nature 288 (1980) 338-344; Thommesen, J.E., et al., MoI. Immunol. 37 (2000) 995-1004; Idusogie, E.E., et al., J. Immunol. 164 (2000) 4178-4184; Hezareh, M., et al., J. Virol. 75 (2001) 12161-12168; Morgan, A., et al., Immunology 86 (1995) 319-324; and EP 0 307 434. Such binding sites are e.g. L234, L235, D270, N297, E318, K320, K322, P331 and P329 (numbering according to EU index of Kabat, see below). Antibodies of subclass IgG1, IgG2 and IgG3 usually show complement activation, C1q binding and C3 activation, whereas IgG4 do not activate the complement system, do not bind C1q and do not activate C3. Preferably the Fc part is a human Fc part. Preferably the Fc part is a human IgGlFc part. Preferably the antibody according to the invention comprises in the human IgG1 Fc part amino acid substitution of Pro329 with glycine or arginine and/or substitutions L234A and L235A, preferably Pro329 with glycine and substitutions L234A and L235A.

Preferably the antibody according to the invention comprises as Fc part an Fc variant of a wild-type human IgG Fc region, said Fc variant comprising an amino acid substitution at position Pro329 and at least one further amino acid substitution, wherein the residues are numbered according to the EU index of Kabat, and wherein said antibody exhibits a reduced affinity to the human FcγRIIIA and/or FcγRIIA and /or FcγRI compared to an antibody comprising the wildtype IgG Fc region, and wherein the ADCC induced by said antibody is reduced to at least 20% of the ADCC induced by the antibody comprising a wild-type human IgG Fc region. In a specific embodiment Pro329 of a wild-type human Fc region in the antibody according to the invention is substituted with glycine or arginine or an amino acid residue large enough to destroy the proline sandwich within the Fc/Fcy receptor interface, that is formed between the proline329 of the Fc and tryptophane residues Trp 87 and Tip 110 of FcyRIII (Sondermann et al.: Nature 406, 267-273 (20 July 2000)). In a further aspect of the invention the at least one further amino acid substitution in the Fc variant is S228P, E233P, L234A, L235A, L235E, N297A, N297D, or P331S and still in another embodiment said at least one further amino acid substitution is L234A (denotes that leucine 234 is substituted by alanine) and L235A of the human IgG1 Fc region or S228P and L235E of the human IgG4 Fc region. Such Fc variants are described in detail in WO2012130831. An advantage of an antibody according to the invention comprising an Fc part, is that the elimination half-life is increased up to ~12 days or even more and offers the opportunity of once or twice/week administrations as compared to TCBs without an Fc portion.

The term "chimeric antibody" refers to an antibody comprising a variable region, i.e., binding region, from one source or species and at least a portion of a constant region derived from a different source or species, usually prepared by recombinant DNA techniques. Chimeric antibodies comprising a murine variable region and a human constant region are preferred. Other preferred forms of "chimeric antibodies" encompassed by the present invention are those in which the constant region has been modified or changed from that of the original antibody to generate the properties according to the invention, especially in regard to C1q binding and/or Fc receptor (FcR) binding. Such chimeric antibodies are also referred to as "class-switched antibodies". Chimeric antibodies are the product of expressed immunoglobulin genes comprising DNA segments encoding immunoglobulin variable regions and DNA segments encoding immunoglobulin constant regions. Methods for producing chimeric antibodies involve conventional recombinant DNA and gene transfection techniques are well known in the art. See, e.g., Morrison, S.L., et al., Proc. Natl. Acad. Sci. USA 81 (1984) 6851-6855; US Patent Nos. 5,202,238 and 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody." See, e.g., Riechmann, L., et al., Nature 332 (1988) 323-327; and Neuberger, M.S., et al., Nature 314 (1985) 268-270. Particularly preferred CDRs correspond to those representing sequences recognizing the targets noted above for chimeric antibodies. Other forms of "humanized antibodies" encompassed by the present invention are those in which the constant region has been additionally modified or changed from that of the original antibody to generate the properties according to the invention, especially in regard to C1q binding and/or Fc receptor (FcR) binding.

The term "human antibody", as used herein, is intended to include antibodies having variable and constant regions derived from human germ line immunoglobulin sequences. Human antibodies are well-known in the state of the art (van Dijk, M.A., and van de Winkel, J.G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production. Transfer of the human germ-line immunoglobulin gene array in such germ-line mutant mice will result in the production of human antibodies upon target challenge (see, e.g., Jakobovits, A., et al., Proc. Natl. Acad. Sci. USA 90 (1993) 2551-2555; Jakobovits, A., et al., Nature 362 (1993) 255-258; Bruggemann, M., et al., Year Immunol. 7 (1993) 33-40). Human antibodies can also be produced in phage display libraries (Hoogenboom, H.R., and Winter, G., J. MoI. Biol. 227 (1992) 381-388; Marks, J.D., et al., J. MoI. Biol. 222 (1991) 581-597). The techniques of Cole et al. and Boerner et al. are also available for the preparation of human monoclonal antibodies (Cole et al., Monoclonal Antibodies and Cancer Therapy, Alan R. Liss, p. 77 (1985); and Boerner, P., et al., J. Immunol. 147 (1991) 86-95). As already mentioned for chimeric and humanized antibodies according to the invention the term "human antibody" as used herein also comprises such antibodies which are modified in the constant region to generate the properties according to the invention, especially in regard to C1q binding and/or FcR binding, e.g. by "class switching" i.e. change or mutation of Fc parts (e.g. from IgG1 to IgG4 and/or IgG1/IgG4 mutation).

The term "recombinant human antibody", as used herein, is intended to include all human antibodies that are prepared, expressed, created or isolated by recombinant means, such as antibodies isolated from a host cell such as a NSO or CHO cell or from an animal (e.g. a mouse) that is transgenic for human immunoglobulin genes or antibodies expressed using a recombinant expression vector transfected into a host cell. Such recombinant human antibodies have variable and constant regions in a rearranged form. The recombinant human antibodies according to the invention have been subjected to in vivo somatic hypermutation. Thus, the amino acid sequences of the VH and VL regions of the recombinant antibodies are sequences that, while derived from and related to human germ line VH and VL sequences, may not naturally exist within the human antibody germ line repertoire in vivo.

The "variable domain" (variable domain of a light chain (VL), variable region of a heavy chain (VH)) as used herein denotes each of the pair of light and heavy chains which is involved directly in binding the antibody to the target. The domains of variable human light and heavy chains have the same general structure and each domain comprises four framework (FR) regions whose sequences are widely conserved, connected by three "hypervariable regions" (or complementarity determining regions, CDRs). The framework regions adopt a β-sheet conformation and the CDRs may form loops connecting the β-sheet structure. The CDRs in each chain are held in their three-dimensional structure by the framework regions and form together with the CDRs from the other chain the target binding site. The antibody heavy and light chain CDR3 regions play a particularly important role in the binding specificity/affinity of the antibodies according to the invention and therefore provide a further object of the invention.

The terms "hypervariable region" or "target-binding region of an antibody" when used herein refer to the amino acid residues of an antibody which are responsible for target-binding. The hypervariable region comprises amino acid residues from the "complementarity determining regions" or "CDRs". "Framework" or "FR" regions are those variable domain regions other than the hypervariable region residues as herein defined. Therefore, the light and heavy chains of an antibody comprise from N- to C-terminus the domains FR1, CDR1, FR2, CDR2, FR3, CDR3, and FR4. CDRs on each chain are separated by such framework amino acids. Especially, CDR3 of the heavy chain is the region which contributes most to target binding. CDR and FR regions are determined according to the standard definition of Kabat et al., Sequences of Proteins of Immunological Interest, 5th ed., Public Health Service, National Institutes of Health, Bethesda, MD (1991).

The term "epitope" includes any polypeptide determinant capable of specific binding to an antibody. In certain embodiments, epitope determinant include chemically active surface groupings of molecules such as amino acids, sugar side chains, phosphoryl, or sulfonyl, and, in certain embodiments, may have specific three dimensional structural characteristics, and or specific charge characteristics. An epitope is a region of a target that is bound by an antibody.

As used herein, "expression" refers to the process by which a nucleic acid is transcribed into mRNA and/or to the process by which the transcribed mRNA (also referred to as transcript) is subsequently being translated into peptides, polypeptides, or proteins. The transcripts and the encoded polypeptides are collectively referred to as gene product. If the polynucleotide is derived from genomic DNA, expression in a eukaryotic cell may include splicing of the mRNA.

The bispecific antibodies according to the invention are preferably produced by recombinant means. Such methods are widely known in the state of the art and comprise protein expression in prokaryotic and eukaryotic cells with subsequent isolation of the antibody polypeptide and usually purification to a pharmaceutically acceptable purity. For the protein expression, nucleic acids encoding light and heavy chains or fragments thereof are inserted into expression vectors by standard methods. Expression is performed in appropriate prokaryotic or eukaryotic host cells like CHO cells, NSO cells, SP2/0 cells, HEK293 cells, COS cells, yeast, or E.coli cells, and the antibody is recovered from the cells (supernatant or cells after lysis).The bispecific antibodies may be present in whole cells, in a cell lysate, or in a partially purified or substantially pure form. Purification is performed in order to eliminate other cellular components or other contaminants, e.g. other cellular nucleic acids or proteins, by standard techniques, including alkaline/SDS treatment, column chromatography and others well known in the art. See Ausubel, F., et al., ed., Current Protocols in Molecular Biology, Greene Publishing and Wiley Interscience, New York (1987).

With the CD19xCD3 T-cell bispecific (TCB) antibody blinatumomab response rates up to 80% have been shown in patients with relapsed/refractory Acute Lymphocytic Leukemia ALL, As for ALL for Multiple Myeloma and other plasma cell diseases there is still a high medical need. Despite all today available treatment, five years after first diagnosis approx 60% of Multiple Myeloma patients already died. There is still a need for an effective treatment for patients with Multiple Myeloma.

The term "a disorder involving plasma cells" refers to a disease with an increase in the serum/plasma level of its corresponding product, the monoclonal immunoglobulin protein (M-protein). M-proteins may consist of both heavy and light chains or of only one type of chain. Plasma cell disorders are multiple myeloma or other B-cell disorders expressing BCMA. Multiple myeloma is a B-cell malignancy characterized by a monoclonal expansion and accumulation of abnormal plasma cells in the bone marrow compartment. Multiple myeloma also involves circulating clonal B cells with same IgG gene rearrangement and somatic hypermutation. Multiple myeloma arises from an asymptomatic, premalignant condition called monoclonal gammopathy of unknown significance (MGUS), characterized by low levels of bone marrow plasma cells and a monoclonal protein. Multiple myeloma cells (MM cells) proliferate at low rate. Multiple myeloma results from a progressive occurrence of multiple structural chromosomal changes (e.g. unbalanced translocations). Multiple myeloma involves the mutual interaction of malignant plasma cells and bone marrow microenvironment (e.g. normal bone marrow stromal cells). Clinical signs of active Multiple myeloma include monoclonal antibody spike, plasma cells overcrowding the bone marrow, lytic bone lesions and bone destruction resulting from overstimulation of osteoclasts (Dimopulos & Terpos, Ann Oncol 2010; 21 suppl 7: vii143-150). Another B-cell disorder involving plasma cells i.e. expressing BCMA is systemic lupus erythematosus (SLE), also known as lupus. SLE is a systemic, autoimmune disease that can affect any part of the body and is represented with the immune system attacking the body's own cells and tissue, resulting in chronic inflammation and tissue damage. It is a Type III hypersensitivity reaction in which antibody-immune complexes precipitate and cause a further immune response (Inaki & Lee, Nat Rev Rheumatol 2010; 6: 326-337).

The invention relates preferably to the therapy of multiple myeloma. The invention relates in a further embodiment also to the therapy of other B-cell disorders involving plasma cells. Such a disorder, wherein plasma cells expressing BCMA are involved, is systemic lupus erythematosus (SLE), also known as lupus. Further disorders, wherein plasma cells expressing BCMA are involved, are disorders involving production of anti-nuclear antibodies (anti-dsDNA antibodies), lupus nephritis, and RA, type 1 autoimmune hepatitis. Plasma cell disorders are also classified according to http://www.merckmanuals.com.

**Table 2**

| Classification of Plasma Cell Disorders | | |
|---|---|---|
| Symptoms | Description | Examples |
| Monoclonal gammopathy of undetermined significance* | | |
| Asymptomatic, usually nonprogressive | Associated with nonlymphoreticular tumors | Carcinomas of the breasts, biliary tree, GI tract, kidneys, and prostate |
| | Associated with chronic inflammatory and infectious conditions | Chronic cholecystitis, osteomyelitis, pyelonephritis, RA, TB |
| Occurring in apparently healthy people | | |
| | Associated with various other disorders | Familial hypercholesterolemia, Gaucher disease, Kaposi sarcoma, lichen myxedematosus, liver disorder, myasthenia gravis, pernicious anemia, thyrotoxicosis |

| Malignant plasma cell disorders | | |
|---|---|---|
| Symptomatic, | Excess production of IgM | [Macroglobulinemia |
| progressive | Most often IgG, IgA, or light chains (Bence Jones) only | Multiple myeloma |
| | Usually light chains (Bence Jones) only, but occasionally intact immunoglobulin molecules (IgG, IgA, IgM, IgD) | Nonhereditary primary systemic amyloidosis |
| | Heavy chain diseases | IgG heavy chain (γ-chain) disease (sometimes benign) |
| | | IgA heavy chain (α-chain) disease |
| | | IgM heavy chain (µ-chain) disease |
| | | IgD heavy chain (δ-chain) disease |
| ^{∗}Age-related incidence. | | |

Multiple Myeloma can be staged according to the International Staging System for Multiple Myeloma (http://www.cancer.org). This system divides myeloma into 3 stages based only on the serum beta-2 microglobulin and serum albumin levels:
Stage I: Serum beta-2 microglobulin is less than 3.5 (mg/L) and the albumin level is above 3.5 (g/L)
Stage II: Neither stage I or III, meaning that either: the beta-2 microglobulin level is between 3.5 and 5.5 (with any albumin level), or the albumin is below 3.5 while the beta-2 microglobulin is less than 3.5.
Stage III: Serum beta-2 microglobulin is greater than 5.5.

However this staging system does not give a hint whether a patient is susceptible for a therapy with a bispecific antibody specifically binding to BCMA and CD3. Also the fact, that BCMA is selectively induced during plasma cell differentiation and expressed at high levels in malignant plasma cells (Ryan, MC et al., Mol. Cancer Ther. 6 (2007) 3009-3018; Novak AJ et al., Blood. 2004 Jan 15;103(2):689-94. Epub 2003 Sep 25.; Maus MV and June CH, Clin Cancer Res 2013;19:2048-60) and therefore patients, expressing BCMA on the surface of their MM cells would be susceptible for a therapy with a bispecific antibody specifically binding to BCMA and CD3 is not sufficient for an effective therapy. The inventors have recognized that MM cells of different patients are differently sensitive to a therapy with a bispecific antibody against CD3 and BCMA. At least one of the following features
- the diagnostic antibody for the measurement for BCMA expression and the therapeutic antibody are related to a certain extent (MFI),
- the E:T ratio for multiple myeloma,
- the amount of soluble BCMA and/or APRIL in a body fluid sample, especially in a bone marrow aspirate sample, if the patient suffers from multiple myeloma and synovial fluid, if the patient suffers from SLE or RA
is of relevance for a successful therapy. Preferably two, three or all four features are combined. Preferably the determination of two, three or all four features are combined for the method of treatment, selecting a therapy, selecting a treatment plan, and predicting the likelihood according to the invention.

The term "standard dose" refers to the FDA approved weekly dose for treatment of the respective plasma cell disorder, especially selected from the group consisting of multiple myeloma, lupus erythematosus and rheumatoid arthritis. If the FDA approved dose differs for different weeks, the tern "standard dose" refers to the dose in the respective week.

The term "at higher doses and/or at a more frequent treatment schedule" means, starting from an acknowledged/approved therapy plan for a prior patient treated with a bispecific antibody specifically binding to BCMA and CD3 (preferably the FDA approved dose), the dose is increased for a factor of 1.5 to 2.0, to 2.0 or even up to a factor of 10 and more and/or the time interval between dose-administrations is shortened from once per week administration to twice per week or even three times a week or even once a day.

In regard to APRIL concentrations above 100 and close to 1000 ng/mL up to a factor of 80 a dose increase is preferred if a APRIL binding to BCMA competing BCMA-T-cell bispecific antibody is used for therapy (see table 9 and figure 5). In a further preferred embodiment the time interval between dose-administrations is shortened from once per week administration to twice per week or even three times a week or even once a day. A preferred therapy plan is one established in patients, which were selected based on that the amount of soluble BCMA was 2.5 ng/mL or lower, and/or APRIL concentration was lower than 100 ng/mL in an isolated body fluid sample of said patients. A preferred therapy for using a bispecific antibody specifically binding to BCMA and CD3 in patients who are above 100 ng/mL of APRIL and/or 2.5 ng/mL of soluble BCMA is to adapt dose or dose interval. This is confirmed by the EC50 values for killing of tumor cells with an APRIL competitive bispecific antibody in Table 9 with a factor of 2.4 at 100 ng/mL APRIL but already a factor of 80 at 1000 ng/mL of APRIL.

The term "treatment plan" refers to a standardized treatment plan. In the context of the present patent application it is about adapting the dose and dosing intervals to the measured parameters BCMA expression (MFI), soluble BCMA, APRIL, and E:T ratio. The guidance given is preferably:
- Do not treat with a TCB if MFI is 50 or less,
- Adapt dose/dose schedule if soluble BCMA above 2.5ng/mL,
- Adapt dose/dose schedule if APRIL above 100 ng/mL or just take a non- competing BCMA-TCB,
- Combine with a T cell expanding/enhancing therapy if E:T is below 0.5:1

The term "selecting a treatment plan that is most effective for a patient which shows MFI of 80 or more, preferably 100 or more", and preferably 200 or more, even more preferably 300 or more over baseline, refers to a method for selecting a treatment plan for a new patient, suffering from a disorder involving plasma cells, comprising:
determining BCMA expression on CD138⁺ CD38⁺ cells of said patient, by using an anti-BCMA antibody with a Kd value, which is 0.70 to 1.3 fold of the Kd value of the anti-BCMA antibody part of said bispecific antibody, and if MFI is found as 80 or more, preferably 100 or more over baseline,
searching, utilizing the result of said determination method for a prior patient suffering from the same disorder with similar (preferably same) representation; and
reviewing the prior treatment plan for the prior patient in order to determine how to improve the treatment of the new patient based on information in the prior treatment plan.

The term "selecting a treatment plan that is most effective for a patient which shows MFI of 80 or more, preferably 100 or more, preferably 200 or more, even more preferably 300 or more over baseline" refers preferably to a method for selecting a treatment plan for a new patient, suffering from a disorder involving plasma cells, comprising:
determining BCMA expression on CD138+ CD38+ cells of said patient, by using an anti-BCMA antibody with a Kd value, which is 0.70 to 1.3 fold of the Kd value of the anti-BCMA antibody part of said bispecific antibody, and if MFI is found as 80 or more, preferably 100 or more over baseline, to treat the patient with BCMA- T-cell bispecific antibody therapy, but to consider not to treat at MFI lower than 100, preferably lower than 50, even preferable lower than 10 over baseline.

The term "selecting a treatment plan that is most effective for a patient which show an E:T ratio of 0.35 : 1, preferably 0.5 : 1 or higher", refers to a method for selecting a treatment plan for a new patient, suffering from a disorder involving plasma cells, comprising:
determining whether the ratio of CD3⁺ cells to CD138⁺ CD38⁺ cells (further named also as E:T ratio) in an isolated body fluid sample of said patient is 0.35 : 1, preferably 0.5 : 1 or higher, and
searching, utilizing the result of said determination method for a prior patient suffering from the same disorder with similar (preferably same) representation; and
reviewing the prior treatment plan for the prior patient in order to determine how to improve the treatment of the new patient based on information in the prior treatment plan.

The term "selecting a treatment plan that is most effective for a patient which show an E:T ratio of 0.35 : 1, preferably 0.5 : 1 or higher", refers preferably to a method for selecting a treatment plan for a new patient, suffering from a disorder involving plasma cells, comprising determining whether the ratio of CD3+ cells to CD138+ CD38+ cells (further named also as E:T ratio) in an isolated body fluid sample of said patient is 0.35 : 1, preferably 0.5 : 1 or higher, and to consider combination with a T-cell proliferative or T cell chemoattractant therapy in case ratio is below 0.35 : 1, preferably 0.5 : 1.

The term "selecting a treatment plan that is most effective for a patient which shows soluble BCMA values of 2.5 ng/mL or higher" refers to a method for selecting a treatment plan for a new patient, suffering from a disorder involving plasma cells, comprising:
determining whether the amount of soluble BCMA in an isolated body fluid sample of said patient is 2.5 ng/mL or higher, and
searching, utilizing the result of said determination method for a prior patient suffering from the same disorder with similar (preferably same) representation; and
reviewing the prior treatment plan for the prior patient in order to determine how to improve the treatment of the new patient based on information in the prior treatment plan.

The term "selecting a treatment plan that is most effective for a patient which shows soluble BCMA values of 2.5 ng/mL or higher" refers preferably to a method for selecting a treatment plan for a new patient, suffering from a disorder involving plasma cells, comprising determining whether the amount of soluble BCMA in an isolated body fluid sample of said patient is 2.5 ng/mL or higher, and to then consider to switch at higher doses and/or at a more frequent treatment schedule.

The term "selecting a treatment plan that is most effective for a patient which show an APRIL value of 100 ng/mL or higher" refers to a method for selecting a treatment plan for a new patient, suffering from a disorder involving plasma cells, comprising:
determining whether the amount of APRIL in an isolated body fluid sample of said patient is 100 ng/mL or higher, and
searching, utilizing the result of said determination method for a prior patient suffering from the same disorder with similar (preferably same) representation; and
reviewing the prior treatment plan for the prior patient in order to determine how to improve the treatment of the new patient based on information in the prior treatment plan.

The term "selecting a treatment plan that is most effective for a patient which show an APRIL value of 100 ng/mL or higher" refers preferably to a method for selecting a treatment plan for a new patient, suffering from a disorder involving plasma cells, comprising determining whether the amount of APRIL in an isolated body fluid sample of said patient is 100 ng/mL or higher, and to then either use a BCMA-T-cell bispecific antibody not competing with APRIL for the binding to BCMA or otherwise to consider to switch at higher doses and/or at a more frequent treatment schedule.

The term "investigation the BCMA related plasma cell status of a patient" relates to the investigation of said plasma cells by one, two, three or all four methods selected from the group consisting of
determining BCMA expression on CD138⁺ CD38⁺ cells of said patient, by using an anti-BCMA antibody with a Kd value, which is 0.70 to 1.3 fold of the Kd value of the anti-BCMA antibody part of said bispecific antibody, and if MFI is found as 80 or more, preferably 100 or more over baseline,
determining whether the ratio of CD3⁺ cells to CD138⁺ CD38⁺ cells (further named also as E:T ratio) in an isolated body fluid sample of said patient is 0.35 : 1, preferably 0.5 : 1 or higher,
determining whether the amount of soluble BCMA in an isolated body fluid sample of said patient is 2.5 ng/mL or higher, and
determining whether the amount of APRIL in an isolated body fluid sample of said patient is 100 ng/mL or higher.

The term "T-cell proliferative therapy" refers to a therapeutic treatment or a biological treatment which induces the proliferation or expansion of T cells such as e.g. recombinant cytokines (e.g. interferons (IFN) IFN-gamma, IFN-alpha; interleukins (IL) IL-1, IL-2, IL-7, IL-9, IL-15, IL-16, IL-17, IL-21), agonistic antibodies against costimulatory molecules, checkpoint inhibitors (e.g. anti-PD-1, anti-PD-L1), preferably the proliferation or expansion of T cells is specific to the tumor site.

The term "T-cell chemoattractant therapy" refers to a therapeutic treatment or a biological treatment which induces the chemotaxis of T cells to the tumor site, such as e.g. chemokines (e.g. CCL1, CCL2, CCL22, CCL17, IP-10).

The term "body fluid sample" refers to an isolated body fluid of a human patient. Preferred body fluids according to the invention are bone marrow aspirate, blood, serum, plasma, urine, saliva, synovial fluid and spinal fluid.

The term "bone marrow aspirate" refers to a sample retrieved by or trephine biopsy. Bone marrow aspiration and trephine biopsy are usually performed on the back of the hipbone, or posterior iliac crest. An aspirate can also be obtained from the sternum (breastbone). Bone marrow aspiration may also be performed on the tibial (shinbone). In case of patients suffering from a disorder involving plasma cells, like malignant cells of multiple myeloma, blood and bone marrow aspirate are the preferred body fluid samples. Especially preferred is to use according to the invention bone marrow aspirate in case of patients suffering from multiple myeloma.

The term "blood sample" refers to a blood sample comprising cells (i.e. erythrocytes (red blood cells), leucocytes (white blood cells), thrombocytes (platelets)) and plasma.

In case of patients suffering from SLE or RA blood samples and preferably synovial fluids (fluid surrounding the inflamed joints) are the preferred body fluid samples used according to the invention.

In case of patients suffering from lupus nephritis and type 1 autoimmune hepatitis blood samples are the preferred body fluid samples used according to the invention.

The term "CD138⁺ CD38⁺ cells" refers to plasma cells in healthy individuals and in patients with multiple myeloma. Because malignant plasma cells are usually found in greater frequency than normal plasma cells in the bone marrow of myeloma patients, CD138⁺ CD38⁺ cells can be considered as myeloma cells of this expression profile when referred to patients with multiple myeloma. CD38 is an antigen expressed on plasma cells. Because plasma cells are the only cells in the bone marrow that express CD138 (i.e. syndecan-1), this marker can be used to identify and isolate this population. Because the immunophenotype of myeloma cells is not significantly different between untreated and treated patients, the CD138 antigen could be used for analysis in both patients groups. Preferably CD138⁺ cells are initially gated followed by subsequent selection using CD38. To distinguish between "normal" plasma cells and "malignant" plasma cells (i.e. myeloma cells), CD56 and CD19 antigens are useful markers to include in the immunophenotypic analysis. From the population of plasma cells identified as CD138⁺ CD38⁺ by flow cytometry, re-gating of cells with CD19⁺ CD56⁻ refers to normal plasma cells and with CD19⁻ CD56⁺ refers to malignant plasma cells- (Rawstron AC, et al. Report of the European Myeloma Network on multiparametric flow cytometry in multiple myeloma and related disorders. Haematologica. 2008;93:431-438, and Tae-Dong Jeong et al., Korean J Hematol. Dec 2012; 47(4): 260-266).

The term "target cell" refer to a cell, expressing BCMA on its surface. In case of the patient is suffering from multiple myeloma, said cell is a multiple myeloma plasma cell. In case of the patient is suffering from SLE or RA said cell is a cell secreting anti-nuclear antibodies, preferably a plasma cell secreting anti-nuclear antibodies. Preferably the target cell is a CD138 + CD38+ cell.

The term "effector cells" refers to cells or a group of cells which can induce cytotoxicity, cell death or apoptosis of tumor cells (e.g. malignant plasma cells or myeloma cells), and refers to peripheral blood mononuclear cells (PBMC), preferably CD3⁺T cells.

The term "CD3⁺ cells or CD3⁺ T cells" refers to cells which are positive for CD3. T cells are also positive for T-cell receptor (TCR) and can also be identified by surface expression of TCR. T cells are also positive for CD45 and negative for CD19 and CD56 and can therefore also be identified by determination of surface expression of CD45 and negative for CD19 (negative) and CD56 (negative). Effector cells can also be identified as CD3⁺ cells, selected from the group consisting of CD3⁺ CD4⁺ helper T cells, CD3⁺ CD8⁺ cytotoxic T cells, CD3⁺ CD45RA⁺ CD197⁻ naive T cells, CD3⁺ CD45RA⁺ CD197⁻ CD4⁺ naive CD4 T cells, CD3⁺ CD45RA⁺ CD197⁻ CD8⁺ naive CD8 T cells, CD3⁺ CD45RA⁻memory T cells, CD3⁺ CD45RA⁻ CD4⁺ memory CD4 T cells, CD3⁺ CD45RA⁻ CD8⁺ memory CD8 T cells, CD3⁺ CD45RA⁻ CD197⁺ central memory T cells, CD3⁺ CD45RA⁻ CD197⁺ CD4⁺ central memory CD4 T cells, CD3⁺ CD45RA⁻ CD197⁺ CD8⁺ central memory CD8, CD3⁺ CD45RA⁻ CD197⁻effector memory T cells, CD3⁺ CD45RA⁻ CD197⁻ CD4⁺ effector memory CD4 T cells; CD3⁺ CD45RA⁻ CD197⁻ CD8⁺ effector memory CD8 T, CD3⁺ CD45RA⁺ CD197⁻ effector T cells, CD3⁺ CD45RA⁺ CD197⁻ CD4⁺ effector CD4 T cells, CD3⁺ CD45RA⁺ CD197⁻ CD8⁺ effector CD8 T cells, CD3⁺ CD4⁺ CD25^{hi} CD127^{lo} regulatory T cells, CD3⁺ PD-1⁻ non-exhausted T cells, and CD3⁺ PD-1⁻ Tim-3⁻ non-exhausted T cells.

The term "baseline determined as Relative Median or Mean Fluorescence Intensity MFI" relates to the baseline defined for the FACS apparatus used for the determination according to the invention. A baseline is defined by MFI of a T-cell as reference.

The term "soluble BCMA" refers to BCMA present in fluid samples of a patient. Preferably an Enzyme-linked immunosorbent assay is used for determination of BCMA concentrations in body fluid samples, preferably serum, preferably plasma. Preferably the assay does not cross react with human APRIL, BAFF, BAFF-R or TACI.

The term "measuring the amount of soluble APRIL" refers preferably to by the use of an ELISA method.

In a further embodiment, the invention relates to a bispecific antibody against CD3ε and BCMA for use in the treatment of autoimmune diseases. The present invention provides methods of determining the responsiveness of a patient to such treatment and related diagnostic assays.

In a further embodiment, the invention relates to a bispecific antibody against CD3ε and BCMA for use in the treatment of multiple myeloma. The present invention provides methods of determining the responsiveness of a patient to such treatment and related diagnostic assays.

All embodiments of the invention relates to the field of therapy and diagnosis of humans.

**In the following specific embodiments of the invention are listed:**
1. A bispecific antibody specifically binding to the extracellular domain of human BCMA (further named also as "BCMA") and human CD3ε (further named also as "CD3"), for use in the treatment of a patient suffering from a disorder involving plasma cells, and whereby in an isolated body fluid sample of said patient, comprising CD138⁺ CD38⁺ cells, BCMA expression on said CD138⁺ CD38⁺ cells, measured by using an anti-BCMA antibody with a Kd value, which is 0.70 to 1.3 fold of the Kd value of the anti-BCMA antibody part of said bispecific antibody, is 100 or more, preferably 200 or more, even more preferably 300 or more over baseline determined as Relative Median or Mean Fluorescence Intensity MFI.
2. The bispecific antibody for use according to embodiment 1, characterized in that said bispecific antibody and said anti-BCMA antibody are monovalent for BCMA binding.
3. The bispecific antibody for use according to embodiment 1, characterized in that said bispecific antibody and said anti-BCMA antibody are bivalent for BCMA binding.
4. The bispecific antibody for use according to embodiment 1, characterized in that said bispecific antibody and said anti-BCMA antibody are trivalent for BCMA binding.
5. The bispecific antibody for use according to any one of embodiments 1 to 4, characterized in that said bispecific antibody comprises as its heavy and light chain CDRs, CDRs of the same amino acid sequences as said anti-BCMA antibody.
6. The bispecific antibody for use according to any one of embodiments 1 to 5, characterized in that said bispecific antibody comprises as its heavy and light chain variable regions, variable regions the same amino acid sequences as said anti-BCMA antibody.
7. A bispecific antibody specifically binding to the extracellular domain of human BCMA and human CD3ε, for use in the treatment of a patient suffering from a disorder involving plasma cells, whereby the ratio of T cells to effector cells (E:T ratio) in an isolated body fluid sample of said patient is 0.35 : 1, preferably 0.5 : 1 or higher.
8. A bispecific antibody specifically binding to the extracellular domain of human BCMA and human CD3ε, for use in the treatment of a patient suffering from a disorder involving plasma cells, whereby said therapy comprises successively
   i) isolating from said patient a body fluid sample,
   ii) measuring the amount of soluble BCMA in said sample, and
   iii) if the amount of soluble BCMA in said sample is 2.5 ng/mL or higher, and
   iv) if said soluble BCMA in said patient sample specifically binds to said bispecific antibody, treating said patient with said bispecific antibody at a higher dose for the first dose or at a more frequent treatment schedule with a shorter period between the first dose and the second dose of said bispecific antibody or with a shorter period between the first dose and the third dose of said bispecific antibody.
9. A bispecific antibody specifically binding to BCMA and CD3ε which competes with soluble BCMA for binding to human BCMA receptor, whereby said antibody competes with APRIL for binding to BCMA and/or blocks APRIL mediated activation of NF-κB for use in the treatment of a patient suffering from a disorder involving plasma cells, whereby said therapy comprises successively
   i) isolating from said patient a body fluid sample comprising plasma cells and T cells,
   ii) measuring the amount of APRIL in said sample by use of an ELISA method, and
   iii) if the amount of APRIL in said patient sample is more than 100 ng/mL, treating said patient with said bispecific antibody at a 50% to 100% higher dose at APRIL concentrations of 100 ng/mL to 1000 ng/mL and above 1000 ng/mL APRIL concentration more than 2 times and up to 80 times higher compared to the dose recommended for a patient with soluble APRIL concentration below 100 ng/mL or treating said patient with a respective more frequent treatment schedule to reach said higher doses with a shorter period between any two doses of said bispecific antibody.
10. A method of determining BCMA protein expression in an isolated body fluid sample comprising CD138⁺ CD38⁺ cells, of a patient, suffering from a disorder involving plasma cells, said method comprises measuring BCMA expression on said CD138⁺ CD38⁺ cells by using an anti-BCMA antibody with a Kd value, which is 0.70 to 1.3 fold of the Kd value of the anti-BCMA antibody part of a bispecific antibody specifically binding to the extracellular domain of human BCMA and human CD3ε, intended for use in the treatment of said patient, and determining whether Relative Median or Mean Fluorescence Intensity MFI is 80 or more, preferably 100 or more, preferably 200 or more, even more preferably 300 or more over baseline.
11. A method of treating a patient, suffering from a disorder involving plasma cells, comprising analyzing isolated body fluid sample comprising CD138⁺ CD38⁺ cells from said patient for BCMA expression on said CD138⁺ CD38⁺ cells by using an anti-BCMA antibody with a Kd value, which is 0.70 to 1.3 fold of the Kd value of the anti-BCMA antibody part of a bispecific antibody specifically binding to the extracellular domain of human BCMA and human CD3ε, intended for use in the treatment of said patient, and if Relative Median or Mean Fluorescence Intensity MFI is 80 or more, preferably 100 or more over baseline, preferably 200 or more, even more preferably 300 or more treating said patient with said bispecific antibody.
12. A method for selecting a treatment plan that is most effective for a patient, suffering from a disorder involving plasma cells, and wherein an isolated body fluid sample of said patient show MFI for BCMA of 80 or more, preferably 100 or more, preferably 200 or more, even more preferably 300 or more over baseline.
13. A method for predicting the likelihood of a patient, suffering from a disorder involving plasma cells, to respond to a treatment with a bispecific antibody specifically binding to BCMA and CD3ε, whereas the cell-surface BCMA expression in an isolated body fluid sample of said patient, comprising CD138⁺ CD38⁺ cells, and measured by using an anti-BCMA antibody with a Kd value, which is 0.70 to 1.3 fold of the Kd value of the anti-BCMA antibody part of said bispecific antibody, as 80 or more, preferably 100 or more, preferably 200 or more, even more preferably 300 or more over baseline determined as Relative Median or Mean Fluorescence Intensity MFI is predictive of the patient's likelihood to respond to said treatment.
14. An in vitro method of determining cell-surface BCMA expression in an isolated body fluid sample, comprising determining whether Relative Median or Mean Fluorescence Intensity MFI for said CD138⁺ CD38⁺ cells, using an anti-BCMA antibody with a Kd value, which is 0.70 to 1.3 fold of the Kd value of the anti-BCMA antibody part of a therapeutic bispecific antibody specifically binding to BCMA and CD3ε, is 80 or more, preferably 100 or more, preferably 200 or more, even more preferably 300 or more over baseline.
15. An in vitro method of selecting a treatment plan that is most effective for treating a patient, suffering from a disorder involving plasma cells, whereby for said patient cell-surface BCMA expression in an isolated body fluid sample, comprising CD138⁺ CD38⁺ cells, measured by using an anti-BCMA antibody with a Kd value, which is 0.70 to 1.3 fold of the Kd value of the anti-BCMA antibody part of a therapeutic bispecific antibody, specifically binding to BCMA and CD3ε, is 80 or more, preferably 100 or more over baseline determined as Relative Median or Mean Fluorescence Intensity MFI, whereby the treatment plan involves the use of a therapeutic bispecific antibody specifically binding to BCMA and CD3ε.
16. A method for selecting a therapy for treating a patient, suffering from a disorder involving plasma cells a therapy, comprising
   i) if cell-surface BCMA expression in an isolated body fluid sample, comprising CD138⁺ CD38⁺ cells, measured by using an anti-BCMA antibody with a Kd value, which is 0.70 to 1.3 fold of the Kd value of the anti-BCMA antibody part of a therapeutic bispecific antibody, specifically binding to BCMA and CD3ε, is 100 or more, preferably 200 or more, even more preferably 300 or more over baseline determined as Relative Median or Mean Fluorescence Intensity MFI, treating said patient with said therapeutic antibody, or
   ii) if cell-surface BCMA expression in an isolated body fluid sample or an isolated blood sample, comprising CD138⁺ CD38⁺ cells, measured by using an anti-BCMA antibody with a Kd value, which is 0.70 to 1.3 fold of the Kd value of the anti-BCMA antibody part of a therapeutic bispecific antibody, specifically binding to BCMA and CD3ε, is lower than 100, preferably lower than 50, even preferable lower than 10 over baseline determined as Relative Median or Mean Fluorescence Intensity MFI, not treating said patient with said therapeutic antibody.
17. A method for determining in an isolated body fluid sample of a patient, suffering from a disorder involving plasma cells, whether the ratio of CD3⁺ cells to CD138⁺ CD38⁺ cells is 0.35 : 1, preferably 0.5 : 1 or higher, preferably 1:1 or higher, more preferably 5:1 or higher, even more preferably 10:1 or higher.
18. A method of treating an patient suffering from a disorder involving plasma cells, comprising analyzing in an isolated body fluid sample of said patient whether the ratio of CD3⁺ cells to CD138⁺ CD38⁺ cells is 0.35 : 1, preferably 0.5 : 1 or higher, preferably 1:1 or higher, more preferably 5:1 or higher, even more preferably 10:1 or higher whereby said treatment involves the use of a therapeutic bispecific antibody specifically binding to BCMA and CD3ε.
19. A method for selecting a treatment plan that is most effective for a patient, suffering from a disorder involving plasma cells, which show in an isolated body fluid sample a ratio of CD3⁺ cells to CD138⁺ CD38⁺ cells of 0.35 : 1, preferably 0.5 : 1 or higher, preferably 1:1 or higher, more preferably 5:1 or higher, even more preferably 10:1 or higher, whereby said treatment plan involves the use of a therapeutic bispecific antibody specifically binding to BCMA and CD3ε.
20. A method for selecting a treatment plan that is most effective for a patient, suffering from a disorder involving plasma cells, whereby
   i) if the ratio of CD3⁺ cells to CD138⁺ CD38⁺ cells in an isolated body fluid sample of said patient is 0.35 : 1, preferably 0.5 : 1 or higher, preferably 1:1 or higher, more preferably 5:1 or higher, even more preferably 10:1 or higher, treating said patient with a therapeutic bispecific antibody specifically binding to BCMA and CD3ε in monotherapy.
   ii) if the ratio of CD3⁺ cells to CD138⁺ CD38⁺ cells in an isolated body fluid sample of said patient is lower than 0.35 : 1, preferably 0.5 : 1, preferably lower than 0.25 : 1, treating said patient with a therapeutic bispecific antibody specifically binding to BCMA and CD3ε in combination with T-cell proliferative therapy or T-cell chemoattractant therapy.
21. A method for predicting the likelihood of a patient, suffering from a disorder involving plasma cells, to respond to a treatment with a bispecific antibody specifically binding to BCMA and CD3ε, by measuring in an isolated body fluid sample of said patient whether the ratio of CD3⁺ cells to CD138⁺ CD38⁺ cells is 0.35 : 1, preferably 0.5 : 1 or higher, preferably 1:1 or higher, more preferably 5:1 or higher, even more preferably 10:1 or higher, which is predictive of the patient's likelihood to respond to a treatment.
22. An in vitro method of determining in an isolated body fluid sample of a patient suffering from a disorder involving plasma cells whether the ratio of CD3⁺ cells to CD138⁺ CD38⁺ cells is 0.35 : 1, preferably 0.5 : 1 or higher, preferably 1:1 or higher, more preferably 5:1 or higher, even more preferably 10:1 or higher,.
23. An in vitro method of selecting a treatment plan that is most effective for treating a patient, suffering from a disorder involving plasma cells, whereby in an isolated body fluid sample of said patient the ratio of CD3⁺ cells to CD138⁺ CD38⁺ cells is determined as 0.35 : 1, preferably 0.5 : 1 or higher, preferably 1:1 or higher, more preferably 5:1 or higher, even more preferably 10:1 or higher and whereby the treatment plan involves the use of a therapeutic bispecific antibody specifically binding to BCMA and CD3ε.
24. A method for selecting a therapy with a bispecific antibody specifically binding to BCMA and CD3ε for a patient, suffering from a disorder involving plasma cells, comprising
   i) if in an isolated body fluid sample of said patient the ratio of CD3⁺ cells to CD138⁺ CD38⁺ cells is 0.35 : 1, preferably 0.5 : 1 or higher, preferably 1:1 or higher, more preferably 5:1 or higher, even more preferably 10:1 or higher, treating said patient with said therapeutic antibody, or
   ii) if in an isolated body fluid sample of said patient the ratio of CD3⁺ cells to CD138⁺ CD38+ cells is lower than 0.35 : 1, preferably 0.5 : 1, preferably lower than 0.25 : 1 treating said patient in combination with T-cell proliferative therapy or T-cell chemoattractant therapy.
25. A method of determining in an isolated body fluid sample comprising CD138⁺ CD38⁺ cells, of a patient suffering from a disorder involving plasma cells, whether the amount of soluble BCMA in said sample is 2.5 ng/mL or higher, preferably 10 ng/mL or higher, more preferably 50 ng/mL or higher, even more preferably 250 ng/mL or higher.
26. A method of treating a patient suffering from a disorder involving plasma cells, comprising determining whether the amount of soluble BCMA in an isolated body fluid sample is 2.5 ng/mL or higher, preferably 10 ng/mL or higher, more preferably 50 ng/mL or higher, even more preferably 250 ng/mL or higher.
27. A method for predicting the likelihood of a patient, suffering from a disorder involving plasma cells, to respond to a treatment with a bispecific antibody specifically binding to BCMA and CD3ε, whereby an amount of soluble BCMA in an isolated body fluid sample of 2.5 ng/mL or higher, preferably 10 ng/mL or higher, more preferably 50 ng/mL or higher, even more preferably 250 ng/mL or higher is predictive of the patient's likelihood to respond to a treatment.
28. An in vitro method of determining in an isolated body fluid sample of a patient suffering from a disorder involving plasma cells, whether the amount of soluble BCMA in said sample is 2.5 ng/mL or higher, preferably 10 ng/mL or higher, more preferably 50 ng/mL or higher, even more preferably 250 ng/mL or higher.
29. A method for selecting a treatment that is most effective for a patient of a patient, suffering from a disorder involving plasma cells, which show in an isolated body fluid sample an amount of soluble BCMA of 2.5 ng/mL or higher, preferably 10 ng/mL or higher, more preferably 50 ng/mL or higher, even more preferably 250 ng/mL or higher, whereby the treatment involves the use of a bispecific antibody specifically binding to BCMA and CD3ε.
30. An in vitro method of selecting a treatment plan that is most effective for treating a patient, suffering from a disorder involving plasma cells, by determining whether the amount of soluble BCMA in an isolated body fluid sample is 2.5 ng/mL or higher, preferably 10 ng/mL or higher, more preferably 50 ng/mL or higher, even more preferably 250 ng/mL or higher, and the treatment plan involves the use of a bispecific antibody specifically binding to BCMA and CD3ε.
31. A method for selecting a therapy for treating a patient, suffering from a disorder involving plasma cells a therapy, comprising
   i) if the amount of soluble BCMA in an isolated body fluid sample of said patient is lower than 2.5 ng/mL, treating said patient with said therapeutic antibody, or
   ii) if the amount of soluble BCMA in said sample is 2.5 ng/mL or higher and,
      if said soluble BCMA in said patient sample does not bind to said bispecific antibody, treating said patient with said therapeutic antibody, or
   iii) if the amount of soluble BCMA in said sample is 2.5 ng/mL or higher and, if said soluble BCMA in said patient sample specifically binds to said bispecific antibody, treating said patient with said bispecific antibody at a higher dose for the first dose or at a more frequent treatment schedule with a shorter period between the first dose and the second dose of said bispecific antibody or with a shorter period between the first dose and the third dose of said bispecific antibody.
32. A method of determining in an isolated body fluid sample comprising CD138⁺ CD38⁺ cells, of a patient suffering from a disorder involving plasma cells, whether the amount the amount of soluble APRIL in said sample is 100 ng/mL or higher, preferably 1000 ng/mL or higher.
33. A method of treating a patient, suffering from a disorder involving plasma cells and diagnosed that the amount of soluble APRIL in an isolated body fluid sample of said patient is 100 ng/mL or higher, preferably 1000 ng/mL or higher, with a bispecific antibody specifically binding to BCMA and CD3ε, characterized in involving the use of a bispecific antibody specifically binding to BCMA and CD3ε.
34. A method for selecting a treatment plan that is most effective for a patient suffering from a disorder involving plasma cells which show in an isolated body fluid sample an amount of soluble APRIL of 100 ng/mL or higher, characterized in that said treatment plan involves the use of a bispecific antibody specifically binding to BCMA and CD3ε.
35. A method for predicting the likelihood of a patient, suffering from a disorder involving plasma cells, to respond to a treatment with a bispecific antibody specifically binding to BCMA and CD3, whereby the amount of soluble APRIL, in an isolated body fluid sample of said patient, is 100 ng/mL or higher is predictive of the patient's likelihood to respond to a treatment.
36. An in vitro method of determining in an isolated body fluid sample of a patient, suffering from a disorder involving plasma cells, whether the amount of soluble APRIL in said sample is 100 ng/mL or higher, preferably 1000 ng/mL or higher.
37. An in vitro method of selecting a treatment plan that is most effective for treating a patient, suffering from a disorder involving plasma cells, by determining whether the amount of soluble APRIL in said sample is 100 ng/mL or higher, preferably 1000 ng/mL or higher, and the treatment plan involves the use of an APRIL competitive bispecific antibody or an APRIL non-competitive bispecific antibody.
38. A method for selecting a therapy for treating a patient, suffering from a disorder involving plasma cells a therapy, comprising
   i) if the amount of soluble APRIL in an isolated body fluid sample of said patient is 100 ng/mL or lower, treating said patient with an APRIL competitive bispecific antibody or APRIL non-competitive bispecific antibody, or
   ii) if the amount of soluble APRIL in said sample is 100 ng/mL or higher, treating said patient with an APRIL non-competitive bispecific antibody.
39. A method of treating a patient, suffering from a disorder involving plasma cells, characterized in that the amount of APRIL in said patient sample is more than 100 ng/mL, treating said patient with said bispecific antibody at a two times higher dose at APRIL concentrations of 100 ng/mL and a further increased dose up to 80 times higher if APRIL concentration increases up to 1000 ng/mL, compared to the dose recommended for a patient with soluble APRIL concentration below 100 ng/mL or treating said patient with a respective more frequent treatment schedule to reach said higher doses with a shorter period between any two doses of said bispecific antibody.
40. A bispecific antibody specifically binding to the extracellular domain of human BCMA and human CD3ε, for use in the treatment of a patient, suffering from a disorder involving plasma cells, characterized in that the amount of APRIL in said patient sample is more than 100 ng/mL, treating said patient with said bispecific antibody at a two times higher dose at APRIL concentrations of 100 ng/mL and a further increased dose up to 80 times higher if APRIL concentration increases up to 1000 ng/mL, compared to the dose recommended for a patient with soluble APRIL concentration below 100 ng/mL or treating said patient with a respective more frequent treatment schedule to reach said higher doses with a shorter period between any two doses of said bispecific antibody.
41. A method for determining a treatment plan for a new patient, suffering from a disorder involving plasma cells, comprising:
   providing, utilizing at least one method for investigation the BCMA related plasma cell status of said new patient;
   searching, utilizing at least the result of one method, for a prior treatment plan for a prior patient suffering from the same disorder with at least one similar representation; and
   reviewing the prior treatment plan for the prior patient in order to determine how to improve the treatment of the new patient based on information in at least one prior treatment plan, whereby the treatment plan involves the use of a bispecific antibody specifically binding to BCMA and CD3ε, its dose and the treatment schedule for the period at least between the first dose and the second dose of said bispecific antibody.
42. A kit for use of determination of cell-surface BCMA expression, comprising Vials or tubes pre-loaded with four labelled-antibodies, one specifically binding to CD138, one to CD38, one to CD19, and one anti-BCMA antibody with a Kd value, which is 0.70 to 1.3 fold of the Kd value of the anti-BCMA antibody part of a therapeutic bispecific antibody specifically binding to BCMA and CD3ε.
43. A kit for use of determination of E:T ratio, comprising: Vials or tubes pre-loaded with four labelled-antibodies to detect malignant PC and T cells one specifically binding to CD138, one to CD38, one to CD19, and one to CD3).
44. A kit for use of determination of soluble BCMA, comprising a polyclonal anti-BCMA antibody as capture antibody, and a detection anti-BCMA antibody with a Kd value, which is 0.70 to 1.3 fold of the Kd value of the anti-BCMA antibody part of a therapeutic bispecific antibody specifically binding to BCMA and CD3ε.
45. 41. A method for determining a treatment plan for a new patient, suffering from a disorder involving plasma cells, comprising providing, utilizing at least one method of the BCMA mediated plasma cell status of said new patient and based on that adapt the acknowledged/approved therapy with a BCMA-T-cell bispecific antibody by adapting dose or treatment schedule.
46. A bispecific antibody specifically binding to the extracellular domain of human B-cell maturation antigen (further named also as "BCMA") and human CD3ε (further named also as "CD3"), for use in the treatment of a patient suffering from multiple myeloma disease, said disease being characterized in that in an isolated body fluid sample of said patient, comprising CD138+ CD38+ cells, BCMA expression on said CD138+ CD38+ cells, measured by using an anti-BCMA antibody with a Kd value, which is 0.70 to 1.3 fold of the Kd value of the anti-BCMA antibody part of said bispecific antibody, is 80 or more over baseline determined as Relative Median or Mean Fluorescence Intensity MFI.
47. A bispecific antibody specifically binding to the extracellular domain of human BCMA and human CD3ε, for use in the treatment of a patient suffering from multiple myeloma disease, said disease being characterized in that the ratio of T cells (effector cells) to target cells (E:T ratio) in an isolated body fluid sample of said patient is 0.35 : 1.
48. A bispecific antibody specifically binding to the extracellular domain of human BCMA and human CD3ε, for use in the treatment of a patient suffering from multiple myeloma disease, said disease being characterized in that in an isolated body fluid sample from said patient the amount of soluble BCMA is 2.5 ng/mL or higher, and said soluble BCMA in said patient sample specifically binds to said bispecific antibody, characterized in that said treatment of said patient with said bispecific antibody is performed at higher doses and/or at a more frequent treatment schedule.
49. A bispecific antibody specifically binding to the extracellular domain of human BCMA and human CD3ε, for use in the treatment of a patient suffering from multiple myeloma disease, said disease being characterized in that in an isolated body fluid sample from said patient the amount of soluble BCMA is 2.5 ng/mL or higher and said soluble BCMA in said patient sample specifically binds to said bispecific antibody, characterized in that said treatment of said patient with said bispecific antibody is performed at a higher dose for the first dose or at a more frequent treatment schedule with a shorter period between the first dose and the second dose of said bispecific antibody or with a shorter period between the first dose and the third dose of said bispecific antibody.
50. A bispecific antibody specifically binding to BCMA and CD3ε which competes with soluble BCMA for binding to human BCMA receptor and/or blocks APRIL mediated activation of NF-κB for use in the treatment of a patient suffering from multiple myeloma disease, said disease being characterized in that in an isolated body fluid sample from said patient the amount of APRIL is more than 100 ng/mL, characterized in that said treatment of said patient with said bispecific antibody is performed at higher doses and/or at a more frequent treatment schedule.
51. A bispecific antibody specifically binding to BCMA and CD3ε which competes with soluble BCMA for binding to human BCMA receptor, whereby said antibody competes with APRIL for binding to BCMA, whereby said antibody competes with APRIL for binding to BCMA, whereby said antibody competes with APRIL for binding to BCMA and/or blocks APRIL mediated activation of NF-κB for use in the treatment of a patient suffering from multiple myeloma disease, said disease being characterized in that in an isolated body fluid sample of said patient comprising plasma cells and T cells, the amount of APRIL is more than 100 ng/mL, characterized in treating said patient with said bispecific antibody at a two times higher dose at APRIL concentrations of 100 ng/mL and a further increased dose up to 80 times higher if APRIL concentration increases up to 1000 ng/mL, compared to the dose recommended for a patient with soluble APRIL concentration below 100 ng/mL or treating said patient with a respective more frequent treatment schedule to reach said higher doses with a shorter period between any two doses of said bispecific antibody.
52. A bispecific antibody specifically binding to the extracellular domain of human B-cell maturation antigen (further named also as "BCMA") and human CD3ε (further named also as "CD3"), for use in the treatment of a patient suffering from multiple myeloma disease, said disease being characterized in that in an isolated body fluid sample of said patient, comprising CD138+ CD38+ cells, BCMA expression on said CD138+ CD38+ cells, measured by using an anti-BCMA antibody with a Kd value, which is 0.70 to 1.3 fold of the Kd value of the anti-BCMA antibody part of said bispecific antibody, is 80 or more over baseline determined as Relative Median or Mean Fluorescence Intensity MFI.
52. A bispecific antibody specifically binding to the extracellular domain of human BCMA and human CD3ε, for use in the treatment of a patient suffering from multiple myeloma disease, said disease being characterized in that the ratio of T cells (effector cells) to target cells (E:T ratio) in an isolated body fluid sample of said patient is 0.35 : 1 or higher.
54. A bispecific antibody for use according to embodiment 52, characterized in that the E:T ratio is 0.35 : 1 to 22 :1.
55. A bispecific antibody specifically binding to the extracellular domain of human BCMA and human CD3ε, for use in the treatment of a patient suffering from multiple myeloma disease, said disease being characterized in that in an isolated body fluid sample from said patient the amount of soluble BCMA is 2.5 ng/mL or higher, and said soluble BCMA in said patient sample specifically binds to said bispecific antibody, characterized in that said treatment of said patient with said bispecific antibody is performed with a dose per week which is 1.5 fold up to 10 fold or/and in that the time interval between dose-administrations is shortened from once per week administration up to once per day compared to a standard dose.
56. The bispecific antibody for use according to embodiment 55, characterized in that said treatment of said patient with said bispecific antibody is performed with a dose per week which is 1.5 fold up to 2.0 fold compared to a standard dose.
57. The bispecific antibody for use according to embodiment 55, characterized in that said treatment of said patient with said bispecific antibody is performed in that the time interval between dose-administrations is shortened from once per week administration up to twice a week compared to the standard dose.
58. A bispecific antibody specifically binding to BCMA and CD3ε which competes with soluble BCMA for binding to human BCMA receptor and/or blocks APRIL mediated activation of NF-κB for use in the treatment of a patient suffering from multiple myeloma disease, said disease being characterized in that in an isolated body fluid sample from said patient the amount of APRIL is higher than 10 ng/mL and up to 100 ng/mL, characterized in that said treatment of said patient with said bispecific antibody is performed per week with a dose which is 1.5 fold up to 20 fold or/and in that the time interval between dose-administrations is shortened from once per week administration up to once a day compared to a standard dose.
59. The bispecific antibody for use according to embodiment 58, characterized in that said treatment of said patient with said bispecific antibody is performed with a dose per week which is 1.5 fold up to a 3.0fold compared to a standard dose.
60. The bispecific antibody for use according to embodiment 58, characterized in that said treatment of said patient with said bispecific antibody is performed in that the time interval between dose-administrations is shortened from once per week administration up to three times a week compared to the standard dose.

### Materials & general methods

### Cell culture techniques

Standard cell culture techniques are used as described in Current Protocols in Cell Biology (2000), Bonifacino, J. S., Dasso, M., Harford, J.B., Lippincott-Schwartz, J. and Yamada, K.M. (eds.), John Wiley & Sons, Inc.

### Isolation of primary human pan T cells from PBMCs

Peripheral blood mononuclear cells (PBMCs) were prepared by Histopaque density centrifugation from enriched lymphocyte preparations (buffy coats) obtained from local blood banks or from fresh blood from healthy human donors. Briefly, blood was diluted with sterile PBS and carefully layered over a Histopaque gradient (Sigma, H8889). After centrifugation for 30 minutes at 450 × g at room temperature (brake switched off), part of the plasma above the PBMC containing interphase was discarded. The PBMCs were transferred into new 50 ml Falcon tubes and tubes were filled up with PBS to a total volume of 50 ml. The mixture was centrifuged at room temperature for 10 minutes at 400 × g (brake switched on). The supernatant was discarded and the PBMC pellet washed twice with sterile PBS (centrifugation steps at 4°C for 10 minutes at 350 × g). The resulting PBMC population was counted automatically (ViCell) and stored in RPMI1640 medium, containing 10% FCS and 1% L-alanyl-L-glutamine (Biochrom, K0302) at 37°C, 5% C0₂ in the incubator until assay start.

T cell enrichment from PBMCs was performed using the Pan T Cell Isolation Kit II (Miltenyi Biotec #130-091-156), according to the manufacturer's instructions. Briefly, the cell pellets were diluted in 40 µ 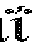 cold buffer per 10 million cells (PBS with 0.5% BSA, 2 mM EDTA, sterile filtered) and incubated with 10 µ 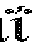 Biotin- Antibody Cocktail per 10 million cells for 10 min at 4°C. 30 µ 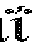 cold buffer and 20 µ 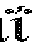 Anti-Biotin magnetic beads per 10 million cells were added, and the mixture incubated for another 15 min at 4°C. Cells were washed by adding 10-20x the current volume and a subsequent centrifugation step at 300 × g for 10 min. Up to 100 million cells were resuspended in 500 µ 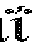 buffer. Magnetic separation of unlabeled human pan T cells was performed using LS columns (Miltenyi Biotec #130-042-401) according to the manufacturer's instructions. The resulting T cell population was counted automatically (ViCell) and stored in AIM-V medium at 37°C, 5% C0₂ in the incubator until assay start (not longer than 24 h).

### Isolation of primary human naive T cells from PBMCs

Peripheral blood mononuclar cells (PBMCs) were prepared by Histopaque density centrifugation from enriched lymphocyte preparations (buffy coats) obtained from local blood banks or from fresh blood from healthy human donors. T-cell enrichment from PBMCs was performed using the Naive CD8⁺ T cell isolation Kit from Miltenyi Biotec (#130-093-244), according to the manufacturer's instructions, but skipping the last isolation step of CD8⁺ T cells (also see description for the isolation of primary human pan T cells).

### BCMApositive human myeloma cell lines

BCMA-positive human myeloma cell lines (NCI-H929, RPMI-8226, U266B1 and L-363) were used. NCI-H929 cells ((H929) ATCC^{®} CRL-9068^{™}) were cultured in 80-90% RPMI 1640 with 10-20% heat-inactivated FCS and could contain 2 mM L-glutamine, 1 mM sodium pyruvate and 50 µM mercaptoethanol. RPMI-8226 cells ((RPMI) ATCC^{®} CCL-155^{™}) were cultured in a media containing 90% RPMI 1640 and 10% heat-inactivated FCS. U266B1 ((U266) ATCC^{®} TIB-196^{™}) cells were cultured in RPMI-1640 medium modified to contain 2 mM L-glutamine, 10 mM HEPES, 1 mM sodium pyruvate, 4500 mg/L glucose, and 1500 mg/L sodium bicarbonate and 15% heat-inactivated FCS. L-363 cell line (Leibniz Institute DSMZ - German collection of microorganisms and cell cultures; DSMZ No. ACC 49) was cultured in 85% RPMI 1640 and 15% heat-inactivated FCS.

### Examples

### Example 1: Optimized measurement of BCMA expression on patient myeloma cells

### Example 1.1: Qualitative measurement of BCMA expression on patient myeloma cells as detected by flow cytometry (median fluorescence intensity)

Blood and bone marrow aspirates were collected from multiple myeloma patients after informed consent is given, in accordance with local ethical committee guidelines and the Declaration of Helsinki. Qualitative expression of BCMA was measured on the cell surface of patient myeloma cells from bone marrow aspirates by flow cytometry. APC-conjugated bivalent BCMA-1 antibody, which has an affinity to human BCMA of 10.9 ± 2.7 nM as detected by surface plasmon resonance, was used to determine the median fluorescence intensity (MFI).

To determine the expression of BCMA receptor on patient bone marrow myeloma cells, immunophenotypic analyses were performed using freshly isolated bone marrow aspirates. Erythrocyte-lysed K₃-EDTA (ethylenediaminetetraacetic acid) anticoagulated whole bone marrow samples were used for the immunophenotypic analyses. A total of 2 × 10⁶ cells per tube were stained using a direct immunofluorescence technique and multicolor staining, which was aimed at the specific identification and immunophenotypic characterization of malignant plasma cells identified as CD138⁺ CD38⁺ CD45⁺ CD56⁺ CD19⁻. The bone marrow cells were then stained using a panel of fluorochrome-conjugated antibodies including at least CD138-APCC750/CD38-FITC/CD56-PE/CD19-PerCP-Cy7/CD45-V450/BCMA-APC for 20 to 30 min on ice, protected from light. Fluorochrome-labelled antibodies used were purchased from BD Biosciences (San Jose, CA) and Caltag Laboratories (San Francisco CA). APC-conjugated bivalent anti-human BCMA-1 antibody was used in the immunophenotypic analyses. Acquisition was performed using a multicolor flow cytometer and installed software (e.g. CantoII device running FACS Diva software or FACS Calibur flow cytometer using the CellQUEST software). The Paint-A-Gate PRO program (BD Biosciences) was used for data analysis. BCMA expression was measured by gating on the malignant plasma cell population and median fluorescence intensity values were determined and compared among the myeloma patients. Relative MFI values of BCMA expression on myeloma cells were calculated by subtracting the absolute MFI value of an APC-conjugated isotope control antibody gated on CD138⁺ CD38⁺ CD45⁺ CD56⁻ CD19⁻ myeloma cells or APC-conjugated BCMA-1 antibody gated on CD3⁺ T cells (known to be BCMA-negative) from the absolute MFI value of an APC-conjugated BCMA-1 antibody gated on myeloma cells. Figure 1 shows the representative FACS histogram plots of (A) Medium-high BCMA expression, (B) moderate BCMA expression and (C) low BCMA expression on patient myeloma cells as detected by flow cytometry (MFI). As shown in Figure 1, there is a clear shift to the right corresponding to positive BCMA expression on patient myeloma cells when compared to the negative control (APC-conjugated BCMA-1 antibody gated on T cells). Based on the relative MFI values, all myeloma patients express BCMA on their malignant plasma cells but BCMA expression varies from low expression (relative MFI values < 10³) to moderate expression (10³ - 0.3 × 10⁴) to medium-high expression (0.3 × 10⁴ - 10⁴). BCMA expression with relative MFI values > 10⁴ was not observed among the patient bone marrow samples investigated. Table 3 summarizes the relative MFI values of BCMA expressed on patient bone marrow myeloma cells.

**Table 3: BCMA expression on patient myeloma cells in bone marrow: relative median fluorescence intensity**

| Patient No. | Relative MFI values | BCMA expression |
|---|---|---|
| A1 | 2636 | Moderate |
| A2 | 3199 | Medium-high |
| A3 | 557 | Low |
| A4 | 342 | Low |
| A5 | 880 | Low |
| A6 | 1387 | Moderate |
| A7 | 235 | Low |
| A8 | 1489 | Moderate |
| A9 | 93 | Low |
| A10 | 88 | Low |
| A11 | 1415 | Moderate |
| A12 | 2396 | Moderate |
| A13 | 356 | Low |
| A14 | 1964 | Moderate |
| A15 | 541 | Low |
| A16 | 858 | Low |
| A17 | 1574 | Moderate |
| A18 | 1147 | Moderate |
| A19 | 1847 | Moderate |
| A20 | 913 | Low |
| A21 | 3422 | Medium-high |
| A22 | 547 | Low |
| A23 | 136 | Low |

### Example 1.2: Quantitative measurement of BCMA on patient myeloma cells as detected by flow cytometry (specific antigen binding capacity)

The quantitative expression of BCMA i.e. the specific antigen binding capacity (SABC) of BCMA was also measured on the cell surface of patient myeloma cells using flow cytometry. The Qifikit (Dako) method was used to quantify BCMA antigen copy number or specific antigen binding capacity on the cell surface of patient's bone marrow myeloma cells in comparison to H929 human myeloma cell line. Bone marrow aspirates were collected and cells were washed with FACS buffer (100 µl/well; 350 × g for 5 min) and adjusted to 1 Mio cells/ml. 50 µl (= 0.5 Mio cells) of the cell suspension were transferred into each well of a 96 round bottom well plate. Then, 50 µl of mouse anti-human BCMA IgG (BioLegend #357502) or a mouse IgG2a isotype control (BioLegend # 401501) diluted in FACS buffer (PBS, 0.1% BSA) to a final concentration of 25 µg/ml (or at saturation concentrations) were added and staining was performed for 30 min at 4°C in the dark. Next, 100 µl of the Set-up or Calibration Beads were added in separate wells and the cells, as well as the beads were washed twice with FACS buffer. Cells and beads were resuspended in 25 µl FACS buffer, containing fluorescein conjugated anti-mouse secondary antibody (at saturation concentrations), provided in the Qifikit. Cells and beads were stained for 45 min at 4°C in the dark. The cells were washed once and all samples were resuspended in 100 µl FACS buffer. Samples were analyzed on a multicolor flow cytometer and installed software (e.g. CantoII device running FACS Diva software or FACS Calibur flow cytometer using the CellQUEST software). The Paint-A-Gate PRO program (BD Biosciences) was used for data analysis. Table 4 summarizes quantitative BCMA expression on patient's bone marrow myeloma cells as measured by specific antigen binding capacity (SABC).

**Table 4: BCMA expression on patient myeloma cells in bone marrow: specific antigen binding capacity**

| Patient No. | SABC values | BCMA expression |
|---|---|---|
| B3 | 679 | Low |
| B4 | 145 | Low |
| B5 | 957 | Low |
| B6 | 969 | Low |
| B7 | 554 | Low |
| B8 | 4'479 | Moderate |
| B9 | 350 | Low |
| B10 | 414 | Low |
| B11 | 2756 | Moderate |
| B12 | 2911 | Moderate |
| B13 | 1267 | Moderate |
| B14 | 3453 | Moderate |
| B15 | 1006 | Moderate |
| B16 | 1097 | Moderate |
| B17 | 1622 | Moderate |
| B18 | 429 | Low |
| B19 | 1684 | Moderate |
| B20 | 383 | Low |
| B21 | 1602 | Moderate |
| B22 | 799 | Low |
| B23 | 204 | Low |
| H929 | 38'000 | Very high |

### Example 1.3: Killing potency of BCMA-TCB is influenced by BCMA expression on the surface of target cells: BCMA^{hi}-expressing H929 vs. BCMA^{med/lo}-expressing U266 vs. BCMA^{med/lo}-expressing L363 vs. BCMA^{lo}-expressing RPMI-8226 myeloma cells

The potency of BCMA-TCB antibodies can be influenced by the level of expression of BCMA on the cell surface of myeloma cells. The killing potency of BCMA-TCB was measured in a redirected T-cell cytotoxicity assay using different human myeloma cell lines as target cells i.e. BCMA^{hi}-expressing H929 vs. BCMA^{med/lo}-expressing U266 myeloma cells.

Briefly, human BCMA^{hi}-expressing H929 or BCMA^{med/lo}-expressing U266 multiple myeloma target cells were harvested with Cell Dissociation Buffer, washed and resuspended in RPMI supplemented with 10% fetal bovine serum (Invitrogen). Approximately, 30,000 cells per well were plated in a round-bottom 96-well plate and the respective dilution of the TCB constructs were added for a desired final concentration (in triplicates); final concentrations of BCMA-TCB ranging from 0.1 pM to 10 nM. For an appropriate comparison, all TCB constructs and controls were adjusted to the same molarity. Human PBMCs (effector) were added into the wells to obtain a final E:T ratio of 10:1. Negative control groups were represented by effector or target cells only. As a positive control for the activation of human pan T cells, 1 µg/ml PHA (Sigma #L8902) was used. For normalization, maximal lysis of the BCMA^{hi}-expressing H929 or BCMA^{med/lo}-expressing U266 myeloma target cells (= 100%) was determined by incubation of the target cells with a final concentration of 1% Triton X-100, inducing cell death. Minimal lysis (= 0%) was represented by target cells co-incubated with effector cells only, i.e. without any T cell bispecific antibody. After 24 h incubation at 37°C, 5% CO₂, LDH release from the apoptotic/necrotic BCMA^{hi}-expressing H929 or BCMA^{med/lo}-expressing U266 myeloma target cells into the supernatant was then measured with the LDH detection kit (Roche Applied Science), following the manufacturer's instructions. The percentage of LDH release was plotted against the concentrations of anti-BCMA/anti-CD3 T cell bispecific antibodies in concentration-response curves. The EC50 values were measured using Prism software (GraphPad) and determined as the TCB antibody concentration that results in 50% of maximum LDH release. As shown in Figure 2, BCMA-2- TCB induced killing of BCMA^{hi}-expressing H929 myeloma cells with an EC50 of 115 pM and maximum killing of 60%, while the same BCMA-TCB antibody was only able to kill BCMA^{med/lo}-expressing U266 myeloma target cells with an EC50 of 370 pM and maximum killing at 18% when performed in a head-to-head comparison. Table 5 summarizes the EC50 values and maximum killing of BCMA-2-TCB to kill BCMA^{hi}-expressing H929 or BCMA^{lo}-expressing U266 myeloma target cells.

The potency of another BCMA-TCB antibody, BCMA-1-TCB, to induce killing of BCMA expressing myeloma cell lines (BCMA^{hi}-expressing H929, BCMA^{mid/lo}-expressing L363 and BCMA^{lo}-expressing RPMI-8226 MM cells) was also tested using similar experimental conditions. As shown in Figure 2.1, BCMA-1- TCB induced killing of (A) BCMA^{hi}-expressing H929 myeloma cells with an EC50 of 8.49 pM and maximum killing of 82.8%, while the same BCMA-1-TCB antibody was only able to kill (B) BCMA^{med/lo}-expressing L363 myeloma target cells with an EC50 of 12.6 pM and maximum killing at 67.1% or (C) BCMA^{lo}-expressing RPMI-8226 with an EC50 of 229.3 pM and maximum killing at 28.1% when performed in a head-to-head comparison. Table 5.1 summarizes the EC50 values and maximum killing of BCMA-1-TCB to kill BCMA^{hi}-expressing H929, BCMA^{med/lo}-expressing L363 or BCMA^{lo}-expressing RPMI-8226 myeloma target cells.

**Table 5: Potency of BCMA-2-TCB to kill BCMA expressing myeloma cell lines is influenced by BCMA expression on target cells**

| Human cell line | Killing potency EC50 (pM) | Maximum killing |
|---|---|---|
| BCMA^{hi}-expressing H929 | 115 | 60% |
| BCMA^{med/lo}-expressing U266 | 370 | 18% |

**Table 5.1: Potency of BCMA-1-TCB to kill BCMA expressing myeloma cell lines is influenced by BCMA expression on target cells**

| Human cell line | Killing potency EC50 (pM) | Maximum killing |
|---|---|---|
| BCMA^{hi}-expressing H929 | 8.49 | 82.8% |
| BCMA^{med/lo}-expressing L363 | 12.6 | 67.1% |
| BCMA^{lo}-expressing RPMI-8226 | 229.3 | 28.1% |

### Example 2: Measurement of effector cells to tumor cells (E:T) ratio in myeloma patient bone marrow aspirates

The potency of BCMA antibodies can be influenced by the level of expression of BCMA on the cell surface of myeloma cells. However, for BCMA-TCB antibodies the killing potency of even cells expressing high levels of BCMA on the surface can also be influenced by E:T ratios which can significantly varied as observed in multiple myeloma patients.

### Example 2.1: Determination of CD3⁺ T cells to CD138⁺ CD38⁺ myeloma cells (E:T) ratio in myeloma patient bone marrow aspirates by flow cytometry

### Example 2.1.1: Measurement of myeloma cells in myeloma patient bone marrow aspirates

To determine the percentage and absolute counts of bone marrow infiltrating malignant plasma cells in myeloma patients, immunophenotypic analyses were performed using freshly isolated bone marrow aspirates. Erythrocyte-lysed K₃-EDTA (ethylenediaminetetraacetic acid) anticoagulated whole bone marrow samples were used for the immunophenotypic analyses. A total of 2 × 10⁶ cells per tube were stained using a direct immunofluorescence technique and multicolor staining, which was aimed at the specific identification and immunophenotypic characterization of malignant plasma cells identified as CD138⁺ CD38⁺ CD45⁺ CD56⁺ CD19⁻. The bone marrow cells were then stained using a panel of fluorochrome-conjugated antibodies including at least CD138-APCC750/CD38-FITC/CD56-PE/CD19-PerCP-Cy7/CD45-V450 for 20 to 30 min on ice, protected from light. Fluorochrome-labelled antibodies used were purchased from BD Biosciences (San Jose, CA) and Caltag Laboratories (San Francisco CA). Acquisition was performed using a multicolor flow cytometer and installed software (e.g. CantoII device running FACS Diva software or FACS Calibur flow cytometer using the CellQUEST software). The Paint-A-Gate PRO program (BD Biosciences) was used for data analysis. Percentage of malignant plasma cells was determined by gating on CD138⁺ CD38⁺ CD45⁺ CD56⁺ CD19⁻ population. To calculate the absolute counts of malignant plasma cells, the percentage of malignant plasma cells was multiplied by the volume of the bone marrow aspirate sample measured for example with a hematology analyzer (Advia^{®} 120 System, Siemens). In some experiments, Trucount^{™} tubes (BD Biosciences, San Jose CA USA) were used to determine the absolute counts of leucocytes in blood.

### Example 2.1.2: Measurement of T cells and T cell subsets in myeloma patient bone marrow aspirates

For BCMA-TCB, effector cells are mainly T cells including many T-cell subsets. To determine the percentage and absolute counts of T cells and T-cell subsets, immunophenotypic analyses were performed using freshly isolated bone marrow aspirates. Erythrocyte-lysed K₃-EDTA (ethylenediaminetetraacetic acid) anticoagulated whole bone marrow samples were used for the immunophenotypic analyses. A total of 2 × 10⁶ cells per tube were stained using a direct immunofluorescence technique and multicolor staining, which was aimed at the specific identification and immunophenotypic characterization of T cells can be identified as CD45⁺ CD3⁺ CD56⁻ or CD3⁺ or CD45⁺ CD19⁻ CD56⁻. Bone marrow cells were then stained using a panel of fluorochrome-conjugated antibodies including CD138-APCC750/CD38-FITC/CD56-PE/CD19-PerCP-Cy7/CD45-V450 for 20 to 30 min on ice, protected from light. Fluorochrome-labelled antibodies used were purchased from BD Biosciences (San Jose, CA) and Caltag Laboratories (San Francisco CA). Acquisition was performed using a multicolor flow cytometer and installed software (e.g. CantoII device running FACS Diva software or FACS Calibur flow cytometer using the CellQUEST software). The Paint-A-Gate PRO program (BD Biosciences) was used for data analysis. Percentage of bone marrow infiltrating T cells was determined by gating on CD45⁺ CD19⁻ CD56⁻population. To calculate the absolute counts of T cells, the percentage of T cells was multiplied by the volume of the bone marrow aspirate sample.

Effector cells representing total T cells or T-cell subsets are also measured by staining of myeloma patient bone marrow cells with fluorochrome-conjugated antibodies: CD3⁺ T cells, CD3⁺ CD4⁺ helper T cells, CD3⁺ CD8⁺ cytotoxic T cells, CD3⁺ CD45RA⁺ CD197⁻ naive T cells, CD3⁺ CD45RA⁺ CD197⁻ CD4⁺ naive CD4 T cells, CD3⁺ CD45RA⁺ CD197⁻ CD8⁺ naive CD8 T cells, CD3⁺ CD45RA⁻memory T cells, CD3⁺ CD45RA⁻ CD4⁺ memory CD4 T cells, CD3⁺ CD45RA⁻ CD8⁺ memory CD8 T cells, CD3⁺ CD45RA⁻ CD197⁺ central memory T cells, CD3⁺ CD45RA⁻ CD197⁺ CD4⁺ central memory CD4 T cells, CD3⁺ CD45RA⁻ CD197⁺ CD8+ central memory CD8, CD3+ CD45RA-CD197⁻ effector memory T cells, CD3⁺ CD45RA⁻ CD197⁻ CD4⁺ effector memory CD4 T cells; CD3⁺ CD45RA⁻ CD197⁻ CD8⁺ effector memory CD8 T, CD3⁺ CD45RA⁺ CD197⁻ effector T cells, CD3⁺ CD45RA⁺ CD197⁻ CD4⁺ effector CD4 T cells, CD3⁺ CD45RA⁺ CD197⁻ CD8⁺ effector CD8 T cells, CD3⁺ CD4⁺ CD25^{hi} CD127^{lo} regulatory T cells, CD3⁺ PD-1⁻ non-exhausted T cells, CD3⁺ PD-1⁻ Tim-3⁻ non-exhausted T cells. To calculate the absolute counts of the T-cell subsets, the percentage of that T-cell subset is multiplied by the volume of the bone marrow aspirate sample.

Because circulating T cells can infiltrate the bone marrow and therefore influence the E:T ratio, the percentage and absolute counts of circulating T cells are also measured. Blood is collected from the patients using heparinized tube or containing sodium citrate. Whole blood is then stained with fluorochrome-conjugated antibody directed at CD3 on ice for 20 min, protected from light. Red blood cells are then lysed with lysis buffer (BD Bioscience) and cells are washed twice with washing buffer. Acquisition is performed using a multicolor flow cytometer and installed software (e.g. CantoII device running FACS Diva software or FACS Calibur flow cytometer using the CellQUEST software). The Paint-A-Gate PRO program (BD Biosciences) is used for data analysis. Percentage of circulating T cells is determined by gating on CD3⁺ population. To calculate the absolute counts of T cells, the percentage of T cells is multiplied by the volume of blood sample.

With the percentages of infiltrating myeloma / malignant plasma cells (i.e. tumor cells) and T cells (e.g. effector cells) measured in the bone marrow aspirates of myeloma patients, E:T ratios could then be determined. As depicted in Table 6, the E:T ratios (T cells to myeloma cells) can vary considerably in multiple myeloma patients.

Evaluation of the T-cell subsets CD4⁺ and CD8⁺ was also measured. Cell suspension was collected from the untreated bone marrow aspirate culture (24h or less) and stained with fluorochrome-conjugated antibodies against human CD4 and human CD8 (BD Bioscience, San Jose CA). Table 6.1. shows the E:T ratios (CD4⁺ T cells to myeloma cells) in multiple myeloma patients and Table 6.2. shows the E:T ratios (CD8⁺ T cells to myeloma cells) in multiple myeloma patients.

E:T ratios with non-exhausted CD4⁺ and CD8⁺ T cells were also evaluated by measuring the percentage of CD4 or CD8 T-cell subset expressing PD-1 or TIM-3 on the cell surface. Cell suspension was collected from the untreated bone marrow aspirate culture and stained with fluorochrome-conjugated antibodies against human PD-1 and human TIM-3 (BD Bioscience, San Jose CA). Table 6.3. shows the E:T ratios (CD4⁺ PD-1⁻ T cells to myeloma cells) in multiple myeloma patients and Table 6.4. shows the E:T ratios (CD8⁺ PD-1⁻ T cells to myeloma cells) in multiple myeloma patients. Table 6.5. shows the E:T ratios (CD4⁺ TIM-3⁻ T cells to myeloma cells) in multiple myeloma patients and Table 6.6. shows the E:T ratios (CD8⁺ TIM-3⁻ T cells to myeloma cells) in multiple myeloma patients.

**Table 6: Effector cells (T cells) to tumor cells (E:T) ratios in untreated multiple myeloma patients**

| Patient No. | Bone marrow-infiltrated myeloma cells (%) | Bone marrow-infiltrated T cells (%) | E:T ratio in bone marrow |
|---|---|---|---|
| C1 | 2.0 | 21.89 | 11:1 (11.0) |
| C2 | 3.3 | 7.07 | 2:1 (2.0) |
| C3 | 2.74 | 18.09 | 7:1 (7.0) |
| C4 | 2.0 | 11.32 | 6:1 (6.0) |
| C5 | 20.18 | 9.12 | 0.5:1 (0.5) |
| C6 | 0.40 | 8.88 | 22:1 (22.0) |
| C7 | 4.37 | 15.75 | 3.6:1 (3.6) |
| C8 | 20.0 | 10.34 | 0.5:1 (0.5) |
| C9 | 8.20 | 8.38 | 1.02 |
| C10 | 2.60 | 8.52 | 3.28 |
| C11 | 18.00 | 6.59 | 0.37 |
| C12 | 22 | 7.7 | 0.35 |
| C13 | 12.40 | 6.72 | 0.54 |
| C14 | 4.20 | 12.21 | 2.91 |
| C15 | 1.76 | 7.16 | 4.07 |
| C16 | 31.30 | 13.62 | 0.44 |
| C17 | 12 | 10.43 | 0.87 |
| C18 | 10 | 27.89 | 2.79 |
| C19 | 2.30 | 5.53 | 2.40 |
| C20 | 7.00 | 2.9 | 0.41 |
| C21 | 6.5 | 6.67 | 1.03 |
| C22 | 6.10 | 8.23 | 1.35 |
| C23 | 5.00 | 14.77 | 2.95 |

**Table 6.1: Effector cells (CD4⁺ T cells) to tumor cells (E:T) ratios in untreated multiple myeloma patient bone marrow aspirates**

| Patient No. | Bone marrow-infiltrated myeloma cells (%) | Bone marrow-infiltrated CD4⁺ T cells (%) | E:T ratio in bone marrow |
|---|---|---|---|
| C12 | 19.24 | 2.6 | 0.14 |
| C13 | 2.02 | 2.6 | 1.29 |
| C17 | 11.98 | 10.6 | 0.88 |
| C18 | 6.35 | 4.8 | 0.76 |
| C19 | 5.86 | 4.5 | 0.77 |
| C21 | 2.27 | 6.1 | 2.69 |
| C22 | 3.35 | 2.3 | 0.69 |
| C23 | 0.76 | 20.5 | 26.97 |

**Table 6.2: Effector cells (CD8⁺ T cells) to tumor cells (E:T) ratios in untreated multiple myeloma patient bone marrow aspirates**

| Patient No. | Bone marrow-infiltrated myeloma cells (%) | Bone marrow-infiltrated CD8⁺ T cells (%) | E:T ratio in bone marrow |
|---|---|---|---|
| C12 | 19.24 | 6.9 | 0.36 |
| C13 | 2.02 | 4.7 | 2.33 |
| C17 | 11.98 | 12.3 | 1.03 |
| C18 | 6.35 | 25.9 | 4.08 |
| C19 | 5.86 | 2.0 | 0.34 |
| C21 | 2.27 | 6.3 | 2.78 |
| C22 | 3.35 | 3.51 | 1.05 |
| C23 | 0.76 | 5.6 | 7.37 |

**Table 6.3: Effector cells (CD4⁺ PD-1⁻ T cells) to tumor cells (E:T) ratios in untreated multiple myeloma patient bone marrow aspirates**

| Patient No. | Bone marrow-infiltrated myeloma cells (%) | Bone marrow-infiltrated CD4⁺ PD-1⁻ T cells (%) | E:T ratio in bone marrow |
|---|---|---|---|
| CC1 | 1.19 | 18.51 | 15.55 |
| CC2 | 1.05 | 10.51 | 10.01 |
| CC3 | 2.12 | 2.00 | 0.94 |
| CC4 | 11.01 | 2.74 | 0.25 |
| CC5 | 12.02 | 1.61 | 0.13 |
| CC6 | 3.08 | 3.85 | 1.25 |
| CC7 | 0.1 | 0.54 | 5.40 |
| CC8 | 2 | 6.10 | 3.05 |

**Table 6.4: Effector cells (CD8⁺ PD-1⁻ T cells) to tumor cells (E:T) ratios in untreated multiple myeloma patient bone marrow aspirates**

| Patient No. | Bone marrow-infiltrated myeloma cells (%) | Bone marrow-infiltrated CD8⁺ PD-1⁻ T cells (%) | E:T ratio in bone marrow |
|---|---|---|---|
| CC1 | 1.19 | 11.75 | 9.87 |
| CC2 | 1.05 | 5.90 | 5.62 |
| CC3 | 2.12 | 3.34 | 1.58 |
| CC4 | 11.01 | 4.06 | 0.37 |
| CC5 | 12.02 | 4.47 | 0.37 |
| CC6 | 3.08 | 5.17 | 1.68 |
| CC7 | 0.1 | 1.57 | 15.70 |
| CC8 | 2 | 5.55 | 2.78 |

**Table 6.5: Effector cells (CD4⁺ TIM-3⁻ T cells) to tumor cells (E:T) ratios in untreated multiple myeloma patient bone marrow aspirates**

| Patient No. | Bone marrow-infiltrated myeloma cells (%) | Bone marrow-infiltrated CD4⁺ TIM-3⁻ T cells (%) | E:T ratio in bone marrow |
|---|---|---|---|
| CC1 | 1.19 | 19.76 | 16.61 |
| CC2 | 1.05 | 11.24 | 10.70 |
| CC3 | 2.12 | 2.02 | 0.95 |
| CC4 | 11.01 | 2.74 | 0.25 |
| CC5 | 12.02 | 1.67 | 0.14 |
| CC6 | 3.08 | 4.07 | 1.32 |
| CC7 | 0.1 | 0.54 | 5.40 |
| CC8 | 2 | 6.76 | 3.38 |

**Table 6.6: Effector cells (CD8⁺ TIM-3⁻ T cells) to tumor cells (E:T) ratios in untreated multiple myeloma patient bone marrow aspirates**

| Patient No. | Bone marrow-infiltrated myeloma cells (%) | Bone marrow-infiltrated CD8⁺ TIM-3⁻ T cells (%) | E:T ratio in bone marrow |
|---|---|---|---|
| CC1 | 1.19 | 12.19 | 10.24 |
| CC2 | 1.05 | 7.07 | 6.73 |
| CC3 | 2.12 | 3.53 | 1.67 |
| CC4 | 11.01 | 4.28 | 0.39 |
| CC5 | 12.02 | 4.84 | 0.40 |
| CC6 | 3.08 | 5.48 | 1.78 |
| CC7 | 0.1 | 1.58 | 15.80 |
| CC8 | 2 | 6.56 | 3.28 |

### Example 2.2: Killing potency of BCMA-TCB is influenced by E:T ratio despite of high BCMA expression detected on the surface of H929 myeloma target cells

The killing potency of BCMA-1-TCB was measured in a redirected T-cell cytotoxicity assay using different E:T ratios and human myeloma cell lines with high vs. low level of BCMA expression on the cell surface.

### Example 2.2.1: Qualitative measurement of BCMA on human myeloma cell lines as detected by flow cytometry (median fluorescence intensity)

The qualitative expression of BCMA was first measured on the cell surface of H929 and U266 myeloma target cells using flow cytometry. Briefly, cells were harvested, washed, counted for viability, resuspended at 50,000 cells/well of a 96-well round bottom plate and incubated with anti-human BCMA antibody (Abcam, #ab54834, mouse IgG1) at 10 µg/ml for 30 min at 4°C (to prevent internalization). A mouse IgG1 was used as isotype control (BD Biosciences, #554121). Cells were then centrifuged (5 min at 350 × g), washed twice and incubated with the FITC-conjugated anti mouse secondary antibody for 30 min at 4°C. At the end of incubation time, cells were centrifuged (5 min at 350 × g), washed twice with FACS buffer, resuspended in 100 ul FACS buffer and analyzed on a CantoII device running FACS Diva software. Figure 3 depicts BCMA expression on H929 and U266 myeloma cells. There was a clear shift to the right as compared to negative for both human myeloma cell lines control with H929 cells being BCMA^{hi}-expressing cells and U266 being BCMA^{med/lo}-expressing cells.

### Example 2.2.2: Quantitative measurement of BCMA on human myeloma cell lines as detected by flow cytometry (specific antigen binding capacity SABC)

The quantitative expression of BCMA i.e. the specific antigen binding capacity (SABC) of BCMA was also measured on the cell surface of human myeloma cell lines using flow cytometry. The Qifikit (Dako, #K0078) method was used to quantify BCMA antigen copy number on the cell surface of H929 (ATCC^{®} CRL-9068^{™}) and U266 (ATCC^{®} TIB-196^{™}) human myeloma cell lines. Cells were once washed with FACS buffer (100 µl/well; 350 × g for 5 min) and adjusted to 1 Mio cells/ml. 50 µl (= 0.5 Mio cells) of the cell suspension are transferred into each well of a 96 round bottom well plate, as indicated. Then, 50 µl of mouse anti-human BCMA IgG (BioLegend #357502) or a mouse IgG2a isotype control (BioLegend # 401501) diluted in FACS buffer (PBS, 0.1% BSA) to a final concentration of 25 µg/ml (or at saturation concentrations) are added and staining is performed for 30 min at 4°C in the dark. Next, 100 µl of the Set-up or Calibration Beads are added in separate wells and the cells, as well as the beads are washed twice with FACS buffer. Cells and beads are resuspended in 25 µl FACS buffer, containing fluorescein conjugated anti-mouse secondary antibody (at saturation concentrations), provided by the Qifikit. Cells and beads are stained for 45 min at 4°C in the dark. The cells are washed once and all samples are resuspended in 100 µl FACS buffer. Samples are analyzed on a multicolor flow cytometer and installed software (e.g. CantoII device running FACS Diva software or FACSCalibur flow cytometer using the CellQUEST software). As shown in Table 7, H929 cells expressed human BCMA with the highest density, up to 5-6-fold higher more than other myeloma cell lines and was defined as BCMA^{hi}-expressing H929 in contrast to U266 which was defined as BCMA^{med/lo}-expressing myeloma cells. BCMA quantitative expression (SABC) correlated well with BCMA qualitative expression (relative MFI).

**Table 7: Quantitative measurement of BCMA expression on human myeloma cell lines as detected by by flow cytometry (specific antigen binding capacity SABC)**

| Human cell line | Relative BCMA SABC values |
|---|---|
| H929 | 50,000 |
| U266 | 6,000 |

**Table 7.1: Quantitative measurement of BCMA expression on human myeloma cell lines as detected by flow cytometry (specific antigen binding capacity SABC)**

| Human myeloma cell lines | Relative BCMA Specific antigen binding capacity (SABC) | | | | |
|---|---|---|---|---|---|
| | Donor 1 | Donor 2 | Donor 3 | Donor 4 | Donor 5 |
| H929 | 19357 | 54981 | 44800 | 100353 | 98050 |
| L363 | 16970 | / | 11300 | 11228 | / |
| U266 | / | 12852 | 11757 | / | 9030 |
| RPMI-8226 | 1165 | 5461 | / | 11361 | 2072 |

### Example 1.3: Potency of BCMA-TCB in the redirected T-cell cytotoxicity assay is influenced by E:T ratio.

The influence of E:T ratio on the killing potency of BCMA-TCB antibodies was tested. Briefly, human BCMA^{hi}-expressing H929 and BCMA^{mid/lo}-expressing U266 multiple myeloma target cells were harvested with Cell Dissociation Buffer, washed and resuspended in RPMI supplemented with 10% fetal bovine serum (Invitrogen). Approximately, 30,000 cells per well were plated in a round-bottom 96-well plate and the respective dilution of the construct was added for a desired final concentration (in triplicates); final concentrations ranging from 0.1 pM to 100 nM. For an appropriate comparison, all TCB constructs and controls were adjusted to the same molarity. Human total T cells (effector) were added into the wells to obtain a final E:T ratio of 5:1. Human PBMC were used as effector cells at different E:T ratios including 10:1, 2.5:1, 1:1. Negative control groups were represented by effector or target cells only. As a positive control for the activation of human pan T cells, 1 µg/ml PHA-M (Sigma #L8902) was used. For normalization, maximal lysis of the H929 MM target cells (= 100%) was determined by incubation of the target cells with a final concentration of 1% Triton X-100, inducing cell death. Minimal lysis (= 0%) was represented by target cells co-incubated with effector cells only, i.e. without any T cell bispecific antibody. After 24h incubation at 37°C, 5% CO₂, LDH release from the apoptotic myeloma target cells into the supernatant was then measured with the LDH detection kit (Roche Applied Science), following the manufacturer's instructions. The percentage of LDH release was plotted against the concentrations of BCMA-TCB antibodies in concentration-response curves. The EC50 values were measured using Prism software (GraphPad) and determined as the TCB antibody concentration that results in 50% of maximum LDH release. As shown in Table 8, the potency of BCMA-1-TCB to kill BCMA^{hi}-expressing H929 cells and BCMA^{mid/lo}-expressing U266 cells was influenced i.e. the killing potency of BCMA-1-TCB was reduced as the E:T ratio diminished. The reduction in killing potency (i.e. increase in EC50 values) was more pronounced in BCMA^{mid/lo}-expressing U266 cell lines and BCMA-1-TCB killing capability was lost when E:T ratio was reduced from 10:1 to 2.5:1 and 1:1.

Based on the results that 1) E:T ratios can vary considerably among multiple myeloma patients and 2) killing potency of BCMA-TCB can be influenced by E:T ratio, a patient stratification method including measurement of E:T ratio before treatment with BCMA-TCB is needed as myeloma patients with high E:T ratio would have more chance to respond to the BCMA-TCB therapy and patients with low E:T ratio could have less chance to respond to the BCMA-TCB therapy.

**Table 8: Potency of BCMA-1-TCB (EC50 values) to kill BCMA expressing myeloma cell lines is influenced by different E:T ratios and BCMA expression on target cells**

| E:T ratio (PBMC:MM) | Killing potency at 24h EC50 [nM] | |
|---|---|---|
| | BCMA^{hi}-expressing H929 cells | BCMA^{med/lo}-expressing U266 cells |
| 10:1 | 0.04 | 0.01 |
| 2.5:1 | 1.5 | Not measurable¹ |
| 1:1 | 7.0 | Not measurable¹ |

| | | |
|---|---|---|
| ¹Not measurable due to minimal killing observed | | |

### Example 3: Detection of soluble BCMA in myeloma patient serum/plasma and bone marrow aspirates by an ELISA-based methods

High levels of soluble BCMA can be found in the serum of untreated multiple myeloma patients as compared to healthy individuals, with soluble levels rising up to 120 ng/mL in some patients (Sanchez et al. Brit J Haematol 2012). As T-cell bispecific antibodies are very potent molecules with femtomolar to picomolar efficacy in in vitro cell-based assays, efficacious clinical doses are expected to be given at very low doses in patients (Bargou et al. Science 2008; 321 (5891); 974-7) and such soluble BCMA in myeloma patient may then affect the potency of BCMA-TCB antibodies by binding to them and preventing them to bind to BCMA on the cell surface of myeloma cells and redirected T-cell killing of malignant plasma cells is then prevented.

### Example 3.1: Measurement of soluble BCMA in myeloma patient serum/plasma and bone marrow aspirates

Peripheral blood and bone marrow aspirates are collected from multiple myeloma patients after informed consent is given, in accordance with local ethical committee guidelines and the Declaration of Helsinki. Serum from a Corvac^{™} serum separator tube (Becton Dickinson) is isolated by centrifugation and stored at -80°C until use. Bone marrow aspirates or blood are collected in heparinized tubes, centrifuged and the supernatant is collected and stored at -80°C until use. In some experiments, patient bone marrow aspirates are cultured for up to 48h before being assayed for soluble BCMA measurement. Serum, plasma and supernatant samples are analyzed by BCMA enzyme-linked immunosorbent assay (ELISA) (R&D Systems, catalogue #DY193E). Serum/plasma/bone marrow samples are diluted 1:50 and BCMA ELISA assay carried out according to the manufacturer's protocol. The ELISA plates are analyzed using a plate reader set to 450 nm. Values represent the mean of triplicate samples on each specimen. Notably, this specific BCMA ELISA kit does not cross react with recombinant human APRIL or BAFF, recombinant human TACI/Fc or recombinant mouse BCMA/Fc or mouse BCMA. BCMA antigen standards or serum/plasma/bone marrow (diluted 1:50) from myeloma patients are incubated using a murine monoclonal anti-human BCMA antibody (catalogue # WH0000608M1; Sigma-Aldrich) or the polyclonal capture antibody provided in the BCMA ELISA. The samples are then assayed accordingly to the BCMA ELISA protocol. Table 8.1 summarizes the levels of soluble BCMA measured in the supernatant of untreated myeloma patient bone marrow aspirates in culture (approx. 48h).

| **Table 8.1: Soluble BCMA levels in cultured bone marrow aspirate supernatant of myeloma patients** Myeloma patient | Soluble BCMA (pg/mL) |
|---|---|
| P1 | 5625 |
| P8 | 4630 |
| P12 | 4951 |
| P13 | 974 |
| P17 | 640 |
| P18 | 1696 |
| P19 | 757 |
| P20 | 3060 |
| P21 | 7340 |
| P22 | 3420 |
| P23 | 3317 |

### Example 3.2: Verification whether BCMA-TCB antibodies bind or not to soluble BCMA in myeloma patient serum/plasma and bone marrow aspirates

Myeloma patient serum/plasma and bone marrow aspirate supernatant samples containing soluble BCMA previously collected and stored at -80°C are tested for binding to BCMA-TCB antibodies using a capture sandwich ELISA method with a polyclonal BCMA antibody against human BCMA ECD used as capture antibody and as detection antibody a BCMA antibody that represents the BCMA binder of the BCMA-TCB bispecific antibody. Briefly, a polyclonal capture BCMA antibody is immobilized on a PVC microtiter plate at a concentration of 1 - 10 µg/mL in carbonate/bicarbonate buffer (pH 9.6). The plate is then covered with an adhesive plastic and incubated overnight at 4°C. The coating solution is then removed and the plate washed twice by filling the wells with 200 µl PBS. The solutions or washes are removed by flicking the plate over a sink. The remaining drops are removed by patting the plate on a paper towel. The remaining protein-binding sites in the coated wells are then blocked by adding 200 µl blocking buffer, 5% non-fat dry milk/PBS, per well. The plate is then covered with an adhesive plastic and incubated for at least 1-2 hours at room temperature or overnight at 4°C. 100 µl of appropriately diluted samples from the soluble BCMA-containing myeloma patient serum/plasma and bone marrow aspirate supernatant samples are added to each well in duplicates. Standards and blank are run with each plate. The microplate is then incubated for 90 min at 37°C. The samples are then removed and the plate is washed twice by filling the wells with 200 µl PBS. 100 µl of diluted biotin-conjugated detection antibody which consists of the BCMA antibody that represents the BCMA binder of the BCMA-TCB bispecific antibody is added to each well. The plate is then covered with an adhesive plastic and incubated for 2 hours at room temperature. The plate is washed four times with PBS. After the wash, 100 µl of streptavidin-conjugated to horse radish peroxidase (HRP) or alkaline phosphatase (ALP) is added to the wells. HRP or ALP-conjugated streptavidin is diluted at the optimal concentration in blocking buffer immediately before use. The plate is then covered with an adhesive plastic and incubated for 1-2 hours at room temperature. The plate is then washed four times with PBS. pNPP (p-Nitrophenyl-phosphate) is used as ALP substrate and added to the wells and incubated at room temperature for 15-30 min. The reaction is then stopped by adding an equal volume of 0.75 M NaOH. The plate is then measured at 405 nm using a plate reader. For HRP substrate, hydrogen peroxide is used and optimal density is read at 450 nm. A standard curve from the data produced from the serial dilutions with concentration on the x axis (log scale) vs. absorbance on the Y axis (linear) is then prepared.

### Example 4: Detection of soluble APRIL or BAFF in myeloma patient serum/plasma and bone marrow

In certain hematological malignancies such as multiple myeloma, the level of circulating BCMA-ligands APRIL and BAFF can be elevated (Moreaux et al. 2004; Blood 103(8): 3148-3157). Thus, the inventors recognize that high levels of ligands in the serum/plasma may interfere with the binding of BCMA-TCB antibodies to BCMA receptor on the tumor cells. In comparison to healthy donors, the levels of circulating APRIL (the high affinity ligand to BCMA) in multiple myeloma patient blood are ~100 ng/mL vs. ~10 ng/mL. For BAFF (the low affinity ligand to BCMA), the levels can fluctuate from 1-1000 ng/mL as compared to ~3 ng/mL in healthy donors. Nearby the tumor cells i.e. in the bone marrow microenvironment of multiple myeloma patients (the bone marrow being an organ constitutively rich in APRIL), APRIL/BAFF concentrations may very well be higher than the levels measured in the serum. More importantly, APRIL is constitutively expressed in the bone marrow microenvironment being an important survival factor to malignant myeloma cells and also being mainly produced and secreted by bone marrow myeloid precursor cells (Matthes et al. Blood 2011; 118 (7): 1838-1844). Thus, the concentrations of APRIL in the bone marrow of myeloma patients, which are expected to be of higher magnitude, up to 1000 ng/mL or even more, are of high relevance in this context. In certain autoimmune diseases such as systemic lupus erythematosus, the levels of circulating APRIL are also elevated with ∼ 85 ng/mL (Koyama et al. 2005; Ann Rheum Dis 64: 1065-1067).

For BCMA-TCB antibodies that compete with soluble APRIL (the BCMA ligand with high affinity) for binding to BCMA receptor, high levels of soluble APRIL may affect their potency, especially when BCMA-TCB antibodies clinical efficacious concentrations are expected to be low as they are potent molecules. Thus, there is a need for measuring soluble APRIL in multiple myeloma patient blood/serum/plasma or bone marrow aspirate samples.

### Example 4.1: Various levels of soluble APRIL can be measured in serum/plasma or bone marrow aspirates of myeloma patients by an ELISA-based method

Peripheral blood and bone marrow aspirates are collected from multiple myeloma patients after informed consent is given, in accordance with local ethical committee guidelines and the Declaration of Helsinki. Serum from a Corvac^{™} serum separator tube (Becton Dickinson) is isolated by centrifugation and stored at -80°C until use. Bone marrow aspirates and blood are collected in heparinized tubes, centrifuged and the supernatant is collected and stored at -80°C until use. In some experiments, patient bone marrow aspirates are cultured for up to 48h before being assayed for soluble APRIL measurement. Serum, plasma and bone marrow supernatant samples are analyzed by APRIL enzyme-linked immunosorbent assay (ELISA) (R&D Systems, catalogue #DY884B). Serum samples are diluted 1:50 and APRIL ELISA assay carried out according to the manufacturer's protocol. The ELISA plates are analyzed using a plate reader set to 450 nm. Values represent the mean of duplicate or triplicate samples on each specimen. APRIL antigen standards or serum/plasma or bone marrow aspirate supernatant (diluted 1:50) from myeloma patients are incubated using a capture antibody provided in the APRIL ELISA kit. The samples are then assayed accordingly to the APRIL ELISA protocol.

### Example 4.2: Soluble APRIL influences the potency of APRIL-competing/blocking BCMA-TCB antibody to kill BCMA-positive myeloma target cells

To verify whether the killing potency of APRIL blocking/competing BCMA-TCB antibodies would be affected by soluble APRIL, APRIL blocking/competing BCMA-TCB antibodies were analyzed for their potential to induce T cell-mediated killing of BCMA-positive myeloma target cells upon crosslinking of the construct via binding of the antigen binding moieties to BCMA on cells in the presence of elevated concentrations of soluble APRIL found in multiple myeloma patients (i.e. 100 ng/mL to 1000 ng/mL). Since APRIL binds to human BCMA with up to 1000-fold higher affinity than BAFF binds to the receptor, high concentrations of soluble APRIL are more relevant in this context than those of soluble BAFF. High levels of soluble APRIL would most likely influence the efficacy of TCB antibodies, especially when the therapeutic is given at very low doses in patients (Bargou et al. Science 2008; 321 (5891); 974-7). Thus, the following experiments were performed.

### Example 4.2.1: APRIL-blocking/competing J6M0-TCB antibody blocks APRIL-dependent NF-κB activation as detected by intracellular phosphorylated NF-κB (flow cytometry)

The effect of soluble APRIL on the killing potency of J6M0-TCB, a BCMA-TCB bispecific antibody made with APRIL/BAFF competing J6M0 (Tai YT et al. Blood 2014 123(29): 3128-28) as BCMA binder was planned to be tested in the redirected T-cell killing assay but before so, experiments were conducted to confirm that J6M0-TCB indeed blocks and competes with APRIL. J6M0-TCB was tested to block APRIL-dependent NF-κB activation. The detection of intracellular phosphorylated NF-κB was measured by flow cytometry, as described in Lafarge et al. BMC Molecular Biol 2007; 8:64. The phospho flow cytometry method is an alternative to the detection of NF-κB activation by ELISA-based luminescence assay which may not be sensitive enough and contains laborious steps (Perez and Nolan. Nat Biotechnol 2002; 20(2):155-62). It was assessed whether binding of J6M0-TCB to BCMA-positive H929 myeloma cells blocks APRIL-dependent NF-xB activation, a known nuclear factor signaling pathway downstream of BCMA receptor. Briefly, H929 cells were starved in RPMI1640 without FCS for 24 h at 37°C in cell incubator. At the end of the starvation time, cells were harvested, counted and cell viability evaluated using ViCell. Viable cells were adjusted to 1 × 10⁶ cells per ml in BSA-containing FACS Stain Buffer (BD Biosciences). 100 µl of this cell suspension were further aliquoted per well into a round-bottom 96-well plate and incubated with 25 µl of the J6M0-TCB antibody or isotype control antibodies at saturating concentration 400 nM (77 µg/ml) for 20 min at 37°C followed by direct incubation of 100 ng/mL or 1 µg/mL recombinant mouse A-APRIL (R&D Systems Europe) for additional 15 min at 37°C. As negative controls, cells were either left untreated or incubated with the corresponding IgG isotype control antibodies 400 nM (77 µg/ml) for a total of 45 min at 37°C. As positive controls, cells were incubated with 100 ng/mL or 1 µg/mL recombinant mouse Δ-APRIL alone (R&D Systems Europe) for 15 min at 37°C. At the end of the stimulation, the cells were centrifuged (360 × g, 4 min), the cell pellet immediately fixed in pre-warmed Cytofix Buffer (BD Biosciences, #554655) and incubated at 37°C for 10 minutes. The cells were then centrifuged, supernatant was removed and the cell pellet was disrupted by vortex. The cells were then permeabilized in ice cold Phosflow Perm Buffer III (BD Biosciences, #558050) for 30 min on ice. The cells were then centrifuged, supernatant was removed and the cell pellet was disrupted by vortex. Cells were resuspended in 100 µL Phosflow Perm Buffer III and the permeabilized cells were stained with anti-NF-xB p65 (pS529) antibody (BD Biosciences, #558423) or an isotype control antibody (Mouse IgG2b, κ, BD Biosciences #555058) for 60 min at room temperature protected from light. After the staining period, the cells were washed with PBS + 0.1% BSA in PBS + 0.1%BSA prior to flow cytometric analysis. The relative median fluorescence intensity obtained from H929 cells treated as described above was measured. The median fluorescence intensity (MFI) signal obtained upon binding of Δ-APRIL in presence of the isotype control was set to one; the other signals were normalized to it. As depicted in Figure 4, the effect of J6M0-TCB antibody with an APRIL competing BCMA binding arm was tested on 1000 ng/mL APRIL mediated NF-κB activation in H929 cells. Addition of soluble APRIL to H929 cells induced NF-κB activation. When H929 cells were exposed to APRIL competing BCMA binding arm J6M0-TCB, there was at least 79.3% decrease in the NF-κB activation signal as measured by phosphoflow cytometry. J6M0 anti-BCMA antibody (WO2012163805) has been reported to block APRIL-induced NF-κB activation. The current results confirm that J6M0 is an anti-BCMA antibody that is competing with APRIL for binding to BCMA and which blocks APRIL downstream signaling.

### Example 4.2.2: High levels of soluble APRIL influence the potency of APRIL competing BCMA-TCB antibody to kill BCMA-expressing H929 myeloma cells (colorimetric LDH release assay)

The effect of soluble APRIL on the killing potency of J6M0-TCB, a BCMA-TCB bispecific antibody made with APRIL/BAFF competing J6M0 (Tai YT et al. Blood 2014 123(29): 3128-28) as BCMA binder was then tested in the redirected T-cell killing assay. Briefly, human BCMA-positive H929 multiple myeloma target cells were harvested with Cell Dissociation Buffer, washed and resuspended in RPMI supplemented with 10% fetal bovine serum (Invitrogen). Approximately, 30,000 cells per well were plated in a round-bottom 96-well plate and the respective dilution of the TCB constructs were added for a desired final concentration (in triplicates); final concentrations of J6M0-TCB antibody ranging from 0.1 pM to 10 nM, in presence or absence of APRIL at final concentration of 100 ng/mL or 1000 ng/mL. For an appropriate comparison, all TCB constructs and controls were adjusted to the same molarity. Human PBMCs (effector) were added into the wells to obtain a final E:T ratio of 10:1. Negative control groups were represented by effector or target cells only. As a positive control for the activation of human pan T cells, 1 µg/mL PHA (Sigma #L8902) was used. For normalization, maximal lysis of the H929 MM target cells (= 100%) was determined by incubation of the target cells with a final concentration of 1% Triton X-100, inducing cell death. Minimal lysis (= 0%) was represented by target cells co-incubated with effector cells only, i.e. without any T cell bispecific antibody. After 24 h incubation at 37°C, 5% CO₂, LDH release from the apoptotic/necrotic H929 myeloma target cells into the supernatant was then measured with the LDH detection kit (Roche Applied Science), following the manufacturer's instructions. The percentage of LDH release was plotted against the concentrations of J6M0-TCB antibody in concentration-response curves. The EC50 values were measured using Prism software (GraphPad) and determined as the TCB antibody concentration that results in 50% of maximum LDH release. As shown in Figure 5, J6M0-TCB antibody induced killing BCMA-positive H929 myeloma cells in presence or absence of exogenous soluble APRIL. As depicted in Figure 5, APRIL blocking/competing J6M0-TCB induced a concentration-dependent killing of BCMA-positive H929 myeloma with a low picomolar potency (EC50_{APRIL0}= 5.8 pM) in the absence of exogenous APRIL. When 100 ng/mL of APRIL was added into the culture, such concentration of ligand only minimally affected the killing potency mediated by J6M0-TCB as shown with an 2.4-fold increase in the EC50 (EC50_{APRIL100}= 14.2 pM). However, when 1000 ng/mL of APRIL was added into the culture the killing potency mediated by J6M0-TCB was greatly reduced as reflected by an increase in the EC50 of 84.3-fold (EC50_{APRIL1000}= 488.9 pM). Table 9 summarizes the EC50 values of APRIL blocking/competing J6M0-TCB in absence and presence of exogenous APRIL.

The results suggest that APRIL blocking/competing BCMA-TCB antibodies could be influenced by high concentrations of soluble APRIL which could well be present in the bone marrow microenvironment or blood of multiple myeloma patients. Translating these observations into the clinical situation means that at a given low therapeutic dose of a BCMA-TCB such as J6M0-TCB in patients with high levels of APRIL in the bone marrow or blood, the myeloma cells may not be killed and the antitumor effect in patients with high levels of soluble APRIL could well be lost.

**Table 9: Potency (EC50) to kill H929 myeloma cells by APRIL/BAFF-competing BCMA-TCB is reduced by high levels of soluble APRIL**

| Exogenous APRIL (ng/mL) | Killing of H929 @ 24h EC50 (pM) | EC50 fold increase |
|---|---|---|
| 0 | 5.8 | - |
| 100 | 14.2 | 2.4x |
| 1000 | 488.9 | 84.3x |

### Example 4.3: Soluble APRIL influences the potency of APRIL-competing/blocking BCMA-TCB antibody to induce T-cell activation

The effect of soluble APRIL on the potency of APRIL-competing/blocking J6M0-TCB to induce T-cell activation was also tested by flow cytometry. T cell activation was measured by evaluating the surface expression of the early activation marker CD69, or the late activation marker CD25 on CD4⁺ and CD8⁺ T cells in the presence or absence of human BCMA-expressing MM cells. Briefly, BCMA-positive H929 cells were harvested with Cell Dissociation buffer, counted and checked for viability. Cells were adjusted to 0.3 × 10⁶ (viable) cells per ml in modified RPMI-1640 medium, 100 µl of this cell suspension were pipetted per well into a round-bottom 96-well plate (as indicated). 50 µl of the (diluted) APRIL-competing/blocking J6M0-TCB antibody was added to the cell-containing wells to obtain a final concentration of 0.3 pM - 30 nM. Human PBMC effector cells were isolated from fresh blood of a healthy donor and adjusted to 6 × 10⁶ (viable) cells per ml in modified RPMI-1640 medium. 50 µl of this cell suspension was added per well of the assay plate to obtain a final E:T ratio of PBMC to myeloma tumor cells of 10: 1. To analyze whether APRIL-competing/blocking J6M0-TCB antibody was able to activate T cells specifically in the presence of target cells expressing human BCMA, wells were included that contained 3 nM of J6M0-TCB antibody, as well as PBMCs, but no target cells. After incubation for 15-28 h (CD69), or 24-48 h (CD25) at 37°C, 5% CO₂, cells were centrifuged (5 min, 350 × g) and washed twice with 150 µl/well PBS containing 0.1% BSA. Surface staining for CD4 (mouse IgGl,K; clone RPA-T4), CD8 (mouse IgGl,K; clone HIT8a; BD #555635), CD69 (mouse IgGl; clone L78; BD #340560) and CD25 (mouse IgGl,K; clone M-A251; BD #555434) was performed at 4°C for 30 min, according to the supplier's suggestions. Cells were washed twice with 150 µl/well PBS containing 0.1% BSA and fixed for 15 min at 4°C, using 100 µl/well fixation buffer (BD #554655). After centrifugation, the samples were resuspended in 200 µl/well PBS with 0.1% BSA and analyzed using a FACS CantoII machine (Software FACS Diva). Figure 6 depicts the expression level of the early activation marker CD69 (B, D), and the late activation marker CD25 (A, C) on CD4⁺ and CD8⁺ T cells after 48 hours of incubation (representative results from two independent experiments). APRIL-competing/blocking J6M0-TCB antibody induced an up-regulation of CD69 and CD25 activation markers in a concentration-dependent and specific manner in the presence of BCMA-positive target cells in absence of exogenous soluble APRIL (squares). When 100 ng/mL of soluble APRIL was added into the culture, a slight shift to the right of the concentration-response curves was observed for both activation markers CD69 and CD25 on CD4⁺ and CD8⁺ T cells. When 1000 ng/mL of soluble APRIL was added into the culture, there was a clear reduction of T-cell activation on both CD4⁺ and CD8⁺ T cells. No activation of CD4⁺ and CD8⁺ T cells was observed when human PBMCs were treated with DP47-TCB control antibody, suggesting that despite binding to CD3 on the T cells T-cell activation does not occur when the TCB antibody does not bind to BCMA-positive target cells (data not shown). The results clearly suggest that high levels of soluble APRIL reduce the potency of BCMA-TCB antibodies to induce T-cell activation upon binding to the tumor target and T cells, especially when the BCMA-TCB is competes with APRIL.

### Example 5: The potency of BCMA-1-TCB antibody to kill myeloma patient malignant plasma cells is more pronounced in patient bone marrow samples with greater E:T ratio and greater BCMA expression (relative MFI) on malignant plasma cells.

Bone marrow aspirates are collected from multiple myeloma patients after informed consent was given, in accordance with local ethical committee guidelines and the Declaration of Helsinki. To evaluate the potency of BCMA-1-TCB antibody to induce redirected T-cell killing of bone marrow-infiltrating malignant plasma cells by bone marrow-infiltrating autologous T cells, whole bone marrow samples were collected from myeloma patients and BCMA-1-TCB antibody was spiked directly into the whole bone marrow samples. Briefly, 200 µl of the whole bone marrow sample were transferred to 96 deep-well plates. BCMA-1-TCB antibody and control antibody dilutions were prepared in sterile PBS and the preparation was added to the respective wells for final concentrations ranging from 0.03 pM to 30 nM. The whole bone marrow-antibody suspension was mixed by gentle shaking and then incubated at 37°C, 5 % CO₂ for 24 h, sealed with paraffin film. After the incubation period, the cell suspension samples were erythrocyte-lysed with K₃-EDTA (ethylenediaminetetraacetic acid) and the cell samples were prepared for the immunophenotypic analyses. 20 µl of a corresponding FACS antibody solution prepared based on an antibody-panel including CD138-APCC750/CD38-FITC/CD5-BV510/CD56-PE/CD19-PerCP-Cy7/CD45-V450/BCMA-APC/Annexin-V-PerCP-Cy5.5 was added into a 96-U-bottom plate. Fluorochrome-labelled antibodies were purchased from BD Biosciences (San Jose, CA) and Caltag Laboratories (San Francisco CA) and in-house APC-conjugated anti-human BCMA antibody was used. The samples were then incubated for 15 minutes in the dark at room temperature and acquired and analyzed using a multilaser flow cytometer. Myeloma cell death was determined by evaluating annexin-V positive expression gated on the malignant plasma cell population CD138⁺ CD38⁺ CD45⁺ CD19⁻ CD56⁺. Percentages of myeloma cell death was then determined at each concentration of BCMA-1-TCB bispecific antibody. As depicted in Figure 7, BCMA-1-TCB induced a concentration dependent specific killing of malignant plasma cells from both patient C1 (A) and patient C8 (B) already after only 24h of incubation. However, killing of myeloma cells was more pronounced in patient C1 bone marrow samples than in patient C8 bone marrow samples. This could be attributed to a more favorable E:T ratio of 11:1 and BCMA expression (i.e. relative MFI value of 2636) in patient C1 bone marrow samples than in patient C8 bone marrow samples with an unfavourable E:T ratio of 0.5:1 and weaker BCMA expression on myeloma cells (i.e. relative MFI value of 1489). The results suggest that measurement of BCMA expression on malignant plasma cells in combination with a measurement of E:T ratio in patient bone marrow may more accurately predict whether myeloma patients may respond to BCMA-TCB treatment.

### Example 6: Patient biological parameters (BCMA relative expression on myeloma cells, E:T ratios, soluble APRIL, and soluble BCMA) in relation to the responsiveness of patient bone marrow samples to BCMA-1-TCB

Samples with moderate relative MFI (ranging from 1000 to 5000) respond to the drug by 71.4% (5/7), and samples with low BCMA expression (relative MFI < 1000) respond to the drug by 33.3% (1/3). Patient samples with a favourable E:T ratio (E:T >1) respond to the drug by 66.7% (4/6). Patient samples with unfavourable E:T ratio < 1 does respond to the drug by 60% (3/5). The combination of at least two biological parameters was evaluated. There was found a correlation of 75% (3/4) when favourable BCMA expression on myeloma cells and favourable E:T ratio were both used for drug response prediction.

**Table 10: Patient biological parameters (BCMA relative expression on myeloma cells, E:T ratios, soluble APRIL, and soluble BCMA) in relation to the responsiveness of patient bone marrow samples to BCMA-1-TCB**

| Patient BM sample | BCMA relative MFI | E:T ratio (T cell: MM PC) | Soluble BCMA (pg/mL) | Response to BCMA-1-TCB |
|---|---|---|---|---|
| 1 | **2636** | **11** | 5625 | **Responsive** |
| 8 | **1489** | 0.5 | 4630 | **Responsive** |
| 12 | **2396** | 0.35 | 4951 | **Responsive** |
| 13 | 356 | 0.54 | 974 | Non responsive |
| 17 | **1574** | 0.87 | 640 | Non responsive |
| 18 | **1147** | **2.79** | 1696 | **Responsive** |
| 19 | **1847** | **2.4** | 757 | **Responsive** |
| 20 | 913 | 0.41 | 3060 | **Responsive** |
| 21 | **3422** | **1.03** | 7340 | Non responsive |
| 22 | 547 | **1.35** | 3420 | Non responsive |
| 23 | 136 | **2.95** | 3317 | **Responsive** |

### Preferred embodiments of the invention are described below and are referred to as E1-27

1. A bispecific antibody specifically binding to the extracellular domain of human B-cell maturation antigen (further named also as "BCMA") and human CD3ε (further named also as "CD3"), for use in the treatment of a patient suffering from multiple myeloma disease, said disease being characterized in that in an isolated body fluid sample of said patient, comprising CD138+ CD38+ cells, BCMA expression on said CD138+ CD38+ cells, measured by using an anti-BCMA antibody with a Kd value, which is 0.70 to 1.3 fold of the Kd value of the anti-BCMA antibody part of said bispecific antibody, is 80 or more over baseline determined as Relative Median or Mean Fluorescence Intensity MFI.
2. A bispecific antibody specifically binding to the extracellular domain of human BCMA and human CD3ε, for use in the treatment of a patient suffering from multiple myeloma disease, said disease being characterized in that the ratio of T cells (effector cells) to target cells (E:T ratio) in an isolated body fluid sample of said patient is 0.35 : 1 or higher.
3. A bispecific antibody for use according to E2, characterized in that the E:T ratio is 0.35 : 1 to 22:1.
4. A bispecific antibody specifically binding to the extracellular domain of human BCMA and human CD3ε, for use in the treatment of a patient suffering from multiple myeloma disease, said disease being characterized in that in an isolated body fluid sample from said patient the amount of soluble BCMA is 2.5 ng/mL or higher, and said soluble BCMA in said patient sample specifically binds to said bispecific antibody, characterized in that said treatment of said patient with said bispecific antibody is performed with a dose per week which is 1.5 fold up to 10 fold or/and in that the time interval between dose-administrations is shortened from once per week administration up to once per day compared to a standard dose.
5. The bispecific antibody for use according to E4, characterized in that said treatment of said patient with said bispecific antibody is performed with a dose per week which is 1.5 fold up to 2.0 fold compared to a standard dose.
6. The bispecific antibody for use according to E4, characterized in that said treatment of said patient with said bispecific antibody is performed in that the time interval between dose-administrations is shortened from once per week administration up to twice a week compared to the standard dose.
7. A bispecific antibody specifically binding to BCMA and CD3ε which competes with soluble BCMA for binding to human BCMA receptor and/or blocks APRIL mediated activation of NF-κB for use in the treatment of a patient suffering from multiple myeloma disease, said disease being characterized in that in an isolated body fluid sample from said patient the amount of APRIL is higher than 10 ng/mL and up to 100 ng/mL, characterized in that said treatment of said patient with said bispecific antibody is performed per week with a dose which is 1.5 fold up to 20 fold or/and in that the time interval between dose-administrations is shortened from once per week administration up to once a day compared to a standard dose.
8. The bispecific antibody for use according to E7, characterized in that said treatment of said patient with said bispecific antibody is performed with a dose per week which is 1.5 fold up to a 3.0 fold compared to a standard dose.
9. The bispecific antibody for use according to E7, characterized in that said treatment of said patient with said bispecific antibody is performed in that the time interval between dose-administrations is shortened from once per week administration up to three times a week compared to the standard dose .
10. A bispecific antibody specifically binding to BCMA and CD3ε which competes with soluble BCMA for binding to human BCMA receptor, whereby said antibody competes with APRIL for binding to BCMA, whereby said antibody competes with APRIL for binding to BCMA and/or blocks APRIL mediated activation of NF-κB for use in the treatment of a patient suffering from multiple myeloma disease, said disease being characterized in that in an isolated body fluid sample of said patient, comprising plasma cells and T cells, and the amount of APRIL is at least 100 ng/mL up to 1000 ng/mL, characterized in that said treatment of said patient with said bispecific antibody is performed with a dose which is 1.5 fold up to 80 fold or/and in that the time interval between dose-administrations is shortened from once per week administration up to once a day compared to a standard dose.
11. The bispecific antibody for use according to E10, characterized in that said treatment of said patient with said bispecific antibody is performed with a dose per week which is 1.5 fold up to a 10 fold compared to a standard dose.
12. The bispecific antibody for use according to E10, characterized in that said treatment of said patient with said bispecific antibody is performed in that the time interval between dose-administrations is shortened from once per week administration up to once a day compared to the standard dose .
13. A bispecific antibody specifically binding to the extracellular domain of human BCMA and human CD3, for use in the treatment of a patient suffering from a disorder involving plasma cells, and whereby in an isolated body fluid sample of said patient, comprising CD138⁺ CD38⁺ cells, BCMA expression on said CD138⁺ CD38⁺ cells, measured by using an anti-BCMA antibody with a Kd value, which is 0.70 to 1.3 fold of the Kd value of the anti-BCMA antibody part of said bispecific antibody, is 80 or over baseline determined as Relative Median or Mean Fluorescence Intensity MFI.
14. The bispecific antibody for use according to any one of E1 to 13, characterized in that said bispecific antibody comprises as its heavy and light chain CDRs, CDRs of the same amino acid sequences as said anti-BCMA antibody.
15. A bispecific antibody specifically binding to the extracellular domain of human BCMA and human CD3ε, for use in the treatment of a patient suffering from a disorder involving plasma cells, whereby the ratio of T cells (effector cells) to target cells (E:T ratio) in an isolated body fluid sample of said patient is 0.35 : 1 to 22:1.
16. A bispecific antibody specifically binding to the extracellular domain of human BCMA and human CD3ε, for use in the treatment of a patient suffering from a disorder involving plasma cells, whereby said therapy comprises successively
   i) isolating from said patient a body fluid sample,
   ii) measuring the amount of soluble BCMA in said sample, and
   iii) if the amount of soluble BCMA in said sample is 2.5 ng/mL or higher, and
   iv) if said soluble BCMA in said patient sample specifically binds to said bispecific antibody, treating said patient with said bispecific antibody with a weekly dose which is compared to a standard dose 1.5 to 10 fold or 1.5 to 2.0 fold and/or the time interval between dose-administrations is shortened from once per week administration to twice per week or even three times a week or once a day.
17. A bispecific antibody specifically binding to BCMA and CD3ε which competes with soluble BCMA for binding to human BCMA receptor whereby said antibody competes with APRIL for binding to BCMA and/or blocks APRIL mediated activation of NF-κB for use in the treatment of a patient suffering from a disorder involving plasma cells, whereby said therapy comprises successively
   i) isolating from said patient a body fluid sample comprising plasma cells and T cells,
   ii) measuring the amount of APRIL in said sample by use of an ELISA method, and
   iii) if the amount of APRIL in said patient sample is higher than 10 ng/ml and up to 100 ng/mL, treating said patient with said bispecific antibody at a two times higher weekly dose and a further increased dose up to 80 times higher if APRIL concentration increases up to 1000 ng/ml, compared to the dose recommended for a patient with soluble APRIL concentration below 10 ng/mL or treating said patient with a respective more frequent treatment schedule to reach said higher doses with a shorter period between any two doses of said bispecific antibody.
18. A method of determining BCMA protein expression in an isolated body fluid sample comprising CD138⁺ CD38⁺ cells, of a patient, suffering from a disorder involving plasma cells, said method comprising measuring BCMA expression on said CD138⁺ CD38⁺ cells by using an anti-BCMA antibody with a Kd value, which is 0.70 to 1.3 fold of the Kd value of the anti-BCMA antibody part of a bispecific antibody specifically binding to the extracellular domain of human BCMA and human CD3ε, intended for use in the treatment of said patient, and determining whether Relative Median or Mean Fluorescence Intensity MFI is 80 or more over baseline.
19. An in vitro method of determining cell-surface BCMA expression in an isolated body fluid sample, comprising determining whether Relative Median or Mean Fluorescence Intensity MFI for said CD138⁺ CD38⁺ cells, using an anti-BCMA antibody with a Kd value, which is 0.70 to 1.3 fold of the Kd value of the anti-BCMA antibody part of a therapeutic bispecific antibody specifically binding to BCMA and CD3ε, is 80 or more over baseline.
20. A method for determining in an isolated body fluid sample of a patient, suffering from a disorder involving plasma cells, whether the ratio of CD3⁺ cells to CD138⁺ CD38⁺ cells is 0.35 : 1 or higher.
21. An in vitro method of determining in an isolated body fluid sample of a patient suffering from a disorder involving plasma cells whether the ratio of CD3⁺ cells to CD138⁺ CD38⁺ cells is 0.35 : 1 or higher.
22. A method of determining in an isolated body fluid sample comprising CD138⁺ CD38⁺ cells, of a patient suffering from a disorder involving plasma cells, whether the amount of soluble BCMA in said sample is 2.5 ng/mL or higher.
23. An in vitro method of determining in an isolated body fluid sample of a patient suffering from a disorder involving plasma cells, whether the amount of soluble BCMA in said sample is 2.5 ng/mL or higher.
24. A method of determining in an isolated body fluid sample comprising CD138⁺ CD38⁺ cells, of a patient suffering from a disorder involving plasma cells, whether the amount of soluble APRIL in said sample is 80 ng/mL or higher.
25. A kit for use of determination of cell-surface BCMA expression, comprising vials or tubes pre-loaded with four labelled-antibodies, one specifically binding to CD138, one to CD38, one to CD19, and one anti-BCMA antibody with a Kd value, which is 0.70 to 1.3 fold of the Kd value of the anti-BCMA antibody part of a therapeutic bispecific antibody specifically binding to BCMA and CD3ε.
26. A kit for use of determination of E:T ratio, comprising vials or tubes pre-loaded with four labelled-antibodies to detect malignant PC and T cells, one specifically binding to CD138, one to CD38, one to CD19, and one to CD3.
27. A kit for use of determination of soluble BCMA, comprising a polyclonal anti-BCMA antibody as capture antibody, and a detection anti-BCMA antibody with a Kd value, which is 0.70 to 1.3 fold of the Kd value of the anti-BCMA antibody part of a therapeutic bispecific antibody specifically binding to BCMA and CD3ε.

## Claims

1. A bispecific antibody specifically binding to the extracellular domain of human B-cell maturation antigen (BCMA) and human CD3ε, for use in the treatment of a patient suffering from a disorder involving plasma cells, wherein in an isolated body fluid sample of said patient comprising CD138+ CD38+ cells, BCMA expression on said CD138+ CD38+ cells is 80 or more over baseline, wherein BCMA expression is determined as Relative Median or Mean Fluorescence Intensity (MFI) using an anti-BCMA antibody that specifically binds to the extracellular domain of BCMA with a Kd value which is 0.70 to 1.3 fold of the Kd value of the anti-BCMA antibody of the bispecific antibody, and wherein the baseline is the MFI of a reference T cell.

2. The bispecific antibody for use according to claim 1, wherein BCMA expression on said CD138+ CD38+ cells is 100 or more over baseline, 200 or more over baseline, or 300 or more over baseline, preferably wherein BCMA expression on said CD138+ CD38+ cells is 1000 to 3000, 1000 to 5000 or 3000 to 10,000 over baseline.

3. The bispecific antibody for use according to claim 1 or 2, wherein:
(a) the CD138+ CD38+ cells are CD138+ CD38+ CD45+ CD19- CD56+ cells; and/or
(b) the reference T cell is a CD3+ T cell.

4. The bispecific antibody for use according to any one of the preceding claims, wherein:
(a) said bispecific antibody comprises as its heavy and light chain CDRs, CDRs of the same amino acid sequences as said anti-BCMA antibody; and/or
(b) the anti-BCMA antibody for measuring the MFI is attached with a fluorophore label and the MFI of the reference T cell is measured using the anti-BCMA antibody using FACS apparatus.

5. A bispecific antibody specifically binding to the extracellular domain of human BCMA and human CD3ε, for use in the treatment of a patient suffering from a disorder involving plasma cells, wherein in an isolated body fluid sample from said patient the amount of soluble BCMA is 2.5 ng/mL or higher, wherein the soluble BCMA specifically binds to the bispecific antibody, and wherein the treatment comprises adapting the dose and/or dose interval of the bispecific antibody.

6. The bispecific antibody for use according to claim 5, wherein the amount of soluble BCMA is 10 ng/mL or higher, 50 ng/mL or higher, or 250 ng/mL or higher.

7. The bispecific antibody for use according to claim 5 or 6, wherein the amount of soluble BCMA is measured by an ELISA assay.

8. A bispecific antibody specifically binding to the extracellular domain of human BCMA and human CD3ε for use in the treatment of a patient suffering from a disorder involving plasma cells, wherein:
(a) the amount of soluble APRIL in an isolated body fluid sample of said patient is 100 ng/mL or lower, preferably 20 ng/mL or lower, and wherein the bispecific antibody is an APRIL competitive bispecific antibody;
(b) the amount of soluble APRIL in an isolated body fluid sample of said patient is 100 ng/mL or higher, preferably 1000 ng/mL or higher, and wherein the bispecific antibody is an APRIL non-competitive bispecific antibody, optionally wherein the binding of the bispecific antibody is not reduced by 100 ng/mL APRIL by more than 20% measured in an ELISA assay compared to the binding of the bispecific antibody to human BCMA without APRIL; or
(c) the amount of soluble APRIL in an isolated body fluid sample of said patient is 100 ng/mL or higher, preferably 1000 ng/mL or higher, and wherein the bispecific antibody is an APRIL competitive bispecific antibody, wherein the treatment comprises adapting the dose and/or dose interval of the bispecific antibody.

9. The bispecific antibody for use according to claim 8, wherein the amount of soluble APRIL is measured by an ELISA assay.

10. The bispecific antibody for use according to any one of claims 5-9, wherein the adaptation of dose and/or dose interval comprises administering the bispecific antibody at higher doses and/or at a more frequent treatment schedule.

11. The bispecific antibody for use according to any one of the preceding claims, wherein the disorder involving plasma cells is multiple myeloma.

12. The bispecific antibody for use according to any one of the preceding claims, wherein the ratio of T cells (effector cells) to target cells (E:T ratio) in the isolated body fluid sample of said patient is 0.5:1 or higher, wherein the target cells express BCMA on their surface, optionally wherein the E:T ratio is 1:1 or higher, 5:1 or higher, 10:1 or higher; and/or 0.5:1 to 22:1.

13. The bispecific antibody for use according to claim 12, wherein:
(a) the T cells in the isolated body fluid sample are CD3+ T cells, preferably wherein the T cells are CD3+ CD8+ cytotoxic T cells; and/or
(b) the target cells in the isolated body fluid sample are plasma cells, CD138+ CD38+ cells or CD138+ CD38+ CD19- CD56+ cells.

14. The bispecific antibody for use according to any one of the preceding claims, wherein the isolated body fluid sample is an isolated blood sample or a bone marrow aspirate.

15. The bispecific antibody according to any one of the preceding claims, wherein said bispecific antibody comprises:
(i) a CDRL1 of SEQ ID NO: 2, a CDRL2 of SEQ ID NO: 3, a CDRL3 of SEQ ID NO: 4, a CDRH1 of SEQ ID NO: 6, a CDRH2 of SEQ ID NO:7 and a CDRH3 of SEQ ID NO: 8; and
(ii) a CDRL3 of SEQ ID NO: 12, and a CDRH3 of SEQ ID NO: 16.
